# EUROPEAN PATENT APPLICATION

(11) **EP 3 388 081 A1**
(43) Date of publication of application: **17.10.2018**
(21) Application number: 17205287.0
(22) Date of filing: 26.05.2011
(51) Int. Cl.: A61K 39/385, A61K 39/00, A61K 39/39, A61K 45/06, A61K 47/02, A61K 47/22, A61K 47/24, A61K 47/50, A61K 9/51, A61K 31/7115, A61K 31/4745, A61K 33/06, A61P 37/04, A61P 31/12, A61P 31/04, A61P 31/10, A61P 25/34

(54) **MULTIVALENT SYNTHETIC NANOCARRIER VACCINES**

(30) Priority: 26.05.2010 US 348728 P; 26.05.2010 US 348717 P; 26.05.2010 US 348713 P; 25.06.2010 US 358635 P
(62) Divisional of application: 11787442.0
(71) Applicant: Selecta Biosciences, Inc., Watertown, MA 02472 (US)
(72) Inventor: BRATZLER, Robert, L., Concord, MA Massachusetts 01742 (US); JOHNSTON, Lloyd, Belmont, MA Massachusetts 02478 (US); LIPFORD, Grayson, B., Watertown, Massachusetts 02472 (US); ZEPP, Charles, Hardwick, MA Massachusetts 01037 (US)
(74) Representative: EIP

(57) **Abstract**

The invention relates, at least in part, to compositions comprising populations of synthetic nanocarriers that comprise different sets of antigens as well as related methods.

## Description

### RELATED APPLICATIONS

This application claims the benefit under 35 U.S.C. §119 of United States provisional applications 61/348713, filed May 26, 2010, 61/348717, filed May 26, 2010, 61/348728, filed May 26, 2010, and 61/358635, filed June 25, 2010, the entire contents of each of which are incorporated herein by reference.

### BACKGROUND OF THE INVENTION

Multivalent vaccines are a useful way of generating an immune response to certain foreign substances that otherwise would not be desirably robust. For instance, vaccinating against multiple strains of a virus may provide more robust cross-protection against multiple strains of that virus as compared to vaccinating using a monovalent vaccine.

However, current multivalent vaccines and methods of making them need improvement. For instance, current approaches of conjugating antigens to protein carriers are complex and provide for low yields. Additionally, new techniques often need to be developed to conjugate new antigens to carrier proteins because conventional techniques can be unsuccessful due to the relative fragility of conventional carrier proteins.

What is needed are compositions and methods that provide for improved multivalent vaccines.

### SUMMARY OF THE INVENTION

In one aspect, a composition comprising a dosage form comprising a first population of synthetic nanocarriers that comprise a first set of surface antigens; a second population of synthetic nanocarriers that comprise a second set of surface antigens; and a pharmaceutically acceptable excipient, wherein the first set of surface antigens and the second set of surface antigens are structurally different is provided.

In another aspect, a composition comprising a dosage form comprising a first population of synthetic nanocarriers that comprise a first set of surface antigens; a second population of synthetic nanocarriers that comprise a second set of surface antigens; and a pharmaceutically acceptable excipient, wherein the first set of surface antigens and the second set of surface antigens are immunologically different is also provided.

In yet another aspect, a composition comprising a dosage form comprising a first synthetic nanocarrier means for presenting a first set of surface antigens; a second synthetic nanocarrier means for presenting a second set of surface antigens; and a pharmaceutically acceptable excipient, wherein the first set of surface antigens and the second set of surface antigens are structurally different is also provided.

In still another aspect, a composition comprising a dosage form comprising a first synthetic nanocarrier means for presenting a first set of surface antigens; a second synthetic nanocarrier means for presenting a second set of surface antigens; and a pharmaceutically acceptable excipient, wherein the first set of surface antigens and the second set of surface antigens are immunologically different is provided.

In one embodiment of any of the compositions provided, the first set of surface antigens comprises 1, 2, 3, 4, 5, 6, 7, 8, 9, 10 or more types of antigens. In another embodiment of any of the compositions provided, the second set of surface antigens comprises 2, 3, 4, 5, 6, 7, 8, 9, 10 or more types of antigens.

In yet another embodiment of any of the compositions provided, the first set of surface antigens comprise antigens obtained or derived from a first infectious genus and the second set of surface antigens comprise antigens obtained or derived from a second infectious genus. In one embodiment, the first infectious genus and the second infectious genus are the same. In still another embodiment of any of the compositions provided, the first set of surface antigens comprise antigens obtained or derived from a first infectious species and the second set of surface antigens comprise antigens obtained or derived from of a second infectious species. In one embodiment, the first infectious species and the second infectious species are the same. In a further embodiment, the first set of surface antigens comprise antigens obtained or derived from a first infectious strain and the second set of surface antigens comprise antigens obtained or derived from a second infectious strain. In one embodiment, the first infectious strain and second infectious strain are the same.

In another embodiment of any of the compositions provided, the first set of surface antigens and/or second set of surface antigens comprise antigens that are obtained or derived from a virus of the Adenoviridae, Picornaviridae, Herpesviridae, Hepadnaviridae, Flaviviridae, Retroviridae, Orthomyxoviridae, Paramyxoviridae, Papillomaviridae, Rhabdoviridae, Togaviridae or Paroviridae family. In one embodiment, the first set of surface antigens and/or second set of surface antigens comprise antigens that are obtained or derived from adenovirus, coxsackievirus, hepatitis A virus, poliovirus, Rhinovirus, Herpes simplex virus, Varicella-zoster virus, Epstein-barr virus, Human cytomegalovirus, Human herpesvirus, Hepatitis B virus, Hepatitis C virus, yellow fever virus, dengue virus, West Nile virus, HIV, Influenza virus, Measles virus, Mumps virus, Parainfluenza virus, Respiratory syncytial virus, Human metapneumovirus, Human papillomavirus, Rabies virus, Rubella virus, Human bocarivus or Parvovirus B19. In another embodiment, the first set of surface antigens and/or second set of surface antigens comprise antigens that are obtained or derived from VI, VII, E1A, E3-19K, 52K, VP1, surface antigen, 3A protein, capsid protein, nucleocapsid, surface projection, transmembrane proteins, UL6, UL18, UL35, UL38, UL19, early antigen, capsid antigen, Pp65, gB, p52, latent nuclear antigen-1, NS3, envelope protein, envelope protein E2 domain, gp120, p24, lipopeptides Gag (17-35), Gag (253-284), Nef (66-97), Nef (116-145), Pol (325-355), neuraminidase, nucleocapsid protein, matrix protein, phosphoprotein, fusion protein, hemagglutinin, hemagglutinin-neuraminidase, glycoprotein, E6, E7, envelope lipoprotein or non-structural protein (NS).
In still another embodiment of any of the compositions provided, the first set of surface antigens and/or second set of surface antigens comprise antigens that are obtained or derived from a bacteria of the Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema Vibrio or Yersinia genus. In one embodiment, the first set of surface antigens and/or second set of surface antigens comprise antigens that are obtained or derived from Bordetella pertussis, Borrelia burgdorferi, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diphtheriae, Enterococcus faecalis, Enterococcus faecium, Escherichia coli, Francisella tularensis, Haemophilus influenzae, Helicobacter pylori, Legionella pneumophila, Leptospira interrogans, Listeria monocytogenes, Mycobacterium leprae, Mycobacterium tuberculosis, Mycobacterium ulcerans, Mycoplasma pneumoniae, Neisseria gonorrhoeae, Neisseria meningitides, Pseudomonas aeruginosa, Rickettsia rickettsii, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae or Yersinia pestis. In another embodiment, the first set of surface antigens and/or second set of surface antigens comprise antigens that are obtained or derived from pertussis toxin (PT), filamentous hemagglutinin (FHA), pertactin (PRN), fimbriae (FIM 2/3), VlsE; DbpA, OspA, Hia, PrpA, MltA, L7/L12, D15, 0187, VirJ, Mdh, AfuA, L7/L12, out membrane protein, LPS, antigen type A, antigen type B, antigen type C, antigen type D, antigen type E, FliC, FliD, Cwp84, alpha-toxin, theta-toxin, fructose 1,6-biphosphate-aldolase (FBA), glyceraldehydes-3-phosphate dehydrogenase (GPD), pyruvate:ferredoxin oxidoreductase (PFOR), elongation factor-G (EF-G), hypothetical protein (HP), T toxin, Toxoid antigen, capsular polysaccharide, Protein D, Mip, nucleoprotein (NP), RD1, PE35, PPE68, EsxA, EsxB, RD9, EsxV, Hsp70, lipopolysaccharide, surface antigen, Sp1, Sp2, Sp3, Glycerophosphodiester Phosphodiesterase, outer membrane protein, chaperone-usher protein, capsular protein (F1) or V protein.

In yet another embodiment of any of the compositions provided, the first set of surface antigens and/or second set of surface antigens comprise antigens that are obtained or derived from a fungus of the Candida, Aspergillus, Cryptococcus, Histoplasma, Pneumocystis or Stachybotrys genus. In one embodiment, the first set of surface antigens and/or second set of surface antigens comprise antigens that are obtained or derived from C. albicans, Aspergillus fumigatus, Aspergillus flavus, Cryptococcus neoformans, Cryptococcus laurentii, Cryptococcus albidus, Cryptococcus gattii, Histoplasma capsulatum, Pneumocystis jirovecii or Stachybotrys chartarum. In another embodiment, the first set of surface antigens and/or second set of surface antigens comprise antigens that are obtained or derived from surface antigen, capsular glycoprotein, Yps3P, Hsp60, Major surface protein, MsgC1, MsgC3, MsgC8, MsgC9 or SchS34.

In still another embodiment of any of the compositions provided, the first set of surface antigens and/or second set of surface antigens comprise antigens that are obtained or derived from any of the infectious agents, viruses, bacteria, proteins, peptides, polypeptides, small molecules, polysaccharides or oligosaccharides provided herein.

In a further embodiment of any of the compositions provided, the first set of surface antigens and second set of surface antigens comprise antigens obtained or derived from an abused or addictive substance. In one embodiment, the abused or addictive substance is cocaine or nicotine.

In yet a further embodiment of any of the compositions provided, the first set of surface antigens and the second set of surface antigens comprise the same surface antigens, and wherein at least one antigen of the first set of surface antigens is presented in a different orientation than as presented in the second set of surface antigens.

In still a further embodiment of any of the compositions provided, the first set of surface antigens and the second set of surface antigens comprise the same surface antigens, and wherein at least one antigen of the first set of surface antigens is presented in a different conformation than as presented in the second set of surface antigens.

In another embodiment of any of the compositions provided, the molecular structure of the first set of surface antigens and the second set of surface antigens are different.

In yet another embodiment of any of the compositions provided, the first set of surface antigens and/or the second set of surface antigens comprise surface antigens with a molecular weight of less than 10,000 Da.

In still another embodiment of any of the compositions provided, the first set of surface antigens and/or the second set of surface antigens comprise surface antigens that comprise peptides, proteins, oligosaccharides, polysaccharides and/or small molecules.

In a further embodiment of any of the compositions provided, at least one surface antigen of the first set of surface antigens and/or at least one surface antigen of the second set of surface antigens has a molecular weight of less than 10,000 Da.

In a yet a further embodiment of any of the compositions provided, the first set of surface antigens comprises surface antigens comprising peptides, and the second set of surface antigens comprises surface antigens with a molecular weight of less than 10,000 Da.

In still a further embodiment of any of the compositions provided, the first set of surface antigens comprises surface antigens comprising peptides, and the second set of surface antigens comprises surface antigens comprising peptides, proteins, oligosaccharides, polysaccharides and/or small molecules. In one embodiment, at least one surface antigen of the second set of surface antigens has a molecular weight of less than 10,000 Da.

In another embodiment of any of the compositions provided, the first set of surface antigens comprises surface antigens comprising proteins, and the second set of surface antigens comprises surface antigens with a molecular weight of less than 10,000 Da.

In yet another embodiment of any of the compositions provided, the first set of surface antigens comprises surface antigens comprising proteins, and the second set of surface antigens comprises surface antigens comprising peptides, proteins, oligosaccharides, polysaccharides and/or small molecules. In one embodiment, at least one surface antigen of the second set of surface antigens has a molecular weight of less than 10,000 Da.

In still another embodiment of any of the compositions provided, the first set of surface antigens comprises surface antigens comprising oligosaccharides, and the second set of surface antigens comprises surface antigens with a molecular weight of less than 10,000 Da.

In a further embodiment of any of the compositions provided, the first set of surface antigens comprises surface antigens comprising oligosaccharides, and the second set of surface antigens comprises surface antigens comprising peptides, proteins, oligosaccharides, polysaccharides and/or small molecules. In one embodiment, at least one surface antigen of the second set of surface antigens has a molecular weight of less than 10,000 Da.

In yet a further embodiment of any of the compositions provided, the first set of surface antigens comprises surface antigens comprising polysaccharides, and the second set of surface antigens comprises surface antigens with a molecular weight of less than 10,000 Da.

In still a further embodiment of any of the compositions provided, the first set of surface antigens comprises surface antigens comprising polysaccharides, and the second set of surface antigens comprises surface antigens comprising peptides, proteins, oligosaccharides, polysaccharides and/or small molecules. In one embodiment, at least one surface antigen of the second set of surface antigens has a molecular weight of less than 10,000 Da.

In another embodiment of any of the compositions provided, the first set of surface antigens comprises surface antigens comprising small molecules, and the second set of surface antigens comprises surface antigens with a molecular weight of less than 10,000 Da.

In still another embodiment of any of the compositions provided, the first set of surface antigens comprises surface antigens comprising small molecules, and the second set of surface antigens comprises surface antigens comprising peptides, proteins, oligosaccharides, polysaccharides and/or small molecules. In one embodiment, at least one surface antigen of the second set of surface antigens has a molecular weight of less than 10,000 Da.

In one embodiment of any of the compositions provided, the compositions further comprise one or more adjuvants. In one embodiment, the first population of synthetic nanocarriers and/or the second population of synthetic nanocarriers further comprise an adjuvant coupled to the synthetic nanocarriers. In another embodiment, the first population of synthetic nanocarriers and/or the second population of synthetic nanocarriers further comprise an adjuvant coupled to the synthetic nanocarriers and the composition comprises one or more admixed adjuvants.

In one embodiment, each of the one or more adjuvants of any of the compositions provided comprises a mineral salt, alum, alum combined with monphosphoryl lipid (MPL) A of Enterobacteria, MPL® (AS04), AS15, a saponin, QS-21,Quil-A, ISCOMs, ISCOMATRIX™, MF59™, Montanide® ISA 51, Montanide® ISA 720, AS02, a liposome or liposomal formulation, AS01, AS15, synthesized or specifically prepared microparticles and microcarriers, bacteria-derived outer membrane vesicles of *N. gonorrheae* or *Chlamydia trachomatis,* chitosan particles, a depot-forming agent, Pluronic® block co-polymers, specifically modified or prepared peptides, muramyl dipeptide, an aminoalkyl glucosaminide 4-phosphate, RC529, a bacterial toxoid, a toxin fragment, an agonist of Toll-Like Receptors 2, 3, 4, 5, 7, 8 or 9, an adenine derivative, immunostimulatory DNA, immunostimulatory RNA, an imidazoquinoline amine, an imidazopyridine amine, a 6,7-fused cycloalkylimidazopyridine amine, a 1,2-bridged imidazoquinoline amine, imiquimod, resiquimod, an agonist for DC surface molecule CD40, a type I interferon, poly I:C, a bacterial lipopolysacccharide (LPS), VSV-G, HMGB-1, flagellin or portions or derivatives thereof, an immunostimulatory DNA molecule comprising CpG, proinflammatory stimuli released from necrotic cells, urate crystals, an activated component of the complement cascade, an activated component of immune complexes, a complement receptor agonist, a cytokine, or a cytokine receptor agonist. In one embodiment, the adjuvants are different. In another embodiment, the adjuvant coupled to the first population of synthetic nanocarriers and/or the adjuvant coupled to the second population of synthetic nanocarriers comprises a TLR-2, -3, -4, -7, -8 or -9 agonist. In yet another embodiment, the adjuvant coupled to the first population of synthetic nanocarriers and/or the adjuvant coupled to the second population of synthetic nanocarriers comprises an immunostimulatory nucleic acid, imidazoquinoline, oxoadenine, MPL, imiquimod or resiquimod. In one embodiment, the admixed adjuvant is an immunostimulatory nucleic acid comprising CpG, AS01, AS02, AS04, AS15, QS-21, a saponin, alum or MPL.

In one embodiment of any of the compositions provided, the first and second populations of synthetic nanocarriers are present in an amount effective to generate an immune response to the first set of surface antigens and the second set of surface antigens in a subject. In one embodiment, the immune response is the generation of antibody titers specific for the first set of surface antigens and the second set of surface antigens.

In another embodiment of any of the compositions provided, the compositions comprise one or more additional populations of synthetic nanocarriers, wherein each additional population of synthetic nanocarriers comprises a set of surface antigens structurally different from the other sets of surface antigens in the composition. In one embodiment, at least one of the one or more additional populations of synthetic nanocarriers further comprise an adjuvant coupled thereto. In another embodiment, the adjuvant coupled to the at least one of the one or more additional populations of synthetic nanocarriers is different from the other adjuvants in the composition.

In yet another embodiment of any of the compositions provided, the first set of surface antigens comprises a first set of monovalent or oligovalent surface antigens; and the second set of surface antigens comprises a second set of monovalent or oligovalent surface antigens.

In still another embodiment of any of the compositions provided, each set of surface antigens is a monovalent or oligovalent set of surface antigens.

In a further embodiment of any of the compositions provided, the populations of synthetic nanocarriers are present in an amount effective to generate an immune response to each set of surface antigens. In one embodiment, the immune response is the generation of antibody titers specific for each set of surface antigens.

In one embodiment of any of the compositions provided, the first and/or second population of synthetic nanocarriers further comprise a universal T cell antigen coupled thereto. In another embodiment, the universal T cell antigen comprises a T helper cell antigen. In yet another embodiment, the T-helper cell antigen comprises a peptide obtained or derived from ovalbumin. In still another embodiment, the peptide obtained or derived from ovalbumin comprises the sequence as set forth in SEQ ID NO: 1. In a further embodiment, the universal T cell antigen is coupled by encapsulation.

In one embodiment of any of the compositions provided, the pharmaceutically acceptable excipient comprises a preservative, a buffer, saline, phosphate buffered saline, a colorant, or a stabilizer.

In another embodiment of any of the compositions provided, synthetic nanocarriers of each of the populations of synthetic nanocarriers comprise lipid-based nanoparticles, polymeric nanoparticles, metallic nanoparticles, surfactant-based emulsions, dendrimers, buckyballs, nanowires, virus-like particles, peptide or protein-based particles, lipid-polymer nanoparticles, spheroidal nanoparticles, cuboidal nanoparticles, pyramidal nanoparticles, oblong nanoparticles, cylindrical nanoparticles, or toroidal nanoparticles. In one embodiment, each of the populations of synthetic nanocarriers comprise one or more polymers. In another embodiment, the one or more polymers comprise a polyester. In still another embodiment, the one or more polymers comprise or further comprise a polyester coupled to a hydrophilic polymer. In yet another embodiment, the polyester comprises a poly(lactic acid), poly(glycolic acid), poly(lactic-co-glycolic acid), or polycaprolactone. In a further embodiment, the hydrophilic polymer comprises a polyether. In still a further embodiment, the polyether comprises polyethylene glycol.

In another aspect, a method comprising administering any of the compositions provided to a subject is provided. In one embodiment, the subject has or is at risk of having an infection or infectious disease. In another embodiment, the subject has or is at risk of having cancer. In yet another embodiment, the subject has or is at risk of having an addiction. In a further embodiment, the composition is administered by oral, subcutaneous, pulmonary, intranasal, intradermal or intramuscular administration.

In yet another aspect, a method comprising preparing a first population of synthetic nanocarriers that comprise a first set of surface antigens; preparing a second population of synthetic nanocarriers that comprise a second set of surface antigens; and combining the first and second populations of synthetic nanocarriers into a dosage form, wherein the first set of surface antigens and the second set of surface antigens are structurally different is provided.

In a further aspect, a method comprising preparing a first population of synthetic nanocarriers that comprise a first set of surface antigens; preparing a second population of synthetic nanocarriers that comprise a second set of surface antigens; and combining the first and second populations of synthetic nanocarriers into a dosage form, wherein the first set of surface antigens and the second set of surface antigens are immunologically different is provided. In one embodiment, the method further comprises administering the dosage form to a subject. In another embodiment, the method further comprises determining whether or not an immune response to each set of surface antigens is generated. In one embodiment, the immune response is the generation of antibody titers specific for each set of surface antigens. In a further embodiment, the method further comprises determining the amount effective to generate the immune response to each set of surface antigens.

In still a further aspect, a process for producing a dosage form of a composition, the process comprising the method steps as defined in any of the methods provided herein is provided.

In one embodiment, any of the compositions provided is for use in therapy or prophylaxis. In another embodiment, any of the compositions provided is for use in any of the methods provided herein. In yet another embodiment, any of the compositions provided is for use in a method of treating or preventing infection or infectious disease. In still another embodiment, any of the compositions provided is for use in a method of treating or preventing cancer. In a further embodiment, any of the compositions provided is for use in a method of treating or preventing an addiction.

In another embodiment, any of the methods comprise administration of any of the compositions by oral, subcutaneous, pulmonary, intranasal, intradermal or intramuscular administration.

In yet another aspect, the use of any of the compositions provided for the manufacture of a medicament for use in any of the methods provided is provided.

### BRIEF DESCRIPTION OF FIGURES

**Fig. 1** shows anti-nicotine (dark gray bars) and anti-ovalbumin (light gray bars) antibody titers in unimmunized mice and mice injected with NC-Nic and NC-OVA (5 animals/group; s.c., 100 µg of each NC per injection, 2 times at 3-wk intervals).
**Fig. 2** shows anti-nicotine, anti-ovalbumin, and anti-L2 peptide antibody titers in unimmunized mice and mice injected with NC-Nic-OVA and NC-L2 (5 animals/group; s.c., 100 µg of each NC per injection, 2 times at 3-wk intervals).
**Fig. 3** shows anti-nicotine, anti-ovalbumin, anti-M2e peptide, and anti-L2 peptide antibody titers in unimmunized mice and mice injected with NC-Nic-OVA and NC-M2e-L2 (5 animals/group; s.c., 100 µg of each NC per injection, 2 times at 3-wk intervals).
**Fig. 4** shows anti-M2e peptide and anti-L2 peptide antibody titers in unimmunized mice and mice injected with NC-M2e and NC-L2 (5 animals/group; s.c., 100 µg of each NC per injection, 2 times at 3-wk intervals).
**Fig. 5** shows anti-HA5 protein and anti-ovalbumin protein antibody titers in unimmunized mice and mice injected with NC-HA5 and NC-OVA (5 animals/group; s.c., 100 µg of each NC per injection, 2 times at 3-wk intervals).
**Fig. 6** shows anti-HA, anti-ovalbumin, anti-M2e peptide, and anti-L2 peptide antibody titers in unimmunized mice and mice injected with NC-HA5, NC-OVA, and NC-M2e-L2 (5 animals/group; s.c., 100 µg of each NC per injection, 2 times at 3-wk intervals).
**Fig. 7** shows antibody titers in mice immunized with a combination of NC-M2e, NC-L2 peptide and NC-nicotine-ovalbumin.
**Fig. 8** shows antibody titers in mice immunized with a combination of NC-3'-nicotine and NC-1'-nicotine.

### DETAILED DESCRIPTION OF THE INVENTION

Before describing the present invention in detail, it is to be understood that this invention is not limited to particularly exemplified materials or process parameters as such may, of course, vary. It is also to be understood that the terminology used herein is for the purpose of describing particular embodiments of the invention only, and is not intended to be limiting of the use of alternative terminology to describe the present invention.

All publications, patents and patent applications cited herein, whether supra or infra, are hereby incorporated by reference in their entirety for all purposes.

As used in this specification and the appended claims, the singular forms "a," "an" and "the" include plural referents unless the content clearly dictates otherwise. For example, reference to "a polymer" includes a mixture of two or more such molecules, reference to "a solvent" includes a mixture of two or more such solvents, reference to "an adhesive" includes mixtures of two or more such materials, and the like.

### INTRODUCTION

The inventors have unexpectedly and surprisingly discovered that the problems and limitations noted above can be overcome by practicing the invention disclosed herein. In particular, the inventors have unexpectedly discovered that it is possible to provide inventive compositions, and related methods, that address the problems and limitations in the art by providing a composition comprising a dosage form comprising: a first population of synthetic nanocarriers that comprise a first set of surface antigens; a second population of synthetic nanocarriers that comprise a second set of surface antigens; and a pharmaceutically acceptable excipient; wherein the first set of surface antigens and the second set of surface antigens are structurally different.

In another aspect, the invention provides a composition comprising: a dosage form comprising: a first population of synthetic nanocarriers that comprise a first set of surface antigens; a second population of synthetic nanocarriers that comprise a second set of surface antigens; and a pharmaceutically acceptable excipient; wherein the first set of surface antigens and the second set of surface antigens are immunologically different.

In an aspect, the invention provides a composition comprising: a dosage form comprising: a first synthetic nanocarrier means for presenting a first set of surface antigens; a second synthetic nanocarrier means for presenting a second set of surface antigens; and a pharmaceutically acceptable excipient; wherein the first set of surface antigens and the second set of surface antigens are structurally different.

In an aspect, the invention provides a composition comprising: a dosage form comprising: a first synthetic nanocarrier means for presenting a first set of surface antigens; a second synthetic nanocarrier means for presenting a second set of surface antigens; and a pharmaceutically acceptable excipient; wherein the first set of surface antigens and the second set of surface antigens are immunologically different.

In another aspect, the invention provides a method comprising: preparing a first population of synthetic nanocarriers that comprise a first set of surface antigens; preparing a second population of synthetic nanocarriers that comprise a second set of surface antigens; and combining the first and second populations of synthetic nanocarriers into a dosage form; wherein the first set of surface antigens and the second set of surface antigens are structurally different.

In yet another aspect, the invention provides a method comprising: preparing a first population of synthetic nanocarriers that comprise a first set of surface antigens; preparing a second population of synthetic nanocarriers that comprise a second set of surface antigens; and combining the first and second populations of synthetic nanocarriers into a dosage form; wherein the first set of surface antigens and the second set of surface antigens are immunologically different.

It has been discovered that it is possible to generate a first and second population of synthetic nanocarriers that comprise a first and second set of surface antigens, respectively, which can be combined together with a pharmaceutically acceptable excipient to create a dosage form. This dosage form can, in certain embodiments, be useful as a multivalent vaccine. The inventors have further noted certain advantages in the creation of the inventive dosage forms, particularly with respect to conventional multivalent vaccines. These include, but are not limited to, minimizing vaccine volumes which is a problem in conventional multivalent vaccines, and minimizing protein-protein interactions present in conventional protein carrier-hapten multivalent vaccines that can lead to non-specific binding and precipitation.

A further advantage of the present invention is that combining different populations of synthetic nanocarriers that comprise sets of surface antigens allows different methods to be used to couple different sets of surface antigens to different populations of synthetic nanocarriers. This can be a significant advantage for embodiments wherein incompatible coupling methods are required to couple sets of surface antigens to populations of synthetic nanocarriers. As an example, vaccines for *Streptococcus pneumonia* (US patent 6,132,723 to Alberta Research Council and WO 2008/143709 to Wyeth) contain multiple antigens. Since the attachment chemistry conditions are not the same for all of the polysaccharide antigens (WO 2008/143709) coupling methods that would attach all of the surface antigens to a single population of synthetic nanocariers in a single coupling environment would be undesirable. Practicing embodiments of the present invention wherein different populations of synthetic nanocarriers are first coupled to certain sets of surface antigens and then combined could ameliorate the problem noted in the art.

Another example of multivalent vaccines that could benefit from this embodiment of the invention comprise vaccines against N. meningitides which is polysaccharide-based and multivalent. Such embodiments may be aimed either at N. meningitidis groups A and C (bivalent) or groups A, C, W135 and Y (tetravalent).

The examples illustrate certain embodiments according to the invention, wherein peptides, polysaccharides, small molecules, etc., are conjugated to a first population of synthetic nanocarriers and/or a second population of synthetic nanocarriers. These populations are then combined to form a composition according to the invention.

The present invention will now be described in more detail.

### DEFINITION

"Abused substance" is any substance taken by a subject (e.g., a human) for purposes other than those for which it is indicated or in a manner or in quantities other than directed by a physician. The abused substance, in some embodiments, is an addictive substance. In some embodiments, the abused substance for inclusion in a nanocarrier is the complete molecule, analog or a portion thereof. "Addictive substance" is a substance that causes obsession, compulsion, or physical dependence or psychological dependence. In some embodiments, the addictive substance for inclusion in a nanocarrier is the complete molecule, analog or a portion thereof.

"Adjuvant" means an agent that does not constitute a specific antigen, but boosts the strength and longevity of immune response to an administered antigen (e.g., a concomitantly administered antigen). Such adjuvants may include, but are not limited to stimulators of pattern recognition receptors, such as Toll-like receptors, RIG-1 and NOD-like receptors (NLR), mineral salts, such as alum, alum combined with monphosphoryl lipid (MPL) A of Enterobacteria, such as Escherihia coli, Salmonella minnesota, Salmonella typhimurium, or Shigella flexneri or specifically with MPL® (AS04), MPL A of above-mentioned bacteria separately, saponins, such as QS-21,Quil-A, ISCOMs, ISCOMATRIX™, emulsions such as MF59™, Montanide® ISA 51 and ISA 720, AS02 (QS21+squalene+ MPL®), liposomes and liposomal formulations such as AS01, AS15, synthesized or specifically prepared microparticles and microcarriers such as bacteria-derived outer membrane vesicles (OMV) of N. gonorrheae, Chlamydia trachomatis and others, or chitosan particles, depot-forming agents, such as Pluronic® block co-polymers, specifically modified or prepared peptides, such as muramyl dipeptide, aminoalkyl glucosaminide 4-phosphates, such as RC529, or proteins, such as bacterial toxoids or toxin fragments.

In embodiments, adjuvants comprise agonists for pattern recognition receptors (PRR), including, but not limited to Toll-Like Receptors (TLRs), specifically TLRs 2, 3, 4, 5, 7, 8, 9 and/or combinations thereof. In other embodiments, adjuvants comprise agonists for Toll-Like Receptors 3, agonists for Toll-Like Receptors 7 and 8, or agonists for Toll-Like Receptor 9; preferably the recited adjuvants comprise imidazoquinolines; such as R848; adenine derivatives, such as those disclosed in US patent 6,329,381 (Sumitomo Pharmaceutical Company), US Published Patent Application 2010/0075995 to Biggadike et al., or WO 2010/018132 to Campos et al.; immunostimulatory DNA; or immunostimulatory RNA.

In specific embodiments, synthetic nanocarriers incorporate as adjuvants compounds that are agonists for toll-like receptors (TLRs) 7 & 8 ("TLR 7/8 agonists"). Of utility are the TLR 7/8 agonist compounds disclosed in US Patent 6,696,076 to Tomai et al., including but not limited to imidazoquinoline amines, imidazopyridine amines, 6,7-fused cycloalkylimidazopyridine amines, and 1,2-bridged imidazoquinoline amines. Preferred adjuvants comprise imiquimod and resiquimod (also known as R848). In specific embodiments, synthetic nanocarriers incorporate a ligand for toll-like receptor (TLR)-9, such as CpGs, which induce type I interferon production, and stimulate T and B cell activation leading to increased antibody production and cytotoxic T cell responses (Krieg et al., CpG motifs in bacterial DNA trigger direct B cell activation. Nature. 1995. 374:546-549; Chu et al. CpG oligodeoxynucleotides act as adjuvants that switch on T helper 1 (Th1) immunity. J. Exp. Med. 1997. 186:1623-1631; Lipford et al. CpG-containing synthetic oligonucleotides promote B and cytotoxic T cell responses to protein antigen: a new class of vaccine adjuvants. Eur. J. Immunol. 1997. 27:2340-2344; Roman et al. Immunostimulatory DNA sequences function as T helper-1-promoting adjuvants. Nat. Med. 1997. 3:849-854; Davis et al. CpG DNA is a potent enhancer of specific immunity in mice immunized with recombinant hepatitis B surface antigen. J. Immunol. 1998. 160:870-876; Lipford et al., Bacterial DNA as immune cell activator. Trends Microbiol. 1998. 6:496-500; US Patent 6,207,646 to Krieg et al.; US Patent 7,223,398 to Tuck et al.; US Patent 7,250,403 to Van Nest et al.; or US Patent 7,566,703 to Krieg et al.).

In specific embodiments, an adjuvant may be an agonist for the DC surface molecule CD40. In certain embodiments, to stimulate immunity rather than tolerance, a synthetic nanocarrier incorporates an adjuvant that promotes DC maturation (needed for priming of naive T cells) and the production of cytokines, such as type I interferons, which promote antibody immune responses and anti-viral immunity. In embodiments, adjuvants also may comprise immunostimulatory RNA molecules, such as but not limited to dsRNA, ssRNA, poly I:C or poly I:poly C12U (available as Ampligen ®, both poly I:C and poly I:polyC12U being known as TLR3 stimulants), and/or those disclosed in F. Heil et al., "Species-Specific Recognition of Single-Stranded RNA via Toll-like Receptor 7 and 8" Science 303(5663), 1526-1529 (2004); J. Vollmer et al., "Immune modulation by chemically modified ribonucleosides and oligoribonucleotides" WO 2008033432 A2; A. Forsbach et al., "Immunostimulatory oligoribonucleotides containing specific sequence motif(s) and targeting the Toll-like receptor 8 pathway" WO 2007062107 A2; E. Uhlmann et al., "Modified oligcoribonucleotide analogs with enhanced immunostimulatory activity" U.S. Pat. Appl. Publ. US 2006241076; G. Lipford et al., "Immunostimulatory viral RNA oligonucleotides and use for treating cancer and infections" WO 2005097993 A2; G. Lipford et al., "Immunostimulatory G,U-containing oligoribonucleotides, compositions, and screening methods" WO 2003086280 A2.

In some embodiments, an adjuvant may be a TLR-4 agonist, such as bacterial lipopolysacharide (LPS), VSV-G, and/or HMGB-1. In some embodiments, adjuvants may comprise TLR-5 agonists, such as flagellin, or portions or derivatives thereof, including but not limited to those disclosed in US Patents 6,130,082, 6,585,980, and 7,192,725.

In some embodiments, adjuvants may be proinflammatory stimuli released from necrotic cells (e.g., urate crystals). In some embodiments, adjuvants may be activated components of the complement cascade (e.g., CD21, CD35, etc.). In some embodiments, adjuvants may be activated components of immune complexes. The adjuvants also include complement receptor agonists, such as a molecule that binds to CD21 or CD35. In some embodiments, the complement receptor agonist induces endogenous complement opsonization of the synthetic nanocarrier. In some embodiments, adjuvants are cytokines, which are small proteins or biological factors (in the range of 5 kD - 20 kD) that are released by cells and have specific effects on cell-cell interaction, communication and behavior of other cells. In some embodiments, the cytokine receptor agonist is a small molecule, antibody, fusion protein, or aptamer.

In embodiments, at least a portion of the dose of adjuvant may be coupled to synthetic nanocarriers, preferably, all of the dose of adjuvant is coupled to synthetic nanocarriers. In other embodiments, at least a portion of the dose of the adjuvant is not coupled to the synthetic nanocarriers. In embodiments, the dose of adjuvant comprises two or more types of adjuvants. For instance, and without limitation, adjuvants that act on different TLR receptors may be combined. As an example, in an embodiment a TLR 7/8 agonist may be combined with a TLR 9 agonist. In another embodiment, a TLR 7/8 agonist may be combined with a TLR 4 agonist. In yet another embodiment, a TLR 9 agonist may be combined with a TLR 3 agonist.

"Administering" or "administration" means providing a substance to a subject in a manner that is pharmacologically useful.

"Amount effective" is any amount of a composition that produces one or more desired immune responses. This amount can be for in vitro or in vivo purposes. For in vivo purposes, the amount can be one that a health practitioner would believe may have a clinical benefit for a subject in need of an antibody response specific to one or more antigens. "Antibody response" means any immune response that results in the production or stimulation of B cells and/or the production of antibodies. In embodiments, therefore, an amount effective is one that a health practitioner would believe may generate an antibody response against the surface antigen(s) of the inventive compositions provided herein. Effective amounts can be monitored by routine methods. An amount that is effective to produce one or more desired immune responses can also be an amount of a composition provided herein that produces a desired therapeutic endpoint or a desired therapeutic result. Therefore, in other embodiments, the amount effective in one that a clinician would believe would provide a therapeutic benefit (including a prophylactic benefit) to a subject provided herein. Such subjects include those that have or are at risk of having cancer, an infection or infectious disease.

The antigen(s) of any of the inventive compositions provided herein can in embodiments be in an amount effective. In some embodiments, the amount effective is one that a health practitioner would believe may generate antibody titers against the sets of surface antigens of the compositions provided herein. "Antibody titer" means the production of a measurable level of antibodies. Preferably, the antibody response or generation of the antibody titer is in a human. In some embodiments, the antibodies are antibodies are of a certain isotype, such as IgG or a subclass thereof. Methods for measuring antibody titers are known in the art and include Enzyme-linked Immunosorbent Assay (ELISA). Methods for measuring antibody response are also described in some detail in the Examples. Preferably, the antibody response or antibody titer is specific to a set of surface antigens. In some embodiments where the synthetic nanocarriers also comprise a universal antigen in addition to a set of surface antigens against which a specific immune response, such as an antibody response or antibody titer, the immune response is specific to the set of surface antigens but not to the universal antigen.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a "maximum dose" be used, that is, the highest safe dose according to sound medical judgment. It will be understood by those of ordinary skill in the art, however, that a patient may insist upon a lower dose or tolerable dose for medical reasons, psychological reasons or for virtually any other reasons.

"Antigen" means a B cell antigen or T cell antigen. In embodiments, antigens are coupled to the synthetic nanocarriers. In other embodiments, antigens are not coupled to the synthetic nanocarriers. In embodiments antigens are coadministered with the synthetic nanocarriers. In other embodiments antigens are not coadministered with the synthetic nanocarriers. "Type(s) of antigens" means molecules that share the same, or substantially the same, antigenic characteristics.

"At least a portion of the dose" means at least some part of the dose, ranging up to including all of the dose.

An "at risk" subject is one in which a health practitioner believes has a chance of having the disease or condition provided herein including, but not limited to, an infection, infectious disease, cancer or an addiction.

"B cell antigen" means any antigen that is or recognized by and triggers an immune response in a B cell (e.g., an antigen that is specifically recognized by a B cell receptor on a B cell). In some embodiments, an antigen that is a T cell antigen is also a B cell antigen. In other embodiments, the T cell antigen is not also a B cell antigen. B cell antigens include, but are not limited to proteins, peptides, small molecules, carbohydrates, oligosaccharides and polysaccharides. In some embodiments, the B cell antigen comprises a non-protein antigen (i.e., not a protein or peptide antigen). In some embodiments, the B cell antigen comprises a carbohydrate, oligosaccharide or polysaccharide associated with an infectious agent. In some embodiments, the B cell antigen comprises a glycoprotein or glycopeptide associated with an infectious agent. The infectious agent can be a bacterium, virus, fungus, protozoan, or parasite. In some embodiments, the B cell antigen comprises a poorly immunogenic antigen. In some embodiments, the B cell antigen comprises an abused or addictive substance or a portion or analog thereof. Addictive substances include, but are not limited to, nicotine, a narcotic, a cough suppressant, a tranquilizer, and a sedative. In some embodiments, the B cell antigen comprises a toxin, such as a toxin from a chemical weapon or natural sources. The B cell antigen may also comprise a hazardous environmental agent. In some embodiments, the B cell antigen comprises a self antigen. In other embodiments, the B cell antigen comprises an alloantigen, an allergen, a contact sensitizer, a degenerative disease antigen, a hapten, an infectious disease antigen, a cancer antigen, an atopic disease antigen, an autoimmune disease antigen, an addictive substance, a xenoantigen, or a metabolic disease enzyme or enzymatic product thereof.

"Couple" or "Coupled" or "Couples" (and the like) means to chemically associate one entity (for example a moiety) with another. In some embodiments, the coupling is covalent, meaning that the coupling occurs in the context of the presence of a covalent bond between the two entities. In non-covalent embodiments, the non-covalent coupling is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. In embodiments, encapsulation is a form of coupling.

"Derived" means adapted or modified from the original source. For example, as a non-limiting example, a peptide antigen derived from an infectious strain may have several non-natural amino acid residues substituted for the natural amino acid residues found in the original antigen found in the infectious strain. The adaptations or modifications may be for a variety of reasons, including but not limited to increased specificity, easier antigen processing, or improved safety.

In embodiments, a peptide or nucleic acid with a sequence with only 50% identity to a natural peptide or nucleic acid, preferably a natural consensus peptide or nucleic acid, would be said to be derived from the natural peptide or nucleic acid. In other embodiments, the material is substantially modified. Substantially modified material means a material that is modified such that the modification significantly affects the chemical or immunological properties of the material in question. Derived peptides and nucleic acids can also include those with a sequence with greater than 50% identity to a natural peptide or nucleic acid sequence if said derived peptides and nucleic acids have altered chemical or immunological properties as compared to the natural peptide or nucleic acid. These chemical or immunological properties comprise hydrophilicity, stability, affinity, and ability to couple with a carrier such as a synthetic nanocarrier.

"Dosage form" means a pharmacologically and/or immunologically active material, such as a vaccine, in a medium, carrier, vehicle, or device suitable for administration to a subject.

"Encapsulate" or "encapsulated" means to enclose within a synthetic nanocarrier, preferably enclose completely within a synthetic nanocarrier. Most or all of a substance that is encapsulated is not exposed to the local environment external to the synthetic nanocarrier. Encapsulation is distinct from adsorption, which places most or all of a substance on a surface of a synthetic nanocarrier, and leaves the substance exposed to the local environment external to the synthetic nanocarrier.

"Immunologically different" refers to a difference between certain surface antigens that can be noted if a sera generated by immunization generates a distinct antibody response spectrum for each of the surface antigens. Surface antigen specific antibodies will only recognize a specific set of surface antigens, and will bind in distinguishable binding patterns to other sets of surface antigens. For example, if immunized with a set of surface antigens A, the antiserum generated will bind to the set of surface antigens A, but not to the set of surface antigens B. If two or more surface antigens are combined on a single synthetic nanocarrier, a panning assay can be designed which will distinguish the binding patterns of the sera relative to the two sets of surface antigens. In embodiments, a first set of surface antigens and a second set of surface antigens are immunologically different. In other embodiments, a first set of surface antigens, a second set of surface antigens, and a third set of surface antigens all are immunologically different.

An "infection" or "infectious disease" is any condition or disease caused by a microorganism, pathogen or other agent, such as a bacterium, fungus, prion or virus. The surface antigens if the inventive compositions provided herein can be obtained or derived from any infectious agent, such as those that can cause the infections or infectious diseases provided herein.

"Infectious genus" means a genus that comprises organisms capable of infecting a subject. In embodiments, surface antigens may be obtained or derived from a first infectious genus, or obtained or derived from a second infectious genus. In embodiments, the first infectious genus and the second infectious genus are the same. In other embodiments, the first infectious genus and the second infectious genus are different.

"Infectious species" means a species that comprises organisms capable of infecting a subject. In embodiments, surface antigens may be obtained or derived from a first infectious species, or obtained or derived from a second infectious species. In embodiments, the first infectious species and the second infectious species are of the same genus. In other embodiments, the first infectious species and the second infectious species are also the same. In some embodiments, the first infectious species and the second infectious species are different but are of the same genus. In other embodiments, the different infectious species are of different genera.

"Infectious strain" means a strain that comprises organisms capable of infecting a subject. In embodiments, surface antigens may be obtained or derived from a first infectious strain, or obtained or derived from a second infectious strain. In embodiments, the first infectious strain and the second infectious strain are of the same species. In other embodiments, the first infectious strain and the second infectious strain are also the same. In still other embodiments, they are of the same species but are of a different strain. In some embodiments, the first infectious strain and the second infectious strain are of different species but of the same genus.

"Isolated nucleic acid" means a nucleic acid that is separated from its native environment and present in sufficient quantity to permit its identification or use. An isolated nucleic acid may be one that is (i) amplified in vitro by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. Any of the nucleic acids provided herein may be isolated. In some embodiments, the antigens in the compositions provided herein are present in the form of an isolated nucleic acid, such as an isolated nucleic acid that encodes an antigenic peptide, polypeptide or protein.

"Isolated peptide, polypeptide or protein" means the peptide, polypeptide or protein is separated from its native environment and present in sufficient quantity to permit its identification or use. This means, for example, the peptide, polypeptide or protein may be (i) selectively produced by expression cloning or (ii) purified as by chromatography or electrophoresis. Isolated peptides, polypeptides or proteins may be, but need not be, substantially pure. Because an isolated peptide, polypeptide or protein may be admixed with a pharmaceutically acceptable carrier in a pharmaceutical preparation, the peptide, polypeptide or protein may comprise only a small percentage by weight of the preparation. The peptide, polypeptide or protein is nonetheless isolated in that it has been separated from the substances with which it may be associated in living systems, i.e., isolated from other peptides, polypeptides or proteins. Any of the peptidse, polypeptides or proteins provided herein may be isolated. In some embodiments, the antigens in the compositions provided herein are peptides, polypeptides or proteins.

"Maximum dimension of a synthetic nanocarrier" means the largest dimension of a nanocarrier measured along any axis of the synthetic nanocarrier. "Minimum dimension of a synthetic nanocarrier" means the smallest dimension of a synthetic nanocarrier measured along any axis of the synthetic nanocarrier. For example, for a spheroidal synthetic nanocarrier, the maximum and minimum dimension of a synthetic nanocarrier would be substantially identical, and would be the size of its diameter. Similarly, for a cuboidal synthetic nanocarrier, the minimum dimension of a synthetic nanocarrier would be the smallest of its height, width or length, while the maximum dimension of a synthetic nanocarrier would be the largest of its height, width or length. In an embodiment, a minimum dimension of at least 75%, preferably at least 80%, more preferably at least 90%, of the synthetic nanocarriers in a sample, based on the total number of synthetic nanocarriers in the sample, is greater than 100 nm. In a embodiment, a maximum dimension of at least 75%, preferably at least 80%, more preferably at least 90%, of the synthetic nanocarriers in a sample, based on the total number of synthetic nanocarriers in the sample, is equal to or less than 5 µm. Preferably, a minimum dimension of at least 75%, preferably at least 80%, more preferably at least 90%, of the synthetic nanocarriers in a sample, based on the total number of synthetic nanocarriers in the sample, is equal to or greater than 110 nm, more preferably equal to or greater than 120 nm, more preferably equal to or greater than 130 nm, and more preferably still equal to or greater than 150 nm. Aspects ratios of the maximum and minimum dimensions of inventive synthetic nanocarriers may vary depending on the embodiment. For instance, aspect ratios of the maximum to minimum dimensions of the synthetic nanocarriers may vary from 1:1 to 1,000,000:1, preferably from 1:1 to 100,000:1, more preferably from 1:1 to 1000:1, still preferably from 1:1 to 100:1, and yet more preferably from 1:1 to 10:1. Preferably, a maximum dimension of at least 75%, preferably at least 80%, more preferably at least 90%, of the synthetic nanocarriers in a sample, based on the total number of synthetic nanocarriers in the sample is equal to or less than 3 µm, more preferably equal to or less than 2 µm, more preferably equal to or less than 1 µm, more preferably equal to or less than 800 nm, more preferably equal to or less than 600 nm, and more preferably still equal to or less than 500 nm. In preferred embodiments, a maximum dimension of at least 75%, preferably at least 80%, more preferably at least 90%, of the synthetic nanocarriers in a sample, based on the total number of synthetic nanocarriers in the sample, is equal to or greater than 100nm, more preferably equal to or greater than 120 nm, more preferably equal to or greater than 130 nm, more preferably equal to or greater than 140 nm, and more preferably still equal to or greater than 150 nm. Measurement of synthetic nanocarrier sizes is obtained by suspending the synthetic nanocarriers in a liquid (usually aqueous) media and using dynamic light scattering (e.g. using a Brookhaven ZetaPALS instrument).

"Molecular weight less than 10,000" means a molecular weight calculated based on the molecular structure of a molecule of less than 10,000.

"Obtained" means taken from a source without substantial modification. Substantial modification is modification that significantly affects the chemical or immunological properties of the material in question. For example, as a non-limiting example, a peptide or nucleic acid with a sequence with greater than 90%, preferably greater than 95%, preferably greater than 97%, preferably greater than 98%, preferably greater than 99%, preferably 100%, identity to a natural peptide or nucleotide sequence, preferably a natural consensus peptide or nucleotide sequence, and chemical and/or immunological properties that are not significantly different from the natural peptide or nucleic acid would be said to be obtained from the natural peptide or nucleotide sequence. These chemical or immunological properties comprise hydrophilicity, stability, affinity, and ability to couple with a carrier such as a synthetic nanocarrier. In embodiments, the obtained material has been taken from the original source and has not been adapted or modified. For example, in embodiments, antigens obtained from a source may comprise the original amino acid residue sequence found in that source. In other embodiments, for example, antigens obtained from a source may comprise the original molecular structure found in that source.

"Oligosaccharide(s)" means a saccharide polymer containing a small number (typically two to twenty) of saccharide units linked by glycosidic bonds. At a high number of saccharide units, oligosaccharides may comprise polysaccharides.

"Peptide(s)" means compounds comprising amino acid residues joined together primarily by peptide bonds between the carboxyl and amino groups of adjacent amino acid residues, and possessing 100 or less amino acid residues. Certain of the peptide bonds in peptide may be replaced by other bond types, for various purposes, such as stabilization or coupling.

"Pharmaceutically acceptable excipient (or carrier)" means a pharmacologically inactive material used together with the recited synthetic nanocarriers to formulate the inventive compositions. Pharmacologically inactive materials can be added to an inventive dosage form to further facilitate administration of the composition. Pharmaceutically acceptable excipients comprise a variety of materials known in the art, including but not limited to saccharides (such as glucose, lactose, and the like), preservatives such as antimicrobial agents, reconstitution aids, colorants, saline (such as phosphate buffered saline), and buffers. Examples, without limitation, of pharmaceutically acceptable excipients include calcium carbonate, calcium phosphate, various diluents, various sugars and types of starch, cellulose derivatives, gelatin, vegetable oils, polyethylene glycols, preservatives, various pharmaceutical carriers, sterile saline, lyophilization stabilizers, and the like. The compositions may be made using conventional pharmaceutical manufacturing and compounding techniques to arrive at useful dosage forms. In an embodiment, inventive synthetic nanocarriers are suspended in sterile saline solution for injection together with a preservative.

"Polysaccharide(s)" means a saccharide polymer made of many saccharide units linked by glycosidic bonds. At a low number of saccharide units, polysaccharides may comprise oligosaccharides.

"Population" means a defined group of synthetic nanocarriers that share one or more common physical or chemical characteristics. Common physical or chemical characteristics may comprise having a common set of surface antigens, common coupled adjuvant(s), common materials making up the bulk nanocarrier, a common shape, a common particle size, and the like. Multiple populations of synthetic nanocarriers may be identified, for example a first population, a second population, a third population, a fourth population, and the like. In an embodiment, three or more populations of synthetic nanocarriers may be present, preferably wherein each population of synthetic nanocarriers comprises a set of surface antigens; and wherein each set of surface antigens is structurally or immunologically different from one another.

"Protein(s)" means compounds, typically having a molecular weight greater than 1000 daltons, comprising amino acid residues joined together primarily by peptide bonds between the carboxyl and amino groups of adjacent amino acid residues. Proteins may also comprise additional bonding structures such as secondary structures, tertiary structures, and the like. Certain of the peptide bonds in proteins may be replaced by other bond types, for various purposes, such as stabilization or coupling.

"Set of monovalent surface antigens" means a set of surface antigens in which the surface antigens are not different, preferably not different either structurally and/or immunologically. In embodiments, the set of monovalent surface antigens is composed of multiple copies of one type of surface antigen that is not different structurally or immunologically (i.e., multiple copies of the same antigen). The multiple copies of this same antigen can be in some embodiments strung together, such as that illustrated in US Publication 2003/0223938. A set of monovalent surface antigens that is composed of multiple copies of one type of surface antigen that is not different structurally or immunologically is not a set of oligovalent (or multivalent) surface antigens.

"Set of oligovalent (or multivalent) surface antigens" means a set of surface antigens in which there are a limited number, that is greater than one, of different types of surface antigens, preferably wherein the difference comprises structural difference and/or immunological difference. In preferred embodiments, the limited number of surface antigens in the set comprise 2 to 15 types of surface antigens, preferably 2 to 10 types of surface antigens, more preferably 2 to 8 types of surface antigens, more preferably 2 to 7 types of surface antigens, more preferably 2 to 6 types of surface antigens, more preferably 2 to 5 types of surface antigens, more preferably 2 to 4 types of surface antigens, more preferably 2 to 3 types of surface antigens, and even more preferably 2 types of surface antigens. In other embodiments, the set of oligovalent (or multivalent surface antigens) comprise at least 2, 3, 4, 5, 6, 7, 8, 9, 10, 11, 12, 13, 14, 15, 16, 17, 18, 19 or 20 or more types of surface antigens.

"Set of surface antigens" means a group of surface antigens that are identified, preferably identified through measurement and/or prediction, based on their properties, preferably their structural and/or their immunological properties. A set of surface antigens may be identified, in part or in whole, based on prediction using the chemical synthetic methods used to synthesize, along with the chemical methods used to couple, the set of surface antigens and/or the population of synthetic nanocarriers of which the set of surface antigens are comprised. Multiple sets of surface antigens can be identified; e.g. a first set, a second set, a third set, and so on.

"Structurally different" or "structural difference" means presenting different molecular structures for interaction with a B cell receptor. In embodiments, this difference can be expressed by comparing the prevalence and types of presented antigens in a set of surface antigens to the prevalence and types of presented antigens in a different set of surface antigens. If the prevalence and/or types of presented antigens are different between the sets, then the sets of surface antigens can be said to be structurally different. In embodiments, the difference in the prevalence and/or types of presented antigens can be ascertained by comparison of the chemical synthetic strategies and formulation strategies used to generate the surface antigens and/or couple the surface antigens to a surface of the synthetic nanocarriers. For example, in an embodiments, if a set of surface antigens was generated using a particular chemical compound or compounds, and another set of surface antigens was generated using a different chemical compound or compounds, then the two sets of surface antigens could be ascertained to be different. In a different embodiment, if surface antigens were generated using three chemical compounds to form a set of three surface antigens, and other surface antigens were generated using two chemical compounds to form a set of two surface antigens, then the two sets of surface antigens could be ascertained to be different. In yet another embodiment, if non-differing chemical synthetic strategies and formulation strategies (including using non-different amounts of materials --within experimental error-- in the strategies) are used to generate two sets of surface antigens and (as appropriate) couple the two sets of surface antigens to surfaces of synthetic nanocarriers, and the two sets of surface antigens possessed the same conformation and orientation, then the two sets of surface antigens likely would not be structurally different. In embodiments, the structural difference between a first set of surface antigens and a second set of surface antigens comprises non-differing sets of molecules that are presented in orientations that differ between the first and second sets of surface antigens. In embodiments, the structural difference between a first set of surface antigens and a second set of surface antigens comprises non-differing sets of molecules that are presented in conformations that differ between the first and second sets of surface antigens. In embodiments, the structural difference between a first set of surface antigens and a second set of surface antigens comprises sets of molecules whose molecular structure is different between the first and second sets of surface antigens.

"Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like.

"Surface antigen(s)" means an antigen found on or around a surface of a synthetic nanocarrier. In preferable embodiments, surface antigens comprise B cell antigens. In embodiments, surface antigens are coupled to a surface of the synthetic nanocarriers.

"Synthetic nanocarrier(s)" means a discrete object that is not found in nature, and that possesses at least one dimension that is less than or equal to 5 microns in size. Albumin nanoparticles are generally included as synthetic nanocarriers, however in certain embodiments the synthetic nanocarriers do not comprise albumin nanoparticles. In embodiments, the inventive synthetic nanocarriers do not comprise chitosan.

A synthetic nanocarrier can be, but is not limited to, one or a plurality of lipid-based nanoparticles(e.g. liposomes) (also referred to herein as lipid nanoparticles, i.e., nanoparticles where the majority of the material that makes up their structure are lipids), polymeric nanoparticles, metallic nanoparticles, surfactant-based emulsions, dendrimers, buckyballs, nanowires, virus-like particles(i.e., particles that are primarily made up of viral structural proteins but that are not infectious or have low infectivity), peptide or protein-based particles (also referred to herein as protein particles, i.e., particles where the majority of the material that makes up their structure are peptides or proteins) (such as albumin nanoparticles) and/or nanoparticles that are developed using a combination of nanomaterials such as lipid-polymer nanoparticles. Synthetic nanocarriers may be a variety of different shapes, including but not limited to spheroidal, cuboidal, pyramidal, oblong, cylindrical, toroidal, and the like. Synthetic nanocarriers according to the invention comprise one or more surfaces. Exemplary synthetic nanocarriers that can be adapted for use in the practice of the present invention comprise: (1) the biodegradable nanoparticles disclosed in US Patent 5,543,158 to Gref et al., (2) the polymeric nanoparticles of Published US Patent Application 20060002852 to Saltzman et al., (3) the lithographically constructed nanoparticles of Published US Patent Application 20090028910 to DeSimone et al., (4) the disclosure of WO 2009/051837 to von Andrian et al., (5) the nanoparticles disclosed in Published US Patent Application 2008/0145441 to Penades et al., (6) the protein nanoparticles disclosed in Published US Patent Application 20090226525 to de los Rios et al., (7) the virus-like particles disclosed in published US Patent Application 20060222652 to Sebbel et al., (8) the nucleic acid coupled virus-like particles disclosed in published US Patent Application 20060251677 to Bachmann et al., (9) the virus-like particles disclosed in WO2010047839A1 or WO2009106999A2, or (10) the nanoprecipitated nanoparticles disclosed in P. Paolicelli et al., "Surface-modified PLGA-based Nanoparticles that can Efficiently Associate and Deliver Virus-like Particles" Nanomedicine. 5(6):843-853 (2010). In embodiments, synthetic nanocarriers may possess an aspect ratio greater than 1:1, 1:1.2, 1:1.5, 1:2, 1:3, 1:5, 1:7, or greater than 1:10.

Synthetic nanocarriers according to the invention that have a minimum dimension of equal to or less than about 100 nm, preferably equal to or less than 100 nm, do not comprise a surface with hydroxyl groups that activate complement or alternatively comprise a surface that consists essentially of moieties that are not hydroxyl groups that activate complement. In a preferred embodiment, synthetic nanocarriers according to the invention that have a minimum dimension of equal to or less than about 100 nm, preferably equal to or less than 100 nm, do not comprise a surface that substantially activates complement or alternatively comprise a surface that consists essentially of moieties that do not substantially activate complement. In a more preferred embodiment, synthetic nanocarriers according to the invention that have a minimum dimension of equal to or less than about 100 nm, preferably equal to or less than 100 nm, do not comprise a surface that activates complement or alternatively comprise a surface that consists essentially of moieties that do not activate complement. In embodiments, synthetic nanocarriers exclude virus-like particles. In embodiments, when synthetic nanocarriers comprise virus-like particles, the virus-like particles comprise non-natural adjuvant (meaning that the VLPs comprise an adjuvant other than naturally occurring RNA generated during the production of the VLPs). In embodiments, synthetic nanocarriers may possess an aspect ratio greater than 1:1, 1:1.2, 1:1.5, 1:2, 1:3, 1:5, 1:7, or greater than 1:10.

"T cell antigen" means any antigen that is recognized by and triggers an immune response in a T cell (e.g., an antigen that is specifically recognized by a T cell receptor on a T cell or an NKT cell via presentation of the antigen or portion thereof bound to a Class I or Class II major histocompatability complex molecule (MHC), or bound to a CD1 complex. In some embodiments, an antigen that is a T cell antigen is also a B cell antigen. In other embodiments, the T cell antigen is not also a B cell antigen. T cell antigens generally are proteins or peptides. T cell antigens may be an antigen that stimulates a CD8+ T cell response, a CD4+ T cell response, or both. The nanocarriers, therefore, in some embodiments can effectively stimulate both types of responses.

In some embodiments the T cell antigen is a 'universal' T cell antigen, or T cell memory antigen, (i.e., one to which a subject has a pre-existing memory and that can be used to boost T cell help to an unrelated antigen, for example an unrelated B cell antigen). Universal T cell antigens include tetanus toxoid, as well as one or more peptides derived from tetanus toxoid, Epstein-Barr virus, or influenza virus. Universal T cell antigens also include a components of influenza virus, such as hemagglutinin, neuraminidase, or nuclear protein, or one or more peptides derived therefrom. In some embodiments, the universal T cell antigen is not one that is presented in a complex with a MHC molecule. In some embodiments, the universal T cell antigen is not complexed with a MHC molecule for presentation to a T helper cell. Accordingly, in some embodiments, the universal T cell antigen is not a T helper cell antigen. However, in other embodiments, the universal T cell antigen is a T helper cell antigen.

In embodiments, a T-helper cell antigen may comprise one or more peptides obtained or derived from tetanus toxoid, Epstein-Barr virus, influenza virus, respiratory syncytial virus, measles virus, mumps virus, rubella virus, cytomegalovirus, adenovirus, diphtheria toxoid, or a PADRE peptide (known from the work of Sette et al. US Patent 7,202,351). In other embodiments, a T-helper cell antigen may comprise ovalbumin or a peptide obtained or derived therefrom. Preferably, the ovalbumin comprises the amino acid sequence as set forth in Accession No. AAB59956, NP_990483.1, AAA48998, or CAA2371. In other embodiments, the peptide obtained or derived from ovalbumin comprises the following amino acid sequence: H-Ile-Ser-Gln-Ala-Val-His-Ala-Ala-His-Ala-Glu-Ile-Asn-Glu-Ala-Gly-Arg-OH (SEQ ID NO: 1). In other embodiments, a T-helper cell antigen may comprise one or more lipids, or glycolipids, including but not limited to: α-galactosylceramide (α - GalCer), α -linked glycosphingolipids (from Sphingomonas spp.), galactosyl diacylglycerols (from Borrelia burgdorferi), lypophosphoglycan (from Leishmania donovani), and phosphatidylinositol tetramannoside (PIM4) (from Mycobacterium leprae). For additional lipids and/or glycolipids useful as T-helper cell antigen, see V. Cerundolo et al., "Harnessing invariant NKT cells in vaccination strategies." Nature Rev Immun, 9:28-38 (2009).

In embodiments, CD4+ T-cell antigens may be derivatives of a CD4+ T-cell antigen that is obtained from a source, such as a natural source. In such embodiments, CD4+ T-cell antigen sequences, such as those peptides that bind to MHC II, may have at least 70%, 80%, 90%, or 95% identity to the antigen obtained from the source. In embodiments, the T cell antigen, preferably a universal T cell antigen or T-helper cell antigen, may be coupled to, or uncoupled from, a synthetic nanocarrier. In some embodiments, the universal T cell antigen or T-helper cell antigen is encapsulated in the synthetic nanocarriers of the inventive compositions.

"Types of surface antigens," "surface antigen types," etc. means a defined group of surface antigens that share one or more common chemical and/or immunological characteristics.

"Vaccine" means a composition of matter that improves the immune response to a particular pathogen or disease. A vaccine typically contains factors that stimulate a subject's immune system to recognize a specific antigen as foreign and eliminate it from the subject's body. A vaccine also establishes an immunologic 'memory' so the antigen will be quickly recognized and responded to if a person is re-challenged. Vaccines can be prophylactic (for example to prevent future infection by any pathogen), or therapeutic (for example a vaccine against a tumor specific antigen for the treatment of cancer). In embodiments, a vaccine may comprise dosage forms according to the invention.

### NANOCARRIER POPULATIONS AND SETS OF SURFACE ANTIGENS

In embodiments, populations of synthetic nanocarriers share common physical or chemical characteristics. In embodiments, such common physical or chemical characteristics may comprise a common set of surface antigens, common coupled adjuvant(s), common materials making up the bulk nanocarrier, a common shape, a common particle size, common surface charge, and the like. Types of adjuvants, materials, shapes and particle sizes are discussed throughout the present application.

In embodiments, a population may share a set of common surface antigens. These common surface antigens may be grouped together based on common physical or chemical characteristics, such as, but not limited to, structural or immunological properties. In embodiments, the common characteristics may comprise common orientation or conformation, or sets of molecules sharing a common molecular structure, or all of the foregoing. In embodiments, common surface antigens may comprise those having a molecular weight less than 10,000. In other embodiments, common surface antigens may comprise being peptides, proteins, oligosaccharides, polysaccharides, or small molecules. In still other embodiments, common surface antigens may comprise those having a molecular weight less than 10,000 and that comprise being peptides, proteins, oligosaccharides, polysaccharides, or small molecules. In other embodiments, the common surface antigens may be grouped together based on the infectious organisms that they were obtained or derived from; and would be categorized as sharing a common genus, species, and/or strain. In embodiments wherein the surface antigens have a molecular weight less than 10,000, common surface antigens may be grouped together based on classes of molecules such as chemical warfare agents, environmental toxins, addictive or abused substances, and physiologically endogenous molecules including but not limited to hormones, lipids and neurotransmitters. In embodiments, sets of common surface antigens may be defined by the strength of their ability to induce an antibody response in vivo. For example one set of surface antigens may have the ability to ability to induce high affinity antibody production in vivo, while another set of common surface antigens may induce low affinity antibody production in vivo.

In embodiments, a set of surface antigens (e.g., a first and/or second set of surface antigens) can comprise antigens obtained or derived from an infectious agent. In some embodiments, the infectious agent is a bacterium, fungus, virus, protozoan, or parasite. In other embodiments, the virus is a pox virus, smallpox virus, ebola virus, marburg virus, measles virus (in embodiments, the antigen can be obtained or derived from hemagglutinin protein, hemagglutinin noose epitope, hemagglutining amino acids 106-114 and/or 519-550, etc.), dengue fever virus, influenza virus, influenza A virus (in embodiments, the antigen can be obtained or derived from HA protein, M2e protein, etc.), influenza H5N1 virus, influenza H1N1 virus, infectious salmon anemia virus, parainfluenza virus, respiratory syncytial virus, rubeola virus, human immunodeficiency virus, human papillomavirus, varicella-zoster virus, herpes simplex virus, cytomegalovirus, Epstein-Barr virus, JC virus, rhabdovirus, rotavirus, rhinovirus, adenovirus, papillomavirus (in embodiments, the antigen can be obtained or derived from L1 or L2 protein), parvovirus, picornavirus, poliovirus, virus that causes mumps, virus that causes rabies, reovirus, rubella virus, togavirus, orthomyxovirus, retrovirus, hepadnavirus, coxsackievirus, equine encephalitis virus, tick-borne encephalitis, Japanese encephalitis virus, yellow fever virus, Rift Valley fever virus, hepatitis A virus, hepatitis B virus, hepatitis C virus, hepatitis D virus, or hepatitis E virus.

In embodiments, a set of surface antigens (e.g., a first and/or second set of surface antigens) comprises or is obtained or derived from a virus of a family of viruses shown below in Table 1. In another embodiment, a set of surface antigens (e.g., a first and/or second set of surface antigens) comprises or is obtained or derived from a virus of a species provided in Table 1. In still another embodiment, a set of surface antigens (e.g., a first and/or second set of surface antigens) comprises or is obtained or derived from an antigen provided in Table 1.

**Table 1: Viral Infectious Agents**

| **Family** | **Exemplary Species** | **Exemplary Antigens** |
|---|---|---|
| Adenoviridae | adenovirus | VI, VII, E1A, E3-19K, 52K |
| Picornaviridae | coxsackievirus,, | VP1 |
| | hepatitis A virus | Surface antigen |
| | poliovirus, | 3A protein, capsid protein |
| | Rhinovirus (e.g., type 16) | nucleocapsid, surface projection, and transmembrane proteins |
| Herpesviridae | Herpes simplex (type 1 and type 2) | Capsid proteins (e.g., UL6, UL18, UL35, UL38, and UL19) |
| | Varicella-zoster virus | Early antigen |
| | Epstein-barr virus, | Early antigen, capsid antigen |
| | Human cytomegalovirus | Pp65, gB, p52 |
| | Human herpesvirus, (e.g., type 8) | Latent nuclear antigen-1 |
| Hepadnaviridae | Hepatitis B virus | surface antigen |
| Flaviviridae | Hepatitis C virus, yellow fever virus, dengue virus, West Nile virus | NS3, Envelop protein (e.g., E2 domain) |
| Retroviridae | HIV | gp120, p24, and lipopeptides Gag (17-35), Gag (253-284), Nef (66-97), Nef (116-145), and Pol (325-355); see Roberts et al., J. Immunol. Methods, 365(1-2):27-37, 2011 |
| Orthomyxoviridae | Influenza virus | neuraminidase, surface antigen, |
| | Measles virus, | Nucleocapsid protein, matrix protein, phosphoprotein, fusion protein, hemagglutinin, hemagglutinin-neuraminidase, glycoprotein, |
| | Mumps virus | |
| | Parainfluenza virus | |
| Paramyxoviridae | Respiratory syncytial virus | |
| | Human metapneumovirus | |
| Papillomaviridae | Human papillomavirus (e.g., type 16 and 18) | E6, E7, capsid antigen |
| Rhabdoviridae | Rabies virus | Envelope lipoprotein |
| Togaviridae | Rubella virus | Capsid protein |
| Paroviridae | Human bocarivus, Parvovirus B19 | Capsid protein, non-structural protein (NS) |

In other embodiments, a set of surface antigens (e.g., a first and/or second set of surface antigens) can comprise antigens obtained or derived from bacterial organisms such as Borrelia species, Bacillus anthracis, Borrelia burgdorferi, Bordetella pertussis, Bordetella parapertussis, Camphylobacter jejuni, Chlamydia species, Chlamydial psittaci, Chlamydial trachomatis, Clostridium species, Clostridium tetani, Clostridium botulinum, Clostridium perfringens, Corynebacterium diphtheriae, Coxiella species, an Enterococcus species, Erlichia species, Escherichia coli, Francisella tularensis, Haemophilus species, Haemophilus influenzae, Haemophilus parainfluenzae, Lactobacillus species, a Legionella species, Legionella pneumophila, Leptospirosis interrogans, Listeria species, Listeria monocytogenes, Mycobacterium species, Mycobacterium tuberculosis, Mycobacterium leprae, Mycoplasma species, Mycoplasma pneumoniae, Neisseria species, Neisseria meningitidis, Neisseria gonorrhoeae, Pneumococcus species (e.g., type 6A, 6B, 3, 4, 14, 19F, etc.), Pseudomonas species, Pseudomonas aeruginosa, Salmonella species, Salmonella typhi, Salmonella enterica, Rickettsia species, Rickettsia ricketsii, Rickettsia typhi, Shigella species, Staphylococcus species, Staphylococcus aureus, Streptococcus species, Streptococccus pnuemoniae, Streptococcus pyrogenes, Streptococcus mutans, Treponema species, Treponema pallidum, a Vibrio species, Vibrio cholerae, Yersinia pestis, and the like.

In embodiments, a set of surface antigens (e.g., a first and/or second set of surface antigens) comprises or is obtained or derived from a bacteria of a genera of bacteria shown below in Table 2. In another embodiment, a set of surface antigens (e.g., a first and/or second set of surface antigens) comprises or is obtained or derived from a bacterial species provided in Table 2. In still another embodiment, a set of surface antigens (e.g., a first and/or second set of surface antigens) comprises or is obtained or derived from an antigen provided in Table 2.

**Table 2: Bacterial Infectious Agents**

| **Pathogenic Bacterial Genera** | **Exemplary Species** | **Exemplary Antigens** |
|---|---|---|
| Bordetella | Bordetella pertussis | pertussis toxin (PT), filamentous hemagglutinin (FHA), pertactin (PRN), and fimbriae (FIM 2/3) |
| Borrelia | Borrelia burgdorferi | VlsE; DbpA and OspA |
| Brucella | Brucella abortus | Hia, PrpA, MltA, L7/L12, D15, 0187, VirJ, Mdh, AfuA |
| | Brucella canis | L7/L12 |
| | Brucella melitensis | Out membrane proteins such as Omp28 |
| | Brucella suis | |
| Campylobacter | Campylobacter jejuni; | LPS, an 100-kD antigen |
| *Chlamydia* and *Chlamydophila* | Chlamydia pneumoniae | See Richard et al., J. Infectious Diseases. 181:S521 (2000) |
| | Chlamydia trachomatis | |
| | Chlamydophila psittaci | |
| Clostridium | Clostridium botulinum | antigen types A, B, C, D, and E |
| | Clostridium difficile | FliC, FliD, and Cwp84 |
| | Clostridium perfringens | alpha-toxin, theta-toxin, fructose 1,6-biphosphate-aldolase (FBA), glyceraldehydes-3-phosphate dehydrogenase (GPD), pyruvate:ferredoxin oxidoreductase (PFOR), elongation factor-G (EF-G), and a hypothetical protein (HP) |
| | Clostridium tetani | T toxin |
| Corynebacterium | Corynebacterium diphtheriae | Toxoid antigen |
| Enterococcus | Enterococcus faecalis | capsular polysaccharides |
| | Enterococcus faecium | |
| Escherichia | Escherichia coli | See Moriel et al., PNAS 107(20):9072-9077 (2010) |
| Francisella | Francisella tularensis | See Havlasova et al., Proteomics 2(7):857-867, 2002 |
| Haemophilus | Haemophilus influenzae | capsular polysaccharides, Protein D, |
| Helicobacter | Helicobacter pylori | See Bumann et al., Proteomics 4(10):2843-2843, 2004 |
| Legionella | Legionella pneumophila | Mip |
| Leptospira | Leptospira interrogans | See Brown et al., Infect Immu 59(5):1772-1777, 1991 |
| Listeria | Listeria monocytogenes | nucleoprotein (NP) |
| Mycobacterium* | Mycobacterium leprae | |
| | Mycobacterium tuberculosis | RD1, PE35, PPE68, EsxA, EsxB, RD9, and EsxV |
| | Mycobacterium ulcerans | |
| Mycoplasma | Mycoplasma pneumoniae | Hsp70 |
| Neisseria | Neisseria gonorrhoeae | |
| | Neisseria meningitidis | See Litt et al., J. Infectious Disease 190(8):1488-1497, 2004 |
| Pseudomonas | Pseudomonas aeruginosa | Lipopolysaccharides |
| Rickettsia | Rickettsia rickettsii | Surface antigen |
| Salmonella | Salmonella typhi | |
| | Salmonella typhimurium | |
| Shigella | Shigella sonnei | |
| Staphylococcus | Staphylococcus aureus | See Vytvtska et al., Proteomics 2(5):580-590, 2002; Etz et al., PNAS 99(10):6573-6578; 2002 |
| | Staphylococcus epidermidis | |
| | Staphylococcus saprophyticus | |
| Streptococcus | Streptococcus agalactiae | |
| | Streptococcus pneumoniae | Sp1, Sp2, Sp3 |
| | Streptococcus pyogenes | Lei et al., J. Infectious Disease 189(1):79-89, 2004 |
| Treponema | Treponema pallidum | Glycerophosphodiester Phosphodiesterase |
| Vibrio | Vibrio cholerae | Outer membrane proteins such as OmpK |
| Yersinia | Yersinia pestis | Chaperone-usher protein, capsular protein (F1), and V protein |

In still other embodiments, a set of surface antigens (e.g., a first and/or second set of surface antigens) can comprise antigens obtained or derived from antigens of fungal, protozoan, and/or parasitic organisms such as Aspergillus species, Candida species, Candida albicans, Candida tropicalis, Cryptococcus species, Cryptococcus neoformans, Entamoeba histolytica, Histoplasma capsulatum, Leishmania species, Nocardia asteroides, Plasmodium falciparum, Toxoplasma gondii, Trichomonas vaginalis, Toxoplasma species, Trypanosoma brucei, Schistosoma mansoni, and the like.

In still other embodiments, a set of surface antigens (e.g., a first and/or second set of surface antigens) can comprise antigens obtained or derived from a toxin, such as O-Alkyl (<C10, incl. cycloalkyl) alkyl (Me, Et, n-Pr or i-Pr)-phosphonofluoridates (e.g. Sarin: O-Isopropyl methylphosphonofluoridate, Soman: O-Pinacolyl methylphosphonofluoridate), O-Alkyl (<C10, incl. cycloalkyl) N,N-dialkyl (Me, Et, n-Pr or i-Pr) phosphoramidocyanidates (e.g. Tabun: O-Ethyl N,N-dimethylphosphoramidocyanidate), O-Alkyl (H or <C10, incl. cycloalkyl) S-2-dialkyl (Me, Et, n-Pr or i-Pr)-aminoethyl alkyl (Me, Et, n-Pr or i-Pr) phosphonothiolates and corresponding alkylated or protonated salts (e.g. VX: O-Ethyl S-2-diisopropylaminoethyl methylphosphonothiolate), Sulfur mustards: 2-Chloroethylchloromethylsulfide, Mustard gas: Bis(2-chloroethyl)sulfide, Bis(2-chloroethylthio)methane, Sesquimustard: 1,2-Bis(2-chloroethylthio)ethane, 1,3-Bis(2-chloroethylthio)-n-propane, 1,4-Bis(2-chloroethylthio)-n-butane, 1,5-Bis(2-chloroethylthio)-n-pentane, Bis(2-chloroethylthiomethyl)ether, O-Mustard: Bis(2-chloroethylthioethyl)ether, Lewisites: Lewisite 1: 2-Chlorovinyldichloroarsine, Lewisite 2: Bis(2-chlorovinyl)chloroarsine, Lewisite 3: Tris(2-chlorovinyl)arsine, Nitrogen mustards: HN1: Bis(2-chloroethyl)ethylamine, HN2: Bis(2-chloroethyl)methylamine, HN3: Tris(2-chloroethyl)amine, Saxitoxin, Ricin, Amiton: O,O-Diethyl S-(2-(diethylamino)ethyl)phosphorothiolate and corresponding alkylated or protonated salts, PFIB: 1,1,3,3,3-Pentafluoro-2-(trifluoromethyl)-1-propene, 3-Quinuclidinyl benzilate (BZ), Phosgene: Carbonyl dichloride, Cyanogen chloride, Hydrogen cyanide and Chloropicrin: Trichloronitromethane.

In other embodiments, a set of surface antigens (e.g., a first and/or second set of surface antigens) comprises or is obtained or derived from a fungus of a genera of fungi shown below in Table 3. In another embodiment, a set of surface antigens (e.g., a first and/or second set of surface antigens) comprises or is obtained or derived from a fungal species provided in Table 3. In still another embodiment, a set of surface antigens (e.g., a first and/or second set of surface antigens) comprises or is obtained or derived from an antigen provided in Table 3.

**Table 3: Fungal Infectious Agents**

| **Genera** | **Exemplary Species** | **Exemplary Antigens** |
|---|---|---|
| Candida | C. albicans | Surface antigens, see also Thomas et al., Proteomics 6(22):6033-6041, 2006 |
| Aspergillus | *Aspergillus fumigatus* and *Aspergillus flavus.* | Stevens et al., Medical Mycology 49 (Suppl. 1):S170-S176, 2011 |
| Cryptococcus | Cryptococcus neoformans, Cryptococcus laurentii and Cryptococcus albidus, Cryptococcus gattii | Capsular glycoproteins, |
| Histoplasma | Histoplasma capsulatum | Yps3P, Hsp60 |
| Pneumocystis | Pneumocystis jirovecii | Major surface proteins (Msg) such as MsgC1, MsgC3, MsgC8, and MsgC9 |
| Stachybotrys | Stachybotrys chartarum | SchS34, |

In still further embodiments, a set of surface antigens (e.g., a first and/or second set of surface antigens) can comprise antigens obtained or derived from an abused or addictive substance. In some embodiments, the abused or addictive substance is a drug, such as an illegal drug, an over-the-counter drug or a prescription drug. In other embodiments, the abused or addictive substance has mood-altering effects, and, therefore, includes inhalants and solvents. In other embodiments, the abused or addictive substance is one that has no mood-altering effects or intoxication properties, and, therefore, includes anabolic steroids. Abused or addictive substances include, but are not limited to, cannabinoids (e.g., hashish, marijuana), depressants (e.g., barbituates, benodiazepines, flunitrazepam (Rohypnol), GHB, methaqualone (quaaludes)), dissociative anesthetics (e.g., ketamine, PCP), hallucinogens (e.g, LSD, mescaline, psilocybin), opioids and morphine derivatives (e.g., codeine, fentanyl, heroin, morphine, opium), stimulants (amphetamine, cocaine, Ecstacy (MDMA), methamphetamine, methylphenidate (Ritalin), nicotine), anabolic steriods, and inhalants. In embodiments, the antigen comprises a cocaine analog, such as norcocaine. In other embodiments, the antigen comprises cotinine.

In embodiments of the present invention, different populations of synthetic nanocarriers that each comprise a set of surface antigens may be combined. The difference between the populations is based on the differences between the sets of surface antigens.

In certain embodiments, these differences can comprise differences in physical or chemical characteristics, such as, but not limited to, structural or immunological properties. In embodiments, the differences may comprise differences in surface antigen orientation or conformation, or differences in molecular structure between sets of surface antigens. In still other embodiments, the difference in surface antigens may be based on the infectious organisms that they were obtained or derived from; and would be categorized as being from a different genus, species, and/or strain. In embodiments wherein the surface antigens have a molecular weight less than 10,000, surface antigens may be different based on chemical classes such as chemical warfare agents, addictive or abused substances, and endogenous molecules including but not limited to hormones, lipids and neurotransmitters.

In embodiments, the differences may comprise differences in surface antigen orientation or conformation. For instance, different points of attachment of a surface antigen to a synthetic nanocarrier would give rise to different presentations of that surface antigen. These different presentations may produce antibodies that recognize different epitopes of the surface antigen. Surface antigens may be presented with different conformations, and may be synthesized or modified to achieve those conformations. For example, peptide or protein truncations may be performed in which results in modified conformational changes in the peptide or protein antigen of interest. Alternatively amino acids or chemical linkers may be added in order to add length or stabilize a specific orientation that alters peptide or protein antigen exposure . Similarly antigens such those as having a molecular weight less than 10,000, or oligosaccharides, or polysaccharides may be altered by addition of a chemical linker, or chemical modification.

In other embodiments, differences between populations of synthetic nanocarriers may be based on differences between sets of surface antigens based on different molecular structures and/or prevalence of the antigens. In some embodiments, the difference may comprise a difference in the prevalence of one or more of the types of surface antigens between the sets.

In embodiments wherein a population comprises a monovalent set of surface antigens, the molecular structure, preferably the antigen type, of its set of surface antigens may be different from the molecular structure of a set of monovalent surface antigens of another population or other populations. In certain embodiments, wherein a population comprises a monovalent set of surface antigens, the prevalence of surface antigens of which its set of surface antigens is comprised may be different from the prevalence of surface antigens of which a set of monovalent surface antigens of another population or other populations is comprised.

In embodiments wherein a population of synthetic nanocarriers comprises a set of oligovalent (or multivalent) surface antigens, various antigen types at different prevalences may be combined within the set to form combinations of such surface antigen types. Accordingly, in embodiments wherein at least one population of synthetic nanocarriers comprises a set of oligovalent (or multivalent) surface antigens, the molecular structure of the set of oligovalent (or multivalent) surface antigens (which can be expressed as a function of both the molecular structure of each type of antigen, along with their prevalence within the set) can be different from the molecular structure of the set of surface antigens of another population or other populations. In embodiments, this may be because another population comprises a monovalent set of surface antigens (wherein the sets of surface antigens would be different by definition) or because another population comprises a set of oligovalent (or multivalent) surface antigens wherein the molecular structures of the two sets of surface antigens (expressed as a molecular structure of each type of antigen and/or a prevalence of each antigen type within the set) are different.

For example the sets of surface antigens can be comprised of a set of enantiomers such as (R) and (S) nicotine. The enantiomers can be present in equal amounts on the same or different nanocarriers and can be present in unequal amounts on the same or different populations of synthetic nanocarriers. In another embodiment, a set of surface antigens may comprise two structurally different but related molecules such as cotinine and nicotine, either optically pure or racemic. The cotinine and nicotine can be present in equal amounts on the same or different populations of synthetic nanocarriers and can be present in unequal amounts on the same or different populations of synthetic nanocarriers. In addition, sets of surface antigens can be comprised of antigens from a single organism comprised of several serotypes such as the capsular antigenic polysaccharides from *Streptococcus Pneumoniae* serotypes 4, 6B, 9V, 14, 18C, 19F, and 23F. The various antigens can be present in equal amounts on the same or different populations of synthetic nanocarriers and can be present in unequal amounts on the same or different populations of synthetic nanocarriers. In embodiments, sets of surface antigens can comprise a family of different antigens from a single organism such as the capsid proteins L1 and L2 of the human papillomavirus. The sets of surface antigens can be present in equal amounts on the same or different populations of synthetic nanocarriers and can be present in unequal amounts on the same or different populations of synthetic nanocarriers. In embodiments, sets of surface antigens can comprise several small molecules of diverse structures such as the war gases VX, sarin and soman. The different compounds can be present in equal amounts on the same or different populations of synthetic nanocarriers and can be present in unequal amounts on the same or different populations of synthetic nanocarriers. For instance, in an embodiment, one set of surface antigens may comprise 50% of VX and 50% sarin, while another set of surface antigens may comprise 80% of VX and 20% sarin, where the percent of surface antigens may be a weight percent or mole percent, and based on the total weight or total number of moles of surface antigens.

In embodiments, differences in sets of surface antigens may comprise providing a population of synthetic nanocarriers that comprises a set of surface antigens from a type or types such as having a molecular weight less than 10,000 and/or being peptides, proteins, oligosaccharides, polysaccharides, or small molecules; and then providing another population of synthetic nanoparticles comprising a different set of surface antigens from a type or types such as having a molecular weight less than 10,000 and/or or being peptides, proteins, oligosaccharides, polysaccharides, or small molecules.

In embodiments, when a first set of surface antigens comprises surface antigens having a molecular weight less than 10,000, a second set of surface antigens comprises peptides, proteins, oligosaccharides, polysaccharides or small molecules (provided the sets are structurally or immunologically different). In embodiments, when a first set of surface antigens comprises surface antigens comprising peptides, a second set of surface antigens comprises surface antigens comprising those having a molecular weight less than 10,000 and/or comprising proteins, oligosaccharides, polysaccharides or small molecules. In embodiments, when a first set of surface antigens comprises surface antigens comprising proteins, a second set of surface antigens comprises surface antigens comprising those having a molecular weight less than 10,000 and/or comprising peptides, oligosaccharides, polysaccharides or small molecules. In embodiments, when a first set of surface antigens comprises surface antigens comprising oligosaccharides, a second set of surface antigens comprises surface antigens comprising those having a molecular weight less than 10,000 and/or comprising peptides, proteins, polysaccharides or small molecules. In embodiments, when a first set of surface antigens comprises surface antigens comprising polysaccharides, a second set of surface antigens comprises surface antigens comprising those having a molecular weight less than 10,000 and/or comprising peptides, proteins, oligosaccharides or small molecules. In embodiments, when a first set of surface antigens comprises surface antigens comprising small molecules, a second set of surface antigens comprises surface antigens comprising those having a molecular weight less than 10,000 and/or comprising peptides, proteins, oligosaccharides or polysaccharides (provided the sets are structurally or immunologically different).

In embodiments, when a second set of surface antigens comprises surface antigens having a molecular weight less than 10,000, a first set of surface antigens comprises peptides, proteins, oligosaccharides, polysaccharides or small molecules (provided the sets are structurally or immunologically different). In embodiments, when a second set of surface antigens comprises surface antigens comprising peptides, a first set of surface antigens comprises surface antigens comprising those having a molecular weight less than 10,000 and/or comprising proteins, oligosaccharides, polysaccharides or small molecules. In embodiments, when a second set of surface antigens comprises surface antigens comprising proteins, a first set of surface antigens comprises surface antigens comprising those having a molecular weight less than 10,000 and/or comprising peptides, oligosaccharides, polysaccharides or small molecules. In embodiments, when a second set of surface antigens comprises surface antigens comprising oligosaccharides, a first set of surface antigens comprises surface antigens comprising those having a molecular weight less than 10,000 and/or comprising peptides, proteins, polysaccharides or small molecules. In embodiments, when a second set of surface antigens comprises surface antigens comprising polysaccharides, a first set of surface antigens comprises surface antigens comprising those having a molecular weight less than 10,000 and/or comprising peptides, proteins, oligosaccharides or small molecules. In embodiments, when a second set of surface antigens comprises surface antigens comprising small molecules, a first set of surface antigens comprises surface antigens comprising those having a molecular weight less than 10,000 and/or comprising peptides, proteins, oligosaccharides or polysaccharides (provided the sets are structurally or immunologically different).

Other differences between populations of synthetic nanocarriers may be based on differences between sets of surface antigens based on the source of the antigens. For instance, in an embodiment, such differences may be based on differences of the infectious genera, species and/or strains from which the surface antigens were obtained or derived. For example, one population of synthetic nanocarriers may comprise a set of surface antigens obtained or derived from a bacterial source, such as E. Coli, mycobacterium tuberculosis, clostridium tetani or bacillus anthracis while another population may comprise a set of surface antigens obtained or derived from a viral source, such as influenza virus, hepatitis B virus, hepatitis C virus, and human herpesvirus. In embodiments, one population of synthetic nanocarriers may comprise a set of surface antigens obtained or derived from a bacterial source, such as those noted above, while another population may comprise a set of surface antigens obtained or derived from fungi such as candida albicans, or pneumocystis jiroveci. In other embodiments, one population of synthetic nanocarriers may comprise a set of surface antigens obtained or derived from a viral source, while another population may comprise a set of surface antigens obtained or derived from parasites such as plasmodium falciparum. Other combinations and sub-combinations along the lines of the illustrations above are contemplated to be within the scope of the present invention.

In other embodiments, the various sets of surfaces antigens may be obtained or derived from infectious genera, species or strains that are not different. In embodiments, those differences may arise from selection of different antigens within the infectious genus, species or strain. For example, one set of surface antigens may be obtained or derived from one viral coat protein, while another set of surface antigens may be obtained or derived from different epitopes from the same or another viral coat protein from the same virus. In an embodiment, different sets of surface antigens may be obtained or derived from one viral protein, for example, the cytomegalovirus (CMV) capsid protein, or other CMV proteins, which may comprise several distinct epitopes. Likewise, different sets of surface antigens may be obtained or derived from different epitopes of diphtheria or tetanus toxin.

In other embodiments, differences between populations of synthetic nanocarriers may be based on differences between sets of surface antigens based on different chemical classes of the antigens. For instance, in the case of molecules having a molecular weight less than 10,000 such differences may be based on differences of the chemical scaffold, or the overall molecular structure, or the activity exhibited by such molecules. For instance, sets of different surface antigens may be obtained or derived from sets of surface antigens of differing structure but with similar activities like opiods such as morphine and heroin. In other embodiments, different sets of surface antigens may be comprised of molecules with similar structures but with differing activities exemplified by enantiomers such as (R) and (S) Ritalin, or (R) and (S) nicotine. In embodiments, difference between sets of surface antigens may comprise compounds and their metabolites such as terfenadine and fexofenadine or astemazole and norastemazole. Differences between sets of surface antigens may also be based on the un-relatedness of the structure of compounds such as nicotine and methamphetamine.

In other embodiments, differences between populations of synthetic nanocarriers may be based on differences between sets of surface antigens based on immunological differences between the sets of surface antigens. In embodiments, sets of surface antigens may be defined by their ability to induce an immune response in vivo. For example, one set of surface antigens may have the ability to induce high levels of high affinity antibody production to an antigen of interest in vivo, while a second set of surface antigens may not induce high levels of high affinity antibody production in vivo to that antigen. As another example a first set of surface antigens may have the ability to generate antibody titers specific to the antigens of the first set of surface antigens, while a second set of surface antigens may have the ability generate antibody titers specific to the antigens of the second set of surface antigens. In embodiments, the second set of surface antigens generate antibody titers specific to the antigens of the second set of surface antigens but not to the antigens of the first set.

### INVENTIVE COMPOSITIONS AND RELATED METHODS

Synthetic nanocarriers may be prepared using a wide variety of methods known in the art. For example, synthetic nanocarriers can be formed by methods as nanoprecipitation, flow focusing using fluidic channels, spray drying, single and double emulsion solvent evaporation, solvent extraction, phase separation, milling, microemulsion procedures, microfabrication, nanofabrication, sacrificial layers, simple and complex coacervation, and other methods well known to those of ordinary skill in the art. Alternatively or additionally, aqueous and organic solvent syntheses for monodisperse semiconductor, conductive, magnetic, organic, and other nanomaterials have been described (Pellegrino et al., 2005, Small, 1:48; Murray et al., 2000, Ann. Rev. Mat. Sci., 30:545; and Trindade et al., 2001, Chem. Mat., 13:3843). Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755, and also US Patents 5578325 and 6007845; P. Paolicelli et al., "Surface-modified PLGA-based Nanoparticles that can Efficiently Associate and Deliver Virus-like Particles" Nanomedicine. 5(6):843-853 (2010)).

In embodiments, the present invention comprises synthetic nanocarrier means for presenting sets of surface antigens, preferably sets of monovalent or oligovalent surface antigens. A particular inventive embodiment comprises a first synthetic nanocarrier means for presenting a first set of surface antigens, preferably a first set of monovalent or oligovalent surface antigens; and a second synthetic nanocarrier means for presenting a second set of surface antigens; preferably a second set of monovalent or oligovalent surface antigens. Such synthetic nanocarrier means for presenting surface antigens are disclosed throughout the present disclosure, and encompass the embodiments disclosed herein.

Various materials may be encapsulated into synthetic nanocarriers as desirable using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006) ; P. Paolicelli et al., "Surface-modified PLGA-based Nanoparticles that can Efficiently Associate and Deliver Virus-like Particles" Nanomedicine. 5(6):843-853 (2010). Other methods suitable for encapsulating materials, such as oligonucleotides, into synthetic nanocarriers may be used, including without limitation methods disclosed in United States Patent 6,632,671 to Unger October 14, 2003.

In certain embodiments, synthetic nanocarriers are prepared by a nanoprecipitation process or spray drying. Conditions used in preparing synthetic nanocarriers may be altered to yield particles of a desired size or property (e.g., hydrophobicity, hydrophilicity, external morphology, "stickiness," shape, etc.). The method of preparing the synthetic nanocarriers and the conditions (e.g., solvent, temperature, concentration, air flow rate, etc.) used may depend on the materials to be coupled to the synthetic nanocarriers and/or the composition of the polymer matrix.

If particles prepared by any of the above methods have a size range outside of the desired range, particles can be sized, for example, using a sieve, differential centrifugation or settling.

Elements of the inventive synthetic nanocarriers such as moieties of which an immunofeature surface is comprised, targeting moieties, polymeric matrices, antigens, adjuvants, and the like may be coupled to the synthetic nanocarrier, e.g., by one or more covalent bonds, or may be coupled by means of one or more linkers. Additional methods of functionalizing synthetic nanocarriers may be adapted from Published US Patent Application 2006/0002852 to Saltzman et al., Published US Patent Application 2009/0028910 to DeSimone et al., or Published International Patent Application WO/2008/127532 A1 to Murthy et al.

Alternatively or additionally, synthetic nanocarriers can be coupled to moieties of which an immunofeature surface is comprised, targeting moieties, adjuvants, antigens and/or other elements directly or indirectly via non-covalent interactions. In non-covalent embodiments, the non-covalent coupling is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. Such couplings may be arranged to be on an external surface or an internal surface of an inventive synthetic nanocarrier. In embodiments, encapsulation and/ or absorption is a form of coupling.

A wide variety of synthetic nanocarriers can be used according to the invention. In some embodiments, synthetic nanocarriers are spheres or spheroids. In some embodiments, synthetic nanocarriers are flat or plate-shaped. In some embodiments, synthetic nanocarriers are cubes or cuboidal. In some embodiments, synthetic nanocarriers are ovals or ellipses. In some embodiments, synthetic nanocarriers are cylinders, cones, or pyramids.

In some embodiments, it is desirable to use a population of synthetic nanocarriers that is relatively uniform in terms of size, shape, and/or composition so that each synthetic nanocarrier has similar properties. For example, at least 80%, at least 90%, or at least 95% of the synthetic nanocarriers, based on the total number of synthetic nanocarriers, may have a minimum dimension or maximum dimension that falls within 5%, 10%, or 20% of the average diameter or average dimension of the synthetic nanocarriers. In some embodiments, a population of synthetic nanocarriers may be heterogeneous with respect to size, shape, and/or composition.

Synthetic nanocarriers can be solid or hollow and can comprise one or more layers. In some embodiments, each layer has a unique composition and unique properties relative to the other layer(s). To give but one example, synthetic nanocarriers may have a core/shell structure, wherein the core is one layer (e.g. a polymeric core) and the shell is a second layer (e.g. a lipid bilayer or monolayer). Synthetic nanocarriers may comprise a plurality of different layers.

In some embodiments, synthetic nanocarriers may optionally comprise one or more lipids. In some embodiments, a synthetic nanocarrier may comprise a liposome. In some embodiments, a synthetic nanocarrier may comprise a lipid bilayer. In some embodiments, a synthetic nanocarrier may comprise a lipid monolayer. In some embodiments, a synthetic nanocarrier may comprise a micelle. In some embodiments, a synthetic nanocarrier may comprise a core comprising a polymeric matrix surrounded by a lipid layer (e.g., lipid bilayer, lipid monolayer, etc.). In some embodiments, a synthetic nanocarrier may comprise a non-polymeric core (e.g., metal particle, quantum dot, ceramic particle, bone particle, viral particle, proteins, nucleic acids, carbohydrates, etc.) surrounded by a lipid layer (e.g., lipid bilayer, lipid monolayer, etc.).

In some embodiments, synthetic nanocarriers can comprise one or more polymers. In some embodiments, such a polymer can be surrounded by a coating layer (e.g., liposome, lipid monolayer, micelle, etc.). In some embodiments, various elements of the synthetic nanocarriers can be coupled with the polymer.

In some embodiments, an immunofeature surface, targeting moiety, antigens, adjuvants, and/or oligonucleotide can be covalently associated with a polymeric matrix. In some embodiments, covalent association is mediated by a linker. In some embodiments, an immunofeature surface, targeting moiety, antigens, adjuvants, and/or oligonucleotide can be noncovalently associated with a polymeric matrix. For example, in some embodiments, an immunofeature surface, targeting moiety, antigens, adjuvants, and/or oligonucleotide can be encapsulated within, surrounded by, and/or dispersed throughout a polymeric matrix. Alternatively or additionally, an immunofeature surface, targeting moiety, antigens, adjuvants, and/or nucleotide can be associated with a polymeric matrix by hydrophobic interactions, charge interactions, van der Waals forces, etc.

A wide variety of polymers and methods for forming polymeric matrices therefrom are known conventionally. In general, a polymeric matrix comprises one or more polymers. Polymers may be natural or unnatural (synthetic) polymers. Polymers may be homopolymers or copolymers comprising two or more monomers. In terms of sequence, copolymers may be random, block, or comprise a combination of random and block sequences. Typically, polymers in accordance with the present invention are organic polymers.

Examples of polymers suitable for use in the present invention include, but are not limited to polyethylenes, polycarbonates (e.g. poly(1,3-dioxan-2one)), polyanhydrides (e.g. poly(sebacic anhydride)), polypropylfumerates, polyamides (e.g. polycaprolactam), polyacetals, polyethers, polyesters (e.g., polylactide, polyglycolide, polylactide-co-glycolide, polycaprolactone, polyhydroxyacids (e.g. poly(P-hydroxyalkanoate))), poly(orthoesters), polycyanoacrylates, polyvinyl alcohols, polyurethanes, polyphosphazenes, polyacrylates, polymethacrylates, polyureas, polystyrenes, polyamines, polylysine, polylysine-PEG copolymers, poly(ethyleneimine), poly(ethylene imine)-PEG copolymers, and polyphosphazines.

In some embodiments, polymers in accordance with the present invention include polymers which have been approved for use in humans by the U.S. Food and Drug Administration (FDA) under 21 C.F.R. § 177.2600, including but not limited to polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates.

In some embodiments, polymers can be hydrophilic. For example, polymers may comprise anionic groups (e.g., phosphate group, sulphate group, carboxylate group); cationic groups (e.g., quaternary amine group); or polar groups (e.g., hydroxyl group, thiol group, amine group). In some embodiments, a synthetic nanocarrier comprising a hydrophilic polymeric matrix generates a hydrophilic environment within the synthetic nanocarrier. In some embodiments, polymers can be hydrophobic. In some embodiments, a synthetic nanocarrier comprising a hydrophobic polymeric matrix generates a hydrophobic environment within the synthetic nanocarrier. Selection of the hydrophilicity or hydrophobicity of the polymer may have an impact on the nature of materials that are incorporated (e.g. coupled) within the synthetic nanocarrier.

In some embodiments, polymers may be modified with one or more moieties and/or functional groups. A variety of moieties or functional groups can be used in accordance with the present invention. In some embodiments, polymers may be modified with polyethylene glycol (PEG), with a carbohydrate, and/or with acyclic polyacetals derived from polysaccharides (Papisov, 2001, ACS Symposium Series, 786:301). Certain embodiments may be made using the general teachings of US Patent No. 5543158 to Gref et al., or WO publication WO2009/051837 by Von Andrian et al.

In some embodiments, polymers may be modified with a lipid or fatty acid group. In some embodiments, a fatty acid group may be one or more of butyric, caproic, caprylic, capric, lauric, myristic, palmitic, stearic, arachidic, behenic, or lignoceric acid. In some embodiments, a fatty acid group may be one or more of palmitoleic, oleic, vaccenic, linoleic, alpha-linoleic, gamma-linoleic, arachidonic, gadoleic, arachidonic, eicosapentaenoic, docosahexaenoic, or erucic acid.

In some embodiments, polymers may be polyesters, including copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some embodiments, exemplary polyesters include, for example, polyhydroxyacids; PEG copolymers and copolymers of lactide and glycolide (e.g., PLA-PEG copolymers, PGA-PEG copolymers, PLGA-PEG copolymers, and derivatives thereof. In some embodiments, polyesters include, for example, poly(caprolactone), poly(caprolactone)-PEG copolymers, poly(L-lactide-co-L-lysine), poly(serine ester), poly(4-hydroxy-L-proline ester), poly[α-(4-aminobutyl)-L-glycolic acid], and derivatives thereof.

In some embodiments, a polymer may be PLGA. PLGA is a biocompatible and biodegradable co-polymer of lactic acid and glycolic acid, and various forms of PLGA are characterized by the ratio of lactic acid:glycolic acid. Lactic acid can be L-lactic acid, D-lactic acid, or D,L-lactic acid. The degradation rate of PLGA can be adjusted by altering the lactic acid:glycolic acid ratio. In some embodiments, PLGA to be used in accordance with the present invention is characterized by a lactic acid:glycolic acid ratio of approximately 85:15, approximately 75:25, approximately 60:40, approximately 50:50, approximately 40:60, approximately 25:75, or approximately 15:85.

In some embodiments, polymers may be one or more acrylic polymers. In certain embodiments, acrylic polymers include, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, polycyanoacrylates, and combinations comprising one or more of the foregoing polymers. The acrylic polymer may comprise fully-polymerized copolymers of acrylic and methacrylic acid esters with a low content of quaternary ammonium groups.

In some embodiments, polymers can be cationic polymers. In general, cationic polymers are able to condense and/or protect negatively charged strands of nucleic acids (e.g. DNA, or derivatives thereof). Amine-containing polymers such as poly(lysine) (Zauner et al., 1998, Adv. Drug Del. Rev., 30:97; and Kabanov et al., 1995, Bioconjugate Chem., 6:7), poly(ethylene imine) (PEI; Boussif et al., 1995, Proc. Natl. Acad. Sci., USA, 1995, 92:7297), and poly(amidoamine) dendrimers (Kukowska-Latallo et al., 1996, Proc. Natl. Acad. Sci., USA, 93:4897; Tang et al., 1996, Bioconjugate Chem., 7:703; and Haensler et al., 1993, Bioconjugate Chem., 4:372) are positively-charged at physiological pH, form ion pairs with nucleic acids, and mediate transfection in a variety of cell lines. In embodiments, the inventive synthetic nanocarriers may not comprise (or may exclude) cationic polymers.

In some embodiments, polymers can be degradable polyesters bearing cationic side chains (Putnam et al., 1999, Macromolecules, 32:3658; Barrera et al., 1993, J. Am. Chem. Soc., 115:11010; Kwon et al., 1989, Macromolecules, 22:3250; Lim et al., 1999, J. Am. Chem. Soc., 121:5633; and Zhou et al., 1990, Macromolecules, 23:3399). Examples of these polyesters include poly(L-lactide-co-L-lysine) (Barrera et al., 1993, J. Am. Chem. Soc., 115:11010), poly(serine ester) (Zhou et al., 1990, Macromolecules, 23:3399), poly(4-hydroxy-L-proline ester) (Putnam et al., 1999, Macromolecules, 32:3658; and Lim et al., 1999, J. Am. Chem. Soc., 121:5633), and poly(4-hydroxy-L-proline ester) (Putnam et al., 1999, Macromolecules, 32:3658; and Lim et al., 1999, J. Am. Chem. Soc., 121:5633).

The properties of these and other polymers and methods for preparing them are well known in the art (see, for example, U.S. Patents 6,123,727; 5,804,178; 5,770,417; 5,736,372; 5,716,404; 6,095,148; 5,837,752; 5,902,599; 5,696,175; 5,514,378; 5,512,600; 5,399,665; 5,019,379; 5,010,167; 4,806,621; 4,638,045; and 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181). More generally, a variety of methods for synthesizing certain suitable polymers are described in Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Patents 6,506,577, 6,632,922, 6,686,446, and 6,818,732.

In some embodiments, polymers can be linear or branched polymers. In some embodiments, polymers can be dendrimers. In some embodiments, polymers can be substantially cross-linked to one another. In some embodiments, polymers can be substantially free of cross-links. In some embodiments, polymers can be used in accordance with the present invention without undergoing a cross-linking step. It is further to be understood that inventive synthetic nanocarriers may comprise block copolymers, graft copolymers, blends, mixtures, and/or adducts of any of the foregoing and other polymers. Those skilled in the art will recognize that the polymers listed herein represent an exemplary, not comprehensive, list of polymers that can be of use in accordance with the present invention.

In some embodiments, the synthetic nanocarriers comprise one or more polymers. The polymeric synthetic nanocarriers, therefore, can also include those described in WO publication WO2009/051837 by Von Andrian et al., including, but not limited to those, with one or more hydrophilic components. Preferably, the one or more polymers comprise a polyester, such as a poly(lactic acid), poly(glycolic acid), poly(lactic-co-glycolic acid), or polycaprolactone. More preferably, the one or more polymers comprise or further comprise a polyester coupled to a hydrophilic polymer, such as a polyether. In embodiments, the polyether comprises polyethylene glycol. Still more preferably, the one or more polymers comprise a polyester and a polyester coupled to a hydrophilic polymer, such as a polyether. In other embodiments, the one or more polymers are coupled to one or more antigens and/or one or more adjuvants. In embodiments, at least some of the polymers are coupled to the antigen(s) and/or at least some of the polymers are coupled to the adjuvant(s). Preferably, when there are more than one type of polymer, one of the types of polymer is coupled to the antigen(s). In embodiments, one of the other types of polymer is coupled to the adjuvant(s). For example, in embodiments, when the nanocarriers comprise a polyester and a polyester coupled to a hydrophilic polymer, such as a polyether, the polyester is coupled to the adjuvant, while the polyester coupled to the hydrophilic polymer, such as a polyether, is coupled to the antigen(s). In embodiments, where the nanocarriers comprise a universal T cell antigen, such as a T helper cell antigen, the universal T cell antigen can be encapsulated in the nanocarrier.

In some embodiments, synthetic nanocarriers do not comprise a polymeric component. In some embodiments, synthetic nanocarriers may comprise metal particles, quantum dots, ceramic particles, etc. In some embodiments, a non-polymeric synthetic nanocarrier is an aggregate of non-polymeric components, such as an aggregate of metal atoms (e.g., gold atoms).

In some embodiments, synthetic nanocarriers may optionally comprise one or more amphiphilic entities. In some embodiments, an amphiphilic entity can promote the production of synthetic nanocarriers with increased stability, improved uniformity, or increased viscosity. In some embodiments, amphiphilic entities can be associated with the interior surface of a lipid membrane (e.g., lipid bilayer, lipid monolayer, etc.). Many amphiphilic entities known in the art are suitable for use in making synthetic nanocarriers in accordance with the present invention. Such amphiphilic entities include, but are not limited to, phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (Span®85) glycocholate; sorbitan monolaurate (Span®20); polysorbate 20 (Tween®20); polysorbate 60 (Tween®60); polysorbate 65 (Tween®65); polysorbate 80 (Tween®80); polysorbate 85 (Tween®85); polyoxyethylene monostearate; surfactin; a poloxomer; a sorbitan fatty acid ester such as sorbitan trioleate; lecithin; lysolecithin; phosphatidylserine; phosphatidylinositol;sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; and combinations thereof. An amphiphilic entity component may be a mixture of different amphiphilic entities. Those skilled in the art will recognize that this is an exemplary, not comprehensive, list of substances with surfactant activity. Any amphiphilic entity may be used in the production of synthetic nanocarriers to be used in accordance with the present invention.

In some embodiments, synthetic nanocarriers may optionally comprise one or more carbohydrates. Carbohydrates may be natural or synthetic. A carbohydrate may be a derivatized natural carbohydrate. In certain embodiments, a carbohydrate comprises monosaccharide or disaccharide, including but not limited to glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid. In certain embodiments, a carbohydrate is a polysaccharide, including but not limited to pullulan, cellulose, microcrystalline cellulose, hydroxypropyl methylcellulose (HPMC), hydroxycellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan. In embodiments, the inventive synthetic nanocarriers do not comprise (or specifically exclude) carbohydrates, such as a polysaccharide. In certain embodiments, the carbohydrate may comprise a carbohydrate derivative such as a sugar alcohol, including but not limited to mannitol, sorbitol, xylitol, erythritol, maltitol, and lactitol.

Compositions according to the invention comprise inventive synthetic nanocarriers in combination with pharmaceutically acceptable excipients, such as preservatives, buffers, saline, or phosphate buffered saline. The compositions may be made using conventional pharmaceutical manufacturing and compounding techniques to arrive at useful dosage forms. In an embodiment, inventive synthetic nanocarriers are suspended in sterile saline solution for injection together with a preservative.

In embodiments, when preparing synthetic nanocarriers as carriers for antigens and/or adjuvants for use in vaccines, methods for coupling the antigens and/or adjuvants to the synthetic nanocarriers may be useful. If the antigen and/or adjuvant is a small molecule it may be of advantage to attach the antigen and/or adjuvant to a polymer prior to the assembly of the synthetic nanocarriers. In embodiments, it may also be an advantage to prepare the synthetic nanocarriers with surface groups that are used to couple the antigen and/or adjuvant to the synthetic nanocarrier through the use of these surface groups rather than attaching the antigen and/or adjuvant to a polymer and then using this polymer conjugate in the construction of synthetic nanocarriers.

Surface antigens can be coupled to the synthetic nanocarriers by a variety of methods. In embodiments, the surface antigen is coupled to an external surface of the synthetic nanocarrier covalently or non-covalently.

In certain embodiments, the coupling can be via a covalent linker. In embodiments, surface antigens and/or adjuvants according to the invention can be covalently coupled to the external surface via a 1,2,3-triazole linker formed by the 1,3-dipolar cycloaddition reaction of azido groups on the surface of the nanocarrier with surface antigens and/or adjuvants containing an alkyne group or by the 1,3-dipolar cycloaddition reaction of alkynes on the surface of the nanocarrier with surface antigens and/or adjuvants containing an azido group. Such cycloaddition reactions are preferably performed in the presence of a Cu(I) catalyst along with a suitable Cu(I)-ligand and a reducing agent to reduce Cu(II) compound to catalytic active Cu(I) compound. This Cu(I)-catalyzed azide-alkyne cycloaddition (CuAAC) can also be referred as the click reaction.

Additionally, the covalent coupling may comprise a covalent linker that comprises an amide linker, a disulfide linker, a thioether linker, a hydrazone linker, a hydrazide linker, an imine or oxime linker, an urea or thiourea linker, an amidine linker, an amine linker, and a sulfonamide linker.

An amide linker is formed via an amide bond between an amine on one component such as the peptide with the carboxylic acid group of a second component such as the nanocarrier. The amide bond in the linker can be made using any of the conventional amide bond forming reactions with suitably protected amino acids or peptides and activated carboxylic acid such N-hydroxysuccinimide-activated ester.

A disulfide linker is made via the formation of a disulfide (S-S) bond between two sulfur atoms of the form, for instance, of R₁-S-S-R₂. A disulfide bond can be formed by thiol exchange of a surface antigens and/or adjuvants containing thiol/mercaptan group(-SH) with another activated thiol group on a polymer or nanocarrier or a nanocarrier containing thiol/mercaptan groups with an antigen and/or adjuvants containing activated thiol group.

A triazole linker, specifically a 1,2,3-triazole of the form 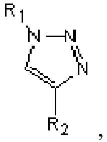 wherein R₁ and R₂ may be any chemical entities, is made by the 1,3-dipolar cycloaddition reaction of an azide attached to a first component such as the nanocarrier with a terminal alkyne attached to a second component such as the antigen and/or adjuvant. The 1,3-dipolar cycloaddition reaction is performed with or without a catalyst, preferably with Cu(I)-catalyst, which links the two components through a 1,2,3-triazole function. This chemistry is described in detail by Sharpless et al., Angew. Chem. Int. Ed. 41(14), 2596, (2002) and Meldal, et al, Chem. Rev., 2008, 108(8), 2952-3015 and is often referred to as "click" reaction or CuAAC.

In embodiments, a polymer containing an azide or alkyne group, terminal to the polymer chain is prepared. This polymer is then used to prepare a synthetic nanocarrier in such a manner that a plurality of the alkyne or azide groups are positioned on the surface of that nanocarrier. Alternatively, the synthetic nanocarrier can be prepared by another route, and subsequently functionalized with alkyne or azide groups. The antigen and/or adjuvant is prepared with the presence of either an alkyne (if the polymer contains an azide) or an azide (if the polymer contains an alkyne) group. The antigen and/or adjuvant is then allowed to react with the nanocarrier via the 1,3-dipolar cycloaddition reaction with or without a catalyst which covalently couples the antigen and/or adjuvant to the particle through the 1,4-disubstituted 1,2,3-triazole linker.

A thioether linker is made by the formation of a sulfur-carbon (thioether) bond in the form, for instance, of R₁-S-R₂. Thioether can be made by either alkylation of a thiol/mercaptan (-SH) group on one component such as the antigen and/or adjuvant with an alkylating group such as halide or epoxide on a second component such as the nanocarrier. Thioether linkers can also be formed by Michael addition of a thiol/mercaptan group on one component such as an antigen and/or adjuvant to an electron-deficient alkene group on a second component such as a polymer containing a maleimide group or vinyl sulfone group as the Michael acceptor. In another way, thioether linkers can be prepared by the radical thiolene reaction of a thiol/mercaptan group on one component such as an antigen and/or adjuvant with an alkene group on a second component such as a polymer or nanocarrier.

A hydrazone linker is made by the reaction of a hydrazide group on one component such as the antigen and/or adjuvant with an aldehyde/ketone group on the second component such as the nanocarrier.

A hydrazide linker is formed by the reaction of a hydrazine group on one component such as the antigen and/or adjuvant with a carboxylic acid group on the second component such as the nanocarrier. Such reaction is generally performed using chemistry similar to the formation of amide bond where the carboxylic acid is activated with an activating reagent.

An imine or oxime linker is formed by the reaction of an amine or N-alkoxyamine (or aminooxy) group on one component such as the antigen and/or adjuvant with an aldehyde or ketone group on the second component such as the nanocarrier.

An urea or thiourea linker is prepared by the reaction of an amine group on one component such as the antigen and/or adjuvant with an isocyanate or thioisocyanate group on the second component such as the nanocarrier.

An amidine linker is prepared by the reaction of an amine group on one component such as the antigen and/or adjuvant with an imidoester group on the second component such as the nanocarrier.

An amine linker is made by the alkylation reaction of an amine group on one component such as the antigen and/or adjuvant with an alkylating group such as halide, epoxide, or sulfonate ester group on the second component such as the nanocarrier. Alternatively, an amine linker can also be made by reductive amination of an amine group on one component such as the antigen and/or adjuvant with an aldehyde or ketone group on the second component such as the nanocarrier with a suitable reducing reagent such as sodium cyanoborohydride or sodium triacetoxyborohydride.

A sulfonamide linker is made by the reaction of an amine group on one component such as the antigen and/or adjuvant with a sulfonyl halide (such as sulfonyl chloride or sulfonyl fluoride) group on the second component such as the nanocarrier.
A sulfone linker is made by Michael addition of a nucleophile to a vinyl sulfone. Either the vinyl sulfone or the nucleophile may be on the surface of the nanoparticle or attached to the antigen or adjuvant.

The antigen or adjuvant can also be conjugated to the nanocarrier via non-covalent conjugation methods. For examples, a negative charged antigen or adjuvant can be conjugated to a positive charged nanocarrier through electrostatic adsorption. A antigen or adjuvant containing a metal ligand can also be conjugated to a nanocarrier containing a metal complex via a metal-ligand complex.

In embodiments, the antigen or adjuvant can be attached to a polymer, for example polylactic acid-block-polyethylene glycol, prior to the assembly of the synthetic nanocarrier or the synthetic nanocarrier can be formed with reactive or activatible groups on its surface. In the latter case, the antigen or adjuvant may be prepared with a group which is compatible with the attachment chemistry that is presented by the synthetic nanocarriers' surface. In other embodiments, a peptide antigen can be attached to VLPs or liposomes using a suitable linker. A linker is a compound or reagent that capable of coupling two molecules together. In an embodiment, the linker can be a homobifuntional or heterobifunctional reagent as described in Hermanson 2008. For example, an VLP or liposome synthetic nanocarrier containing a carboxylic group on the surface can be treated with a homobifunctional linker, adipic dihydrazide (ADH), in the presence of EDC to form the corresponding synthetic nanocarrier with the ADH linker. The resulting ADH linked synthetic nanocarrier is then conjugated with a peptide antigen and/or adjuvant containing an acid group via the other end of the ADH linker on NC to produce the corresponding VLP or liposome peptide conjugate.

For detailed descriptions of available conjugation methods, see Hermanson G T "Bioconjugate Techniques", 2nd Edition, Published by Academic Press, Inc., 2008. In addition to covalent attachment the antigen and/or adjuvant can be coupled by adsorbtion to a pre-formed synthetic nanocarrier or it/they can be coupled by encapsulation during the formation of the synthetic nanocarrier.

In embodiments, surface antigens can be non-covalently coupled to synthetic nanocarriers using various non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. In embodiments, encapsulation is a form of coupling. When coupling charged surface antigens, the synthetic nanocarriers can be produced in the presence of surfactants which become adsorbed to surfaces of the synthetic nanocarrier and in doing so they impart a charge to the synthetic nanocarrier. Charged surface antigens can then be non-covalently attached to the charged synthetic nanocarrier by a charge-charge interaction (see for example O'Hagen WO2000006123A1).

In embodiments, the inventive synthetic nanocarriers can be combined with one or more adjuvants by admixing in the same vehicle or delivery system. Such adjuvants may include, but are not limited to the adjuvant provided herein, such as mineral salts, such as alum, alum combined with monphosphoryl lipid (MPL) A of Enterobacteria, such as Escherihia coli, Salmonella minnesota, Salmonella typhimurium, or Shigella flexneri or specifically with MPL® (AS04), MPL A of above-mentioned bacteria separately, saponins, such as QS-21,Quil-A, ISCOMs, ISCOMATRIX™, emulsions such as MF59™, Montanide® ISA 51 and ISA 720, AS02 (QS21+squalene+ MPL®), liposomes and liposomal formulations such as AS01, AS15, synthesized or specifically prepared microparticles and microcarriers such as bacteria-derived outer membrane vesicles (OMV) of N. gonorrheae, Chlamydia trachomatis and others, or chitosan particles, depot-forming agents, such as Pluronic® block co-polymers, specifically modified or prepared peptides, such as muramyl dipeptide, aminoalkyl glucosaminide 4-phosphates, such as RC529, or proteins, such as bacterial toxoids or toxin fragments. The doses of such other adjuvants can be determined using conventional dose ranging studies.

In embodiments, the inventive synthetic nanocarriers can be combined with other antigens different, similar or identical to those coupled to a nanocarrier (with or without adjuvant, utilizing or not utilizing another delivery vehicle) administered separately at a different time-point and/or at a different body location and/or by a different immunization route or with another antigen and/or adjuvant-carrying synthetic nanocarrier administered separately at a different time-point and/or at a different body location and/or by a different immunization route.

Populations of synthetic nanocarriers may be combined to form dosage forms according to the present invention using traditional pharmaceutical mixing methods. These include liquid-liquid mixing in which two or more suspensions, each containing one or more subset of nanocarriers, are directly combined or are brought together via one or more vessels containing diluent. As synthetic nanocarriers may also be produced or stored in a powder form, dry powder-powder mixing could be performed as could the re-suspension of two or more powders in a common media. Depending on the properties of the nanocarriers and their interaction potentials, there may be advantages conferred to one or another route of mixing.

Typical inventive compositions that comprise synthetic nanocarriers may comprise inorganic or organic buffers (e.g., sodium or potassium salts of phosphate, carbonate, acetate, or citrate) and pH adjustment agents (e.g., hydrochloric acid, sodium or potassium hydroxide, salts of citrate or acetate, amino acids and their salts) antioxidants (e.g., ascorbic acid, alpha-tocopherol), surfactants (e.g., polysorbate 20, polysorbate 80, polyoxyethylene9-10 nonyl phenol, sodium desoxycholate), solution and/or cryo/lyo stabilizers (e.g., sucrose, lactose, mannitol, trehalose), osmotic adjustment agents (e.g., salts or sugars), antibacterial agents (e.g., benzoic acid, phenol, gentamicin), antifoaming agents (e.g., polydimethylsilozone), preservatives (e.g., thimerosal, 2-phenoxyethanol, EDTA), polymeric stabilizers and viscosity-adjustment agents (e.g., polyvinylpyrrolidone, poloxamer 488, carboxymethylcellulose) and co-solvents (e.g., glycerol, polyethylene glycol, ethanol).

Compositions according to the invention comprise inventive synthetic nanocarriers in combination with pharmaceutically acceptable excipients or carriers. The compositions may be made using conventional pharmaceutical manufacturing and compounding techniques to arrive at useful dosage forms. Techniques suitable for use in practicing the present invention may be found in Handbook of Industrial Mixing: Science and Practice, Edited by Edward L. Paul, Victor A. Atiemo-Obeng, and Suzanne M. Kresta, 2004 John Wiley & Sons, Inc.; and Pharmaceutics: The Science of Dosage Form Design, 2nd Ed. Edited by M. E. Auten, 2001, Churchill Livingstone. In an embodiment, inventive synthetic nanocarriers are suspended in sterile saline solution for injection together with a preservative.

Doses of dosage forms contain varying amounts of populations of synthetic nanocarriers according to the invention. The amount of synthetic nanocarriers present in the inventive dosage forms can be varied according to the nature of the sets of surface antigens, the therapeutic benefit to be accomplished, and other such parameters. In embodiments, dose ranging studies can be conducted to establish optimal therapeutic amount of synthetic nanocarriers to be present in the dosage form. In embodiments, first and second populations are present in an amount effective to generate an immune response to the first set of surface antigens and the second set of surface antigens upon administration to a subject. It may be possible to determine amounts of the first, second, and/or subsequent populations effective to generate an immune response using conventional dose ranging studies and techniques in subjects. Inventive dosage forms may be administered at a variety of frequencies. In a preferred embodiment, at least one administration of the dosage form is sufficient to generate a pharmacologically relevant response. In more preferred embodiment, at least two administrations, at least three administrations, or at least four administrations, of the dosage form are utilized to ensure a pharmacologically relevant response.

It is to be understood that the compositions of the invention can be made in any suitable manner, and the invention is in no way limited to compositions that can be produced using the methods described herein. Selection of an appropriate method may require attention to the properties of the particular moieties being associated. In embodiments, methods of manufacture comprise preparing a first population of synthetic nanocarriers that comprise a first set of surface antigens; preparing a second population of synthetic nanocarriers that comprise a second set of surface antigens; and combining the first and second populations of synthetic nanocarriers into a pharmaceutical dosage form; wherein the first set of surface antigens and the second set of surface antigens are structurally or immunologically different.

In some embodiments, inventive synthetic nanocarriers are manufactured under sterile conditions or are terminally sterilized. This can ensure that resulting composition are sterile and non-infectious, thus improving safety when compared to non-sterile compositions. This provides a valuable safety measure, especially when subjects receiving synthetic nanocarriers have immune defects, are suffering from infection, and/or are susceptible to infection. In some embodiments, inventive synthetic nanocarriers may be lyophilized and stored in suspension or as lyophilized powder depending on the formulation strategy for extended periods without losing activity.

The inventive compositions may be administered by a variety of routes of administration, including but not limited to parenteral (such as subcutaneous, intramuscular, intravenous, or intradermal); oral; transnasal, intranasal, transmucosal, sublingual, rectal, ophthalmic, transdermal, transcutaneous or by a combination of these routes.

The compositions and methods described herein can be used to induce, enhance, modulate, stimulate, suppress, direct or redirect an immune response. The compositions and methods described herein can be used in the diagnosis, prophylaxis and/or treatment of conditions such as cancers, infectious diseases, metabolic diseases, degenerative diseases, autoimmune diseases, inflammatory diseases, immunological diseases, or other disorders and/or conditions. The compositions and methods described herein can also be used for the prophylaxis or treatment of an addiction, such as an addiction to nicotine or a narcotic. The compositions and methods described herein can also be used for the prophylaxis and/or treatment of a condition resulting from the exposure to a toxin, hazardous substance, environmental toxin, or other harmful agent.

The subjects provided herein can have or be at risk of having an addiction to an abused or addictive substance.

The subjects provided herein can have or be at risk of having cancer. Cancers include, but are not limited to, breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia, e.g., B Cell CLL; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor.

The subjects provided herein can have or be at risk of having an infection or infectious disease. Infections or infectious diseases include, but are not limited to, viral infectious diseases, such as AIDS, Chickenpox (Varicella), Common cold, Cytomegalovirus Infection, Colorado tick fever, Dengue fever, Ebola hemorrhagic fever, Hand, foot and mouth disease, Hepatitis, Herpes simplex, Herpes zoster, HPV, Influenza (Flu), Lassa fever, Measles, Marburg hemorrhagic fever, Infectious mononucleosis, Mumps, Norovirus, Poliomyelitis, Progressive multifocal leukencephalopathy, Rabies, Rubella, SARS, Smallpox (Variola), Viral encephalitis, Viral gastroenteritis, Viral meningitis, Viral pneumonia, West Nile disease and Yellow fever; bacterial infectious diseases, such as Anthrax, Bacterial Meningitis, Botulism, Brucellosis, Campylobacteriosis, Cat Scratch Disease, Cholera, Diphtheria, Epidemic Typhus, Gonorrhea, Impetigo, Legionellosis, Leprosy (Hansen's Disease), Leptospirosis, Listeriosis, Lyme disease, Melioidosis, Rheumatic Fever, MRSA infection, Nocardiosis, Pertussis (Whooping Cough), Plague, Pneumococcal pneumonia, Psittacosis, Q fever, Rocky Mountain Spotted Fever (RMSF), Salmonellosis, Scarlet Fever, Shigellosis, Syphilis, Tetanus, Trachoma, Tuberculosis, Tularemia, Typhoid Fever, Typhus and Urinary Tract Infections; parasitic infectious diseases, such as African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, Trichomoniasis and Trypanosomiasis; fungal infectious disease, such as Aspergillosis, Blastomycosis, Candidiasis, Coccidioidomycosis, Cryptococcosis, Histoplasmosis, Tinea pedis (Athlete's Foot) and Tinea cruris; prion infectious diseases, such as Alpers' disease, Fatal Familial Insomnia, Gerstmann-Sträussler-Scheinker syndrome, Kuru and Variant Creutzfeldt-Jakob disease.

### EXAMPLES

The invention will be more readily understood by reference to the following examples, which are included merely for purposes of illustration of certain aspects and embodiments of the present invention and not as limitations.

Those skilled in the art will appreciate that various adaptations and modifications of the just-described embodiments can be configured without departing from the scope and spirit of the invention. Other suitable techniques and methods known in the art can be applied in numerous specific modalities by one skilled in the art and in light of the description of the present invention described herein.

Therefore, it is to be understood that the invention can be practiced other than as specifically described herein. The above description is intended to be illustrative, and not restrictive. Many other embodiments will be apparent to those of skill in the art upon reviewing the above description. The scope of the invention should, therefore, be determined with reference to the appended claims, along with the full scope of equivalents to which such claims are entitled.

### Example 1: Formulation for First Population of Nanocarriers (Prophetic)

Synthetic nanocarriers comprising PLGA-R848 conjugate (adjuvant), PLA-PEG-N3 conjugate (linker to peptide antigen) and ova peptide (T-cell antigen) are prepared via a double emulsion method wherein the ova peptide is encapsulated in the synthetic nanocarriers. To a suspension of the synthetic nanocarriers (10 mg/mL in PBS (pH 7.4 buffer), 5 mL, containing about 12.5 mg (MW: 20, 000; 0.000625 mmol) of PLA-PEG-N3) is added HPV L1-peptide comprising an acetylene linker (33 mg) under gentle stirring. A solution of sodium ascorbate (100 mM in H2O, 0.3 mL) is added, followed by CuSO4 solution (10 mM in water, 0.6 mL). The resulting light yellow suspension is stirred at 20 C for 15 h and additional CuSO4 solution (0.3 mL) and sodium ascorbate solution (0.15 mL) are added. The suspension is stirred for 5 h at 20 C and diluted with PBS buffer (pH 7.4) to 10 mL and is centrifuged to remove the supernatant. The residual nanocarrier pellets are washed twice with PBS buffer. The washed NCs are then re-suspended in 5 mL of PBS buffer and stored frozen. The conjugation of L1 peptide on the surface of the synthetic nanocarriers is confirmed by HPLC analysis of the digested synthetic nanocarriers and by bioassay.

### Example 2: Formulation for Second Population of Nanocarriers (Prophetic)

Using the general procedures outlines in Example 1 above, synthetic nanocarriers comprising PLA-R848, PLA-PEG-N3 and encapsulated ova peptide are prepared and conjugated with an HPV L2 peptide to provide L2 peptide conjugated synthetic nanocarriers.

### Example 3: Formulation Combining First and Second Populations of Nanocarriers (Prophetic)

The synthetic nanocarrier preparations from Examples 1 and 2 above are thawed and diluted in PBS to a final concentration of 5 mg of nanocarriers per milliliter. Equal aliquots of each (0.5 mL) are combined to provide a population of nanocarriers that contain both the HPV L1 and L2 peptides.

### Example 4: Preparations of Nanocarriers

### Preparation of NC-Nic-OVA

PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-Nicotine (S-642), poly-D/L lactide-block-poly(ethylene glycol)-(±)-trans-3'-hydroxymethylnicotine ether with PEG block of approximately 5,000 Da and PLA block of approximately 21,000 Da was custom manufactured at Princeton Global Synthesis (300 George Patterson Drive #206, Bristol, PA 19007.) PLA-PEG-Maleimide, block co-polymer consisting of a poly-D/L-lactide (PLA) block of approximately 22000 Da and a polyethylene glycol (PEG) block of approximately 2900 Da that is terminated by a maleimide functional group was synthesized from commercial starting materials by generating the PLA block by ring-opening polymerization of dl-lactide with HO-PEG-Maleimide with dl-lactide. Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: 0.13N HCl in purified water.
Solution 2: PLGA-R848 @ 50 mg/mL, PLA-PEG-Nicotine @ 25 mg/mL, and PLA-PEG-Maleimide @ 25 mg/mL in dichloromethane was prepared by dissolving each polymer separately in dichloromethane at 100 mg/mL then combining 2 parts PLGA-R848 solution to 1 part each PLA-PEG-Nicotine solution and PLA-PEG-Maleimide solution.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 40 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated and then the pellet was re-suspended in phosphate buffered saline to achieve a nanocarrier suspension having a nominal concentration of 10 mg/mL on a polymer basis. The nanocarrier suspension was stored frozen at -20C until further use.

**Table 4: NC-Nic-OVA Characterization**

| Nanocarrier | Effective Diameter (nm) | TLR Agonist, % w/w | T-cell agonist, % w/w |
|---|---|---|---|
| NC-Nic-OVA | 215 | R848, 4.2 | None |

(1) NC with PEG-Nicotine and PEG-MAL on the surface, prepared as above; 6.5 mg/mL suspension in PBS buffer.
(2) OVA protein (Ovalbumin from egg white): Worthington, Lot# POK12101, MW: 46000.
(3) Traut's reagent (2-iminothiolane.HCl): MP Biomedical, Lot# 8830KA, MW: 137.6
(4) pH 8 buffer (sodium phosphate , 20 mM with 0.5 mM EDTA).
(5) pH 7 1x PBS buffer.
OVA protein (10 mg) was dissolved in 1 mL pH 8 buffer. A freshly made solution of Traut's reagent in pH 8 buffer (0.25 mL, 2 mg/mL) was added to the OVA protein solution. The resulting solution was stirred under argon in the dark for 1.5 h. The solution was diafiltered with MWCO 3K diafilter tube and washed with pH 8 buffer twice. The resulting modified OVA with thiol group was dissolved in 1 mL pH 8 buffer under argon. The NC suspension (3 mL, 6.5 mg/mL) was centrifuged to remove the supernatant. The modified OVA solution was then mixed with the NC pellets. The resulting suspension was stirred at rt under argon in the dark for 12 h. The NC suspension was then diluted to 10 mL with pH 7 PBS and centrifuged. The resulting NC was pellet washed with 2x10 mL pH 7 PBS. The NC-Nic-OVA conjugates were then resuspended in pH 7 PBS (ca. 6 mg/mL, 3 mL) stored at 4°C.

### Preparation of NC-OVA

PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-Maleimide, block co-polymer consisting of a poly-D/L-lactide (PLA) block of approximately 22000 Da and a polyethylene glycol (PEG) block of approximately 2900 Da that is terminated by a maleimide functional group was synthesized from commercial starting materials by generating the PLA block by ring-opening polymerization of dl-lactide with HO-PEG-Maleimide. Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: 0.13N HCl in purified water.
Solution 2: PLGA-R848 @ 50 mg/mL and PLA-PEG-Maleimide @ 50 mg/mL in dichloromethane was prepared by dissolving each polymer separately in dichloromethane at 100 mg/mL then combining 1 part PLGA-R848 solution to 1 part PLA-PEG-Maleimide solution.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 40 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated, and then the pellet was re-suspended in phosphate buffered saline to achieve a nanocarrier suspension having a nominal concentration of 10 mg/mL on a polymer basis. The nanocarrier suspension was stored frozen at -20°C until further use.

**Table 5: NC-OVA Characterization**

| Nanocarrier | Effective Diameter (nm) | TLR Agonist, % w/w | T-cell agonist, % w/w |
|---|---|---|---|
| NC-OVA | 208 | R848, 4.3 | None |

(1) NC with PEG-MAL on the surface, prepared as above; 6 mg/mL suspension in PBS buffer.
(2) OVA protein (Ovalbumin from egg white): Worthington, Lot# POK12101, MW: 46000.
(3) Traut's reagent (2-iminothiolane.HCl): MP Biomedical, Lot# 8830KA, MW: 137.6.
(4) pH 8 buffer (sodium phosphate , 20 mM with 0.5 mM EDTA).
(5) pH 7 1x PBS buffer.

OVA protein (20 mg) was dissolved in 1 mL pH 8 buffer. A freshly made solution of Traut's reagent in pH 8 buffer (0.5 mL, 2 mg/mL) was added to the OVA protein solution. The resulting solution was stirred under argon in the dark for 1.5 h. The solution was diafiltered with MWCO 3K diafilter tube and washed with pH 8 buffer twice. The resulting modified OVA with thiol group was dissolved in 1 mL pH 8 buffer under argon. The NC suspension (4 mL, 6 mg/mL) was centrifuged to remove the supernatant. The modified OVA solution was then mixed with the NC pellets. The resulting suspension was stirred at rt under argon in the dark for 12 h. The NC suspension was then diluted to 10 mL with pH 7 PBS and centrifuged. The resulting NC was pellet washed with 2x10 mL pH 7 PBS. The NC-OVA conjugates were then resuspended in pH 7 PBS (ca. 6 mg/mL, 4 mL) stored at 4°C.

### Preparation of NC-HA5

Ovalbumin peptide 323-339 amide acetate salt, was purchased from Bachem Americas Inc. (3132 Kashiwa Street, Torrance CA 90505. Product code 4065609.) PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-Maleimide, block co-polymer consisting of a poly-D/L-lactide (PLA) block of approximately 22000 Da and a polyethylene glycol (PEG) block of approximately 2900 Da that is terminated by a maleimide functional group, was synthesized from commercial starting materials by generating the PLA block by ring-opening polymerization of dl-lactide with HO-PEG-Maleimide. Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: Ovalbumin peptide 323-339 @ 20mg/mL was prepared in 0.13N HCl at room temperature.
Solution 2: PLGA-R848 @ 50 mg/mL and PLA-PEG-Maleimide @ 50 mg/mL in dichloromethane was prepared by dissolving each polymer separately in dichloromethane at 100 mg/mL then combining 1 part PLGA-R848 solution to 1 part PLA-PEG-Maleimide solution.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 40 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated, and then the pellet was re-suspended in phosphate buffered saline to achieve a nanocarrier suspension having a nominal concentration of 10 mg/mL on a polymer basis. The nanocarrier suspension was stored frozen at -20°C until further use.

**Table 6: NC-HA5 Characterization**

| Nanocarrier | Effective Diameter (nm) | TLR Agonist, % w/w | T-cell agonist, % w/w |
|---|---|---|---|
| NC-HA5 | 216 | R848, 3.6 | Ova peptide 323-339, 2.0 |

(1) NC with PEG-MAL on the surface, prepared as above; 6.7 mg/mL suspension in PBS buffer.
(2) HA5 protein: Recombinant Hemagglutinin, A/Vietnam/1203/2004, MW: 72000, supplied as a solution in pH 7 PBS-tween buffer (0.55 mg/mL).
(3) Traut's reagent (2-iminothiolane.HCl): MP Biomedical, Lot# 8830KA, MW: 137.6.
(4) pH 8 buffer (sodium phosphate , 20 mM with 0.5 mM EDTA).
(5) pH 7 1x PBS buffer.

HA5 protein (0.21 g in 0.38 mL pH 7.1 PBS-tween buffer) was diluted to 0.5 mL with pH 8 buffer. A freshly made solution of Traut's reagent in pH 8 buffer (0.02 mL, 2 mg/mL) was added to the HA5 protein solution. The resulting solution was stirred under argon in the dark for 1.5 h. The solution was diafiltered with MWCO 3K diafilter tube and washed with pH 8 buffer twice. The resulting modified HA5 protein with thiol group was dissolved in 0.5 mL pH 8 buffer under argon. The NC suspension (3 mL, 6.7 mg/mL) was centrifuged to remove the supernatant. The modified HA5 solution was then mixed with the NC pellets. The resulting suspension was stirred at rt under argon in the dark for 12 h. The NC suspension was then diluted to 10 mL with pH 7 PBS and centrifuged. The resulting NC was pellet washed with 2x10 mL pH 7 PBS. The NC-HA5 conjugates were then resuspended in pH 7 PBS (ca. 6 mg/mL, 3 mL) stored at 4°C.

### Preparation of NC-L2, NC-M2e or NC-M2e-L2

Ovalbumin peptide 323-339 amide acetate salt, was purchased from Bachem Americas Inc. (3132 Kashiwa Street, Torrance CA 90505. Product code 4065609.) PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-C6-N₃, block co-polymer consisting of a poly-D/L-lactide (PLA) block of approximately 23000 Da and a polyethylene glycol (PEG) block of approximately 2000 Da that is terminated by an amide-conjugated C₆H₁₂ linker to an azide, was synthesized by conjugating HO-PEG-COOH to an amino-C₆H₁₂-azide and then generating the PLA block by ring-opening polymerization of the resulting HO-PEG-C6-N3 with dl-lactide. Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: Ovalbumin peptide 323-339 @ 20mg/mL was prepared in 0.13N HCl at room temperature.
Solution 2: PLGA-R848 @ 50 mg/mL and PLA-PEG-C6-N₃ @ 50 mg/mL in dichloromethane was prepared by dissolving each separately at 100 mg/mL in dichloromethane then combining in equal parts by volume.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 40 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated, and then the pellet was re-suspended in phosphate buffered saline to achieve a nanocarrier suspension having a nominal concentration of 10 mg/mL on a polymer basis. Two identical batches were created and then combined to form a single homogenous suspension at which was stored frozen at - 20°C until further use.

**Table 7: NC-L2, NC-M2e or NC-M2e-L2 Characterization**

| Nanocarrier | Effective Diameter (nm) | TLR Agonist, % w/w | Antigen, % w/w |
|---|---|---|---|
| NC-L2, NC-M2e or NC-M2e-L2 | 209 | R848, 4.2 | Ova 323-339 peptide, 2.4 |

(1) Nanocarriers with surface PEG-C6-N3 containing PLGA-R848 and Ova-peptide, prepared as above, 7 mg/mL suspension in PBS.
(2) HPV16 L2 peptide modified with an alkyne linker attached to C-terminal Lys amino group; Bachem Americas, Inc, Lot B06055, MW 2595, TFA salt; Sequence: H-Ala-Thr-Gln-Leu-Tyr-Lys-Thr-Cys-Lys-Gln-Ala-Gly-Thr-Cys-Pro-Pro-Asp-Ile-Ile-Pro-Lys-Val-Lys(5-hexynoyl)-NH2(with Cys-Cys disulfide bond).
(3) Catalysts: CuSO4, 100 mM in DI water; THPTA ligand, 200 mM in DI water; sodium ascorbate, 200 mM in DI water freshly prepared.
(4) pH 7.4 PBS buffer.

The NC suspension (7 mg/mL, 4 mL) was concentrated to ca. 1 mL in volume by centrifugation. A solution of L2 peptide (20 mg) in 2 mL PBS buffer was added. A pre-mixed solution of 0.2 mL of CuSO4 (100 mM) and 0.2 mL of THPTA ligand (200 mM) was added, followed by 0.4 mL of sodium ascorbate (200 mM). The resulting light yellow suspension was stirred in dark at ambient room temperature for 18 h. The suspension was then diluted with PBS buffer to 10 mL and centrifuged to remove the supernatant. The NC-L2 conjugates were further pellet washed twice with 10 mL PBS buffer and resuspended in pH 7.4 buffer at final concentration of ca. 6 mg/mL (ca. 4 mL) and stored at 4°C.
(1) Nanocarriers with surface PEG-C6-N3 containing PLGA-R848 and Ova-peptide, prepared as above, 7 mg/mL suspension in PBS.
(2) M2e peptide modified with an alkyne linker attached to C-terminal Gly; CS Bio Co, Catalog No. CS4956, Lot: H308, MW 2650, TFA salt; Sequence: H-Met-Ser-Leu-Leu-Thr-Glu-Val-Glu-Thr-Pro-Thy-Arg-Asn-Glu-Trp-Glu-Cys-Arg-Cys-Ser-Asp-Gly-Gly-NHCH2CCH.
(3) Catalysts: CuSO4, 100 mM in DI water; THPTA ligand, 200 mM in DI water; sodium ascorbate, 200 mM in DI water freshly prepared.
(4) pH 7.4 PBS buffer.

The NC suspension (7 mg/mL, 4 mL) was concentrated to ca. 1 mL in volume by centrifugation. A solution of M2e peptide (20 mg) in 2 mL PBS buffer was added. A pre-mixed solution of 0.2 mL of CuSO4 (100 mM) and 0.2 mL of THPTA ligand (200 mM) was added, followed by 0.4 mL of sodium ascorbate (200 mM). The resulting light yellow suspension was stirred in dark at ambient room temperature for 18 h. The suspension was then diluted with PBS buffer to 10 mL and centrifuged to remove the supernatant. The NC-M2e conjugates were further pellet washed twice with 10 mL PBS buffer and resuspended in pH 7.4 buffer at final concentration of ca. 6 mg/mL (ca. 4 mL) and stored at 4°C.
(1) Nanocarriers with surface PEG-C6-N3 containing PLGA-R848 and Ova-peptide, prepared as above, 7 mg/mL suspension in PBS.
(2) HPV16 L2 peptide modified with an alkyne linker attached to C-terminal Lys amino group; Bachem Americas, Inc, Lot B06055, MW 2595, TFA salt; Sequence: H-Ala-Thr-Gln-Leu-Tyr-Lys-Thr-Cys-Lys-Gln-Ala-Gly-Thr-Cys-Pro-Pro-Asp-Ile-Ile-Pro-Lys-Val-Lys(5-hexynoyl)-NH2(with Cys-Cys disulfide bond).
(3) M2e peptide modified with an alkyne linker attached to C-terminal Gly; CS Bio Co, Catalog No. CS4956, Lot: H308, MW 2650, TFA salt; Sequence: H-Met-Ser-Leu-Leu-Thr-Glu-Val-Glu-Thr-Pro-Thy-Arg-Asn-Glu-Trp-Glu-Cys-Arg-Cys-Ser-Asp-Gly-Gly-NHCH2CCH.
(4) Catalysts: CuSO4, 100 mM in DI water; THPTA ligand, 200 mM in DI water; sodium ascorbate, 200 mM in DI water freshly prepared.
(5) pH 7.4 PBS buffer.

The NC suspension (7 mg/mL, 2 mL) was concentrated to ca. 0.5 mL in volume by centrifugation. A mixture of L2 peptide (5 mg) and M2e peptide (5 mg) in 1 mL PBS buffer was added. A pre-mixed solution of 0.2 mL of CuSO4 (100 mM) and 0.2 mL of THPTA ligand (200 mM) was added, followed by 0.4 mL of sodium ascorbate (200 mM). The resulting light yellow suspension was stirred in dark at ambient room temperature for 18 h. The suspension was then diluted with PBS buffer to 10 mL and centrifuged to remove the supernatant. The NC-M2e-L2 conjugates were further pellet washed twice with 10 mL PBS buffer and resuspended in pH 7.4 buffer at final concentration of ca. 6 mg/mL (ca. 2 mL) and stored at 4°C.

### Example 5: Immunization with Two Monovalent Antigen Nanocarriers Leads to Immune Response to Both Antigens

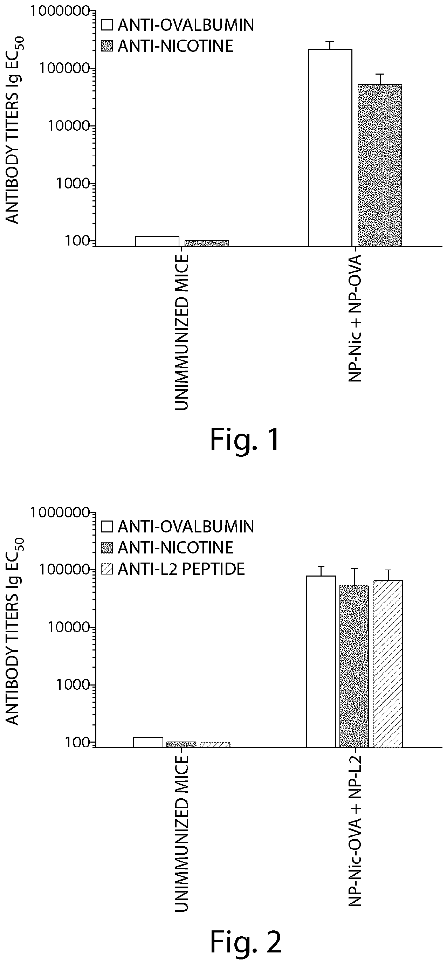

Anti-nicotine (dark gray bars) and anti-ovalbumin (light gray bars) antibody titers in unimmunized mice and mice injected with NC-Nic and NC-OVA (as prepared in Example 4) (5 animals/group; s.c., 100 µg of each NC per injection, 2 times at 3-wk intervals) were measured. Titers for day 33 after immunization with NC are shown in Fig. 1 (ELISA against polylysine-nicotine or ovalbumin protein) (group 1: unimmunized; group 2: immunized with NC-Nic and NC-OVA).

Mice were injected with 100 µg of NC-Nic (nanocarrier exhibiting nicotine on the outer surface and containing OP-II helper peptide and R848 adjuvant in the NC) and 100 µg of NC-OVA (nanocarrier exhibiting ovalbumin on the outer surface and containing OP-II helper peptide and R848 adjuvant in the NC) (subcutaneously, hind limbs) at 3-week intervals (days 0 and 21). Serum anti-nicotine and anti-ovalbumin antibody titers were measured at day 33 after immunization with NC. Anti-nicotine and anti-ovalbumin antibody titers (EC₅₀) as measured by ELISA against polylysine-nicotine or ovalbumin protein are shown (Fig. 1). Titers for control unimmunized mice are also shown. The results demonstrate that mice immunized with a combination of two monovalent antigen nanocarriers (NC-Nic and NC-OVA) generate antibodies to both antigens.

### Example 6: Immunization with Monovalent and Bivalent Antigen Nanocarriers Lead to Immune Response to All Three Antigens

Anti-nicotine, anti-ovalbumin, and anti-L2 peptide antibody titers in unimmunized mice and mice injected with NC-Nic-OVA and NC-L2 (as prepared in Example 4) (5 animals/group; s.c., 100 µg of each NC per injection, 2 times at 3-wk intervals) were measured. Titers for day 33 after immunization with NC are shown in Fig. 2 (ELISA against polylysine-nicotine, ovalbumin protein, or PLA-PEG-L2 peptide) (group 1: unimmunized; group 2: immunized with NC-Nic-OVA and NC-L2).

Mice were injected with 100 µg of NC-Nic-OVA (nanocarrier exhibiting nicotine and ovalbumin on the outer surface and containing OP-II helper peptide and R848 adjuvant in the NC) and 100 µg of NC-L2 (nanocarrier exhibiting HPV L2 peptide (aa17-36) on the outer surface and containing OP-II helper peptide and R848 adjuvant in the NC) (subcutaneously, hind limbs) at 3-week intervals (days 0 and 21). Serum anti-nicotine, anti-ovalbumin, and anti-L2 peptide antibody titers were measured at day 33 after immunization with NC. Anti-nicotine, anti-ovalbumin, and anti-L2 peptide antibody titers (EC₅₀) as measured by ELISA against polylysine-nicotine, ovalbumin protein, and L2 peptide are shown (Fig. 2). Titers for control unimmunized mice are also shown. The results demonstrate that mice immunized with a combination of one monovalent and one bivalent antigen nanocarrier (NC-Nic-OVA and NC-L2) generate antibodies to all three antigens.

### Example 7: Immunization with Two Bivalent Antigen Nanocarriers Leads to Immune Response to all Four Antigens

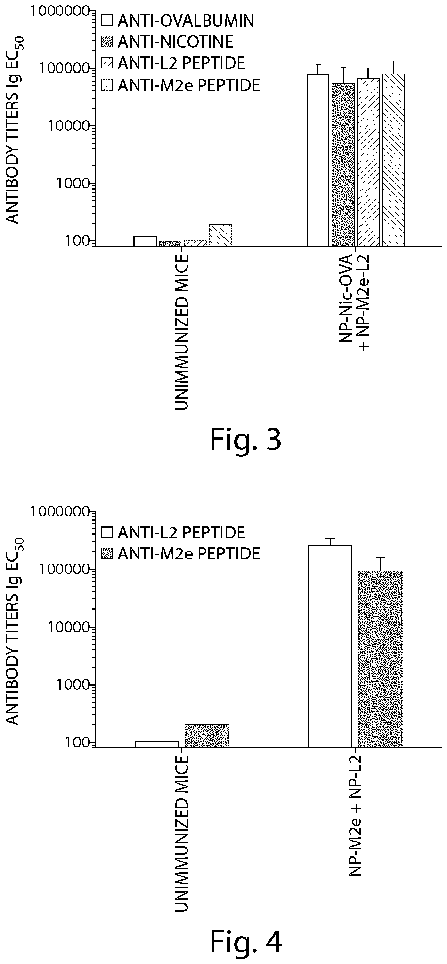

Anti-nicotine, anti-ovalbumin, anti-M2e peptide, and anti-L2 peptide antibody titers in unimmunized mice and mice injected with NC-Nic-OVA and NC-M2e-L2 (as prepared in Example 4) (5 animals/group; s.c., 100 µg of each NC per injection, 2 times at 3-wk intervals) were measured. Titers for day 33 after immunization with NC are shown in Fig. 3 (ELISA against polylysine-nicotine, ovalbumin protein, PLA-PEG-M2e peptide, or PLA-PEG-L2 peptide) (group 1: unimmunized; group 2: immunized with NC-Nic-OVA and NC-M2e-L2).

Mice were injected with 100 µg of NC-Nic-OVA (nanocarrier exhibiting nicotine and ovalbumin on the outer surface and containing OP-II helper peptide and R848 adjuvant in the NC) and 100 µg of NC-M2e-L2 (nanocarrier exhibiting influenza M2e peptide (aa2-27) and HPV L2 peptide (aa17-36) on the outer surface and containing OP-II helper peptide and R848 adjuvant in the NC) (subcutaneously, hind limbs) with 3-week intervals (days 0 and 21). Serum anti-nicotine, anti-ovalbumin, anti-M2e peptide, and anti-L2 peptide antibody titers were measured at day 33 after immunization with NC. Anti-nicotine, anti-ovalbumin, anti-M2e peptide, and anti-L2 peptide antibody titers (EC₅₀) as measured by ELISA against polylysine-nicotine, ovalbumin protein, M2e peptide, and L2 peptide are shown (Fig. 3). Titers for control unimmunized mice are also shown. The results demonstrate that mice immunized with a combination of two bivalent antigen nanocarriers (NC-Nic-OVA and NC-M2e-L2) generate antibodies to all four antigens.

### Example 8: Immunization with Two Monovalent Peptide Antigen Nanocarriers Leads to Immune Response to Both Peptide Antigens

Anti-M2e peptide and anti-L2 peptide antibody titers in unimmunized mice and mice injected with NC-M2e and NC-L2 (as prepared in Example 4) (5 animals/group; s.c., 100 µg of each NC per injection, 2 times at 3-wk intervals) were measured. Titers for day 33 after immunization with NC are shown in Fig. 4 (ELISA against PLA-PEG-M2e peptide or PLA-PEG-L2 peptide) (group 1: unimmunized; group 2: immunized with NC-M2e and NC-L2).

Mice were injected with 100 µg of NC-M2e (nanocarrier exhibiting influenza M2e peptide (aa2-27) on the outer surface and containing OP-II helper peptide and R848 adjuvant in the NC) and 100 µg of NC- L2 (nanocarrier exhibiting HPV L2 peptide (aa17-36) on the outer surface and containing OP-II helper peptide and R848 adjuvant in the NC) (subcutaneously, hind limbs) at 3-week intervals (days 0 and 21). Serum anti-M2e peptide and anti-L2 peptide antibody titers were measured at day 33 after immunization with NC. Anti-M2e peptide and anti-L2 peptide antibody titers (EC₅₀) as measured by ELISA against M2e peptide and L2 peptide are shown (Fig. 4). Titers for control unimmunized mice are also shown. These results demonstrate that mice immunized with a combination of two monovalent peptide antigen nanocarriers (NC-M2e and NC-L2) generate antibodies to both peptide antigens.

### Example 9: Immunization with Two Monovalent Protein Antigen Nanocarriers Leads to Immune Response to Both Protein Antigens

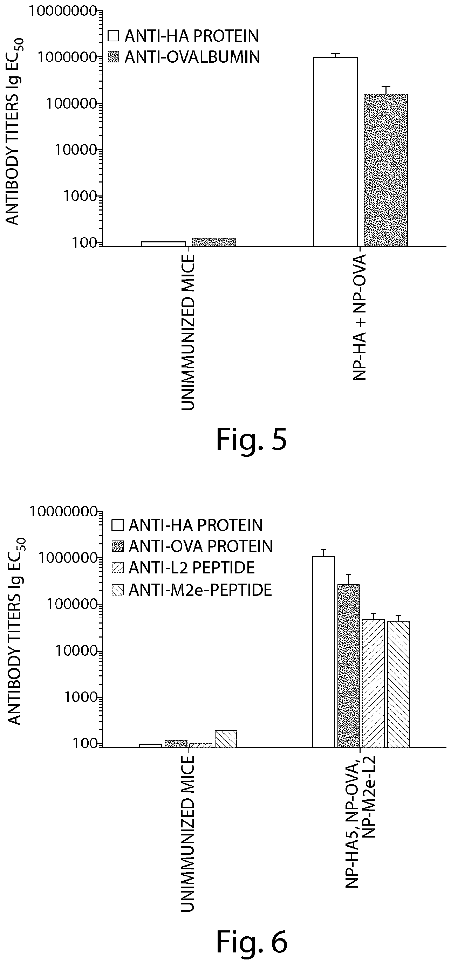

Anti-HA5 protein and anti-ovalbumin protein antibody titers in unimmunized mice and mice injected with NC-HA5 and NC-OVA (as prepared in Example 4) (5 animals/group; s.c., 100 µg of each NC per injection, 2 times with 3-wk intervals) were measured. Titers for day 33 after immunization with NC are shown in Fig. 5 (ELISA against H5N1 HA protein or ovalbumin protein) (group 1: unimmunized; group 2: immunized with NC-HA5 and NC-OVA).

Mice were injected with 100 µg of NC-HA5 protein (nanocarrier exhibiting influenza H5N1 HA protein on the outer surface and containing OP-II helper peptide and R848 adjuvant in the NC) and 100 µg of NC- OVA (nanocarrier exhibiting ovalbumin on the outer surface and containing OP-II helper peptide and R848 adjuvant in the NC) (subcutaneously, hind limbs) at 3-week intervals (days 0 and 21). Serum anti-HA5 and anti-ovalbumin antibody titers were measured at day 33 after immunization with NC. Anti-HA5 and anti-ovalbumin antibody titers (EC₅₀) as measured by ELISA against H5N1 HA protein and ovalbumin protein are shown (Fig. 5). Titers for control unimmunized mice are also shown. These results demonstrate that mice immunized with a combination of two monovalent protein antigen nanocarriers (NC-HA5 and NC-OVA) generate antibodies to both protein antigens.

### Example 10: Immunization with Two Monovalent and One Bivalent Antigen Nanocarriers Leads to Immune Response to all Four Antigens

Anti-HA, anti-ovalbumin, anti-M2e peptide, and anti-L2 peptide antibody titers in unimmunized mice and mice injected with NC-HA5, NC-OVA, and NC-M2e-L2 (as prepared in Example 4) (5 animals/group; s.c., 100 µg of each NC per injection, 2 times at 3-wk intervals) were measured. Titers for day 33 after immunization with NC are shown in Fig. 6 (ELISA against HA protein, ovalbumin protein, PLA-PEG-M2e peptide, or PLA-PEG-L2 peptide) (group 1: unimmunized; group 2: immunized with NC-HA5, NC-OVA, and NC-M2e-L2).

Mice were injected with 100 µg of NC-HA5 protein (nanocarrier exhibiting influenza H5N1 HA protein on the outer surface and containing OP-II helper peptide and R848 adjuvant in the NC), 100 µg of NC-OVA (nanocarrier exhibiting ovalbumin on the outer surface and containing OP-II helper peptide and R848 adjuvant in the NC), and 100 µg of NC-M2e-L2 (nanocarrier exhibiting influenza M2e peptide (aa2-27) and HPV L2 peptide (aa17-36) on the outer surface and containing OP-II helper peptide and R848 adjuvant in the NC) (subcutaneously, hind limbs) at 3-week intervals (days 0 and 21). Serum anti-HA, anti-ovalbumin, anti-M2e peptide, and anti-L2 peptide antibody titers were measured at day 33 after immunization with NC. Anti-HA, anti-ovalbumin, anti-M2e peptide, and anti-L2 peptide antibody titers (EC₅₀) as measured by ELISA against HA protein, ovalbumin protein, M2e peptide, and L2 peptide are shown (Fig. 6). Titers for control unimmunized mice are also shown. These results demonstrate that mice immunized with a combination of two monovalent and one bivalent antigen nanocarrier (NC-HA5, NC-OVA, and NC-M2e-L2) generate antibodies to all four antigens.

### Example 11: Immunization with Two Monovalent and One Bivalent Antigen Nanocarriers Leads to Immune Response to all Four Antigens

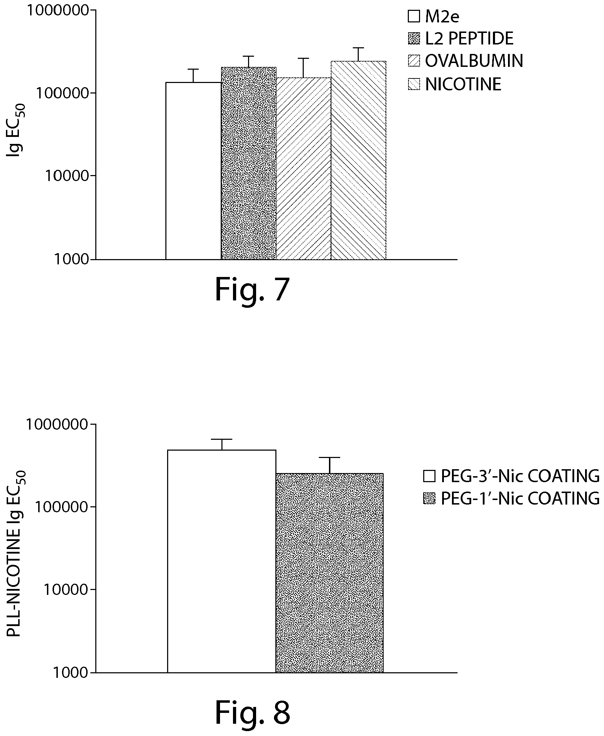

Antibody titers in mice immunized with a combination of NC-M2e, NC-L2 peptide and NC-nicotine-ovalbumin (as prepared in Example 4) were measured. NC-M2e and NC-L2 peptide contained OP-II T-helper peptide (2.0% and 2.4%, correspondingly) and R848 adjuvant (3.6% and 4.3%, correspondingly); NC-nicotine-ovalbumin contained R848 adjuvant (4.2%). Each bar of Fig. 7 represents the titer against antigen. Five animals per group were immunized s.c. with 120 µg of each NC per injection, 2 times at 3-wk intervals. Titers for day 33 after the first immunization are shown (ELISA done against PLA-PEG-M2e, PLA-PEG-L2, ovalbumin and polylysine-nicotine, correspondingly).

These results demonstrate that immunization with a combination of two NCs each carrying a different peptide antigen together with a NC carrying another two antigens results in generation of antibodies to all four NC-carried antigens. When identical amounts of three NC, the first containing surface M2e peptide from influenza A virus (ectodomain of M2 matrix protein, amino acids 2-27), the second containing surface L2 peptide from HPV virus (amino acids 17-36 from L2 capsid protein of HPV-16), and the third carrying surface nicotine and ovalbumin protein were used for animal immunization, a strong humoral response was induced in all animals against all four NC-coupled antigens (Fig. 7). No reactivity was detected in the sera of preimmune mice.

### Example 12: Immunization with Two Monovalent Nanocarriers with Antigen in Different Steric Orientations Leads to Immune Response both Orientations

### Preparation of NC-3'-Nicotine

Ovalbumin peptide 323-339 amide acetate salt, was purchased from Bachem Americas Inc. (3132 Kashiwa Street, Torrance CA 90505. Product code 4065609.) PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-Nicotine (S-642), poly-D/L lactide-block-poly(ethylene glycol)-(±)-trans-3'-hydroxymethylnicotine ether with PEG block of approximately 5,000 Da and PLA block of approximately 21,000 Da was custom manufactured at Princeton Global Synthesis (300 George Patterson Drive #206, Bristol, PA 19007.) Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: Ovalbumin peptide 323-339 @ 20mg/mL was prepared in 0.13N HCl at room temperature.
Solution 2: PLGA-R848 @ 50 mg/mL, PLA-PEG-Nicotine @ 25 mg/mL, and PLA @ 25 mg/mL in dichloromethane were prepared by dissolving each polymer separately in dichloromethane at 100 mg/mL then combining 2 parts PLGA-R848 solution to 1 part each PLA-PEG-Nicotine solution and PLA solution.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 40 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated, and then the pellet was re-suspended in phosphate buffered saline to achieve a nanocarrier suspension having a nominal concentration of 10 mg/mL on a polymer basis. The nanocarrier suspension was stored frozen at -20°C until further use.

**Table 8: NC-3'-Nicotine Characterization**

| Nanocarrier | Effective Diameter (nm) | TLR Agonist, % w/w | T-cell agonist, % w/w |
|---|---|---|---|
| NC-3'-Nicotine | 193 | R848, 4.2 | Ova 323-339 peptide, 2.1 |

### Preparation of NC-1'-Nicotine

Ovalbumin peptide 323-339 amide acetate salt, was purchased from Bachem Americas Inc. (3132 Kashiwa Street, Torrance CA 90505. Product code 4065609.) PLGA-R848, poly-D/L-lactide-co-glycolide, 4-amino-2-(ethoxymethyl)-α,α-dimethyl-1H-imidazo[4,5-c]quinoline-1-ethanol amide of approximately 7,000 Da made from PLGA of 3:1 lactide to glycolide ratio and having approximately 8.5% w/w conjugated resiquimod content was custom manufactured at Princeton Global Synthesis (300 George Patterson Drive #206, Bristol, PA 19007.) PLA-PEG-1'-Nic, a block co-polymer consisting of a poly-D/L-lactide (PLA) block of approximately 23000 Da and a polyethylene glycol (PEG) block of approximately 2000 Da that is conjugated to nicotine via a 4-carbon linkage to the 1' amino group on nicotine was synthesized. In brief, nicotine with a butyl-alcohol linker at the 1' position was made into HO-PEG-1'-Nic by polymerization with ethylene oxide, and the PLA extension was then generated by ring-opening polymerization of the HO-PEG-1'-Nic with dl-lactide. PLA with an inherent viscosity of 0.22 dL/g was purchased from SurModics Pharmaceuticals (756 Tom Martin Drive, Birmingham, AL 35211. Product Code 100 DL 2A.) Polyvinyl alcohol PhEur, USP (85-89% hydrolyzed, viscosity of 3.4-4.6 mPa.s) was purchased from EMD Chemicals Inc. (480 South Democrat Road Gibbstown, NJ 08027. Part Number 4-88).

Solutions were prepared as follows:
Solution 1: Ovalbumin peptide 323-339 @ 20mg/mL was prepared in 0.13N HCl at room temperature.
Solution 2: PLGA-R848 @ 50 mg/mL, PLA-PEG-1'-Nic @ 25 mg/mL, and PLA @ 25 mg/mL in dichloromethane was prepared by dissolving each polymer separately in dichloromethane at 100 mg/mL then combining 2 parts PLGA-R848 solution to 1 part each PLA-PEG-1'-Nic solution and PLA solution.
Solution 3: Polyvinyl alcohol @ 50 mg/mL in 100 mM in 100mM phosphate buffer, pH 8.
Solution 4: 70 mM phosphate buffer, pH 8.

A primary (W1/O) emulsion was first created using Solution 1 and Solution 2. Solution 1 (0.2 mL) and Solution 2 (1.0 mL) were combined in a small glass pressure tube and sonicated at 50% amplitude for 40 seconds using a Branson Digital Sonifier 250. A secondary (W1/O/W2) emulsion was then formed by adding Solution 3 (2.0 mL) to the primary emulsion, vortexing to create a course dispersion, and then sonicating at 30% amplitude for 60 seconds using the Branson Digital Sonifier 250.

The secondary emulsion was added to an open 50 mL beaker containing 70 mM phosphate buffer solution (30 mL) and stirred at room temperature for 2 hours to allow the dichloromethane to evaporate and the nanocarriers to form in suspension. A portion of the suspended nanocarriers was washed by transferring the nanocarrier suspension to a centrifuge tube, spinning at 21,000 rcf for 45 minutes, removing the supernatant, and re-suspending the pellet in phosphate buffered saline. This washing procedure was repeated, and then the pellet was re-suspended in phosphate buffered saline to achieve a nanocarrier suspension having a nominal concentration of 10 mg/mL on a polymer basis. The nanocarrier suspension was stored frozen at -20°C until further use.

**Table 9: NC-1'-Nicotine Characterization**

| Nanocarrier | Effective Diameter (nm) | TLR Agonist, % w/w | T-cell agonist, % w/w |
|---|---|---|---|
| NC-1'-Nicotine | 238 | R848, 3.9 | Ova 323-339 peptide, 2.8 |

### Immunization and Results

Antibody titers in mice immunized with a combination of NC-3'-nicotine and NC-1'-nicotine were measured. NC-3'-nicotine and NC-1'-nicotine contained OP-II T-helper peptide (2.1%) and R848 adjuvant (4.2%). Each bar of Fig. 8 represents the titer against antigen. Five animals per group were immunized s.c. with 120 µg of each NC per injection, 2 times at 3-wk intervals. Titers for day 33 after the first immunization are shown (ELISA done against polylysine-nicotine, respectively).

These results show that immunization with a combination of two NCs each carrying the same antigen but in different steric orientations results in the generation of antibodies against both of these different orientations of the same antigen. When identical amounts of two NCs, the first containing surface nicotine attached to NC in the 3'-position and the second attached to NC in the 1'-position were used for animal immunization, a strong humoral response was induced in all animals against both orientations of nicotine (Fig. 8). No reactivity was detected in the sera of preimmune mice.

### Example 13: Preparations of Polymers and Nanocarriers

### Preparation of PLGA-R848

PLGA-R848 was prepared by reaction of PLGA polymer containing an acid end group with R848 in the presence of coupling agent such as HBTU as follows. A mixture of PLGA (Lakeshores Polymers, MW ∼5000, 7525DLG1A, acid number 0.7 mmol/g, 10 g, 7.0 mmol) and HBTU (5.3 g, 14 mmol) in anhydrous EtOAc (160 mL) was stirred at room temperature under argon for 50 minutes. Compound R848 (2.2 g, 7 mmol) was added, followed by diisopropylethylamine (DIPEA) (5 mL, 28 mmol). The mixture was stirred at room temperature for 6 h and then at 50-55°C overnight (about 16 h). After cooling, the mixture was diluted with EtOAc (200 mL) and washed with saturated NH4Cl solution (2 x 40 mL), water (40 mL) and brine solution (40 mL). The solution was dried over Na2SO4 (20 g) and concentrated to a gel-like residue. Isopropyl alcohol (IPA) (300 mL) was then added and the polymer conjugate precipitated out of solution. The polymer was then washed with IPA (4 x 50 mL) to remove residual reagents and dried under vacuum at 35-40°C for 3 days as a white powder (10.26 g, MW by GPC is 5200, R848 loading is 12% by HPLC).

In a similar manner, PLA-R848 was prepared by the reaction of PLA-CO2H (polylactide with acid ending group) with R848.

### Preparation of PLA-PEG-CO2H

A mixture of HO-PEG-CO2H (MW: 2000, 1.0 g, 0.5 mmol), dl-lactide (10.8 g, 75 mmol) and Na2SO4 (15 g) in a 100 mL round bottom flask was dried under vacuum at 60°C for 2 days. Anhydrous toluene (30 ML) was added, and the mixture was heated to reflux under argon. Sn(Oct)2 (0.162 mL, 0.5 mmol) was added. The mixture was refluxed under argon overnight and cooled to ambient room temperature. The mixture was diluted with CH2Cl2 (200 mL) and filtered through a pad of Celite. The filtrate was concentrated to a dense sticky residue. 10% MeOH in diethyl ether (200 mL) was added to precipitate out the polymer with vigorous stirring. The polymer was further washed with 10% MeOH in ether (100 mL) and dried under vacuum at 30°C to give the PLA-PEG-CO2H copolymer as an off-white foamy solid (10.0 g, H NMR in CDCl3 showed the polymer has MW of 21000).

### Preparation of PLA-PEG-NH2

A mixture of HO-PEG-NH2.HCl (MW: 3500, 1.0 g, 0.28 mmol), dl-lactide (6.1 g, 42 mmol) and Na2SO4 (10 g) in a 100 mL round bottom flask was dried under vacuum at 60°C for 1 day. Anhydrous toluene (30 ML) was added and the mixture was heated to 90°C under argon. Sn(Oct)2 (0.1 mL, 0.28 mmol) was added. The mixture was refluxed under argon overnight and cooled to ambient room temperature. The mixture was diluted with ethyl acetate (200 mL) and filtered through a pad of Celite. The filtrate was concentrated to a dense sticky residue. 10% MeOH in t-butyl methyl ether (MTBE) (200 mL) was added to precipitate out the polymer with vigorous stirring. The polymer was further washed with 5% MeOH in MTBE (50 mL) and MTBE (50 mL) and dried under vacuum at 30°C to give the PLA-PEG-NH2.HCl copolymer as an off-white foamy solid (5.0 g, H NMR in CDCl3 showed the polymer has MW of 18000).

### Preparation of PLA-PEG-PEG3-N3

PLA-PEG-N3 polymer was prepared by ring opening polymerization of HO-PEG-azide with dl-lactide in the presence of a catalyst such as Sn(Oct)2 as follows. HO-PEG-CO2H (MW 3500, 1.33 g, 0.38 mmol) was treated with NH2-PEG3-N3 (MW 218.2, 0.1 g, 0.458 mmol) in the presence of DCC (MW 206, 0.117 g, 0.57 mmol) and NHS (MW 115, 0.066 g, 0.57 mmol) in dry DCM (10 mL) overnight. After filtration to remove insoluble byproduct (DCC-urea), the solution was concentrated and then diluted with ether to precipitate out the polymer, HO-PEG-PEG3-N3 (1.17 g). After drying, HO-PEG-PEG3-N3 (MW 3700, 1.17 g, 0.32 mmol) was mixed with dl-lactide (recrystallized from EtOAc, MW 144, 6.83 g, 47.4 mmol) and Na2SO4 (10 g) in a 100 mL flask. The solid mixture was dried under vacuum at 45°C overnight and dry toluene (30 mL) was added. The resulting suspension was heated to 110°C under argon and Sn(Oct)2 (MW 405, 0.1 mL, 0.32 mmol) was added. The mixture was heated at reflux for 18 h and cooled to rt. The mixture was diluted with DCM (50 mL) and filtered. After concentration to an oily residue, MTBE (200 mL) was added to precipitate out the polymer which was washed once with 100 mL of 10% MeOH in MTBE and 50 mL of MTBE. After drying, PLA-PEG-PEG3-N3 was obtained as a white foam (7.2 g, average MW: 23,700 by H NMR).

### Preparation of PLA-PEG-C6-N3

HO-PEG-CO2H (MW 3500, 1.00 g, 0.29 mmol) was treated with 6-azido-1-hexylamine (H2N-C6-N3) (MW 142, 0.081 g, 0.57 mmol) in the presence of DCC (MW 206, 0.118 g, 0.57 mmol) and NHS (MW 115, 0.066 g, 0.57 mmol) in dry DCM (10 mL) overnight. After filtration to remove insoluble byproduct (DCC-urea), the solution was concentrated and then diluted with MTBE to precipitate out the polymer which was then washed twice with MTBE and dried under vacuum at 30°C overnight to give HO-PEG-C6-N3 polymer (1.1g). HO-PEG-C6-N3 polymer (1.1 g, 0.29 mmol) and dl-lactide (6.5 g, 45 mmol) were mixed in dry toluene (60 mL). The mixture was heated to reflux while 30 mL of toluene was removed by azeotrope distillation. The resulting solution was cooled to 100°C and Sn(Oct)2 (0.095 mL, 0.29 mmol) was added. The solution was heated at reflux under argon overnight and cooled to rt. The solution was then added to 150 mL of 2-propanol to precipitate out the polymer which was washed with 2-propanol (100mL) and dried under vacuum at 30°C for 2 days to give PLA-PEG-C6-N3 copolymer as an off-white solid (6.8 g, MW by GPC is 27000 with DPI of 1.5).

### Preparation of PLA-PEG(5K)-CONH2NH2

A mixture of HO-PEG(5k)-CO2H (JenKem Technology, USA) (MW: 5000, 1.0 g, 0.2 mmol), tert-butyl carbazate (Boc-hydrazide) (MW: 132, 0.053 g, 0.4 mmol), DCC (MW 206, 0.083 g, 0.4 mmol) and N-hydroxysuccinimide (NHS) (MW 115, 0.05 g, 0.4 mmol) in dry DCM (15 mL) was stirred at rt for 25 h. The insoluble DCC-urea was removed by filtration and the filtrate was concentrated. The residual was added to 50 mL of MTBE to precipitate out the polymer which was washed twice with 40 mL of MTBE and dried under vacuum for 2 days to give HO-PEG(5k)-CONHNHtBoc as a white powder (1.07 g). HO-PEG(5k)-CONHNHtBoc polymer (1.07 g, 0.20 mmol) and dl-lactide (4.32 g, 30 mmol) were mixed in dry toluene (70 mL). The mixture was heated to reflux while 50 mL of toluene was removed by azeotrope distillation. The resulting solution was cooled to 100°C and Sn(Oct)2 (0.065 mL, 0.20 mmol) was added. The solution was heated at reflux under argon for 22 h and cooled to rt. The solution was then added to 150 mL of 2-propanol to precipitate out the polymer which was washed with 2-propanol (60 mL) and dried under vacuum at 30°C for 2 days to give PLA-PEG(5k)-CONHNHtBoc copolymer as a white solid chunk. The polymer was dissolved in 50 mL of dry DCM and cooled with ice water. Trifluoroacetic acid (TFA) (15 mL) was added and the resulting solution was stirred at rt overnight. The yellowish solution was concentrated to dryness. The residual was added to 200 mL of 2-propanol to precipitate out the polymer which was washed with 100 mL of 2-propanol. The polymer was dried at 30°C under vacuum to give the desired polymer as PLA-PEG(5k)-CONHNH2 (3.4 g, MW by NMR: 24000).

### Preparation of PLA-PEG-MAL

HO-PEG(3K)-maleimide (HO-PEG-MAL) (Laysan Bio, Inc) (MW: 3000, 0.6 g, 0.2 mmol) was mixed with dl-lactide (recrystallized from EtOAc, MW 144, 4.32 g, 30 mmol) and Na2SO4 (4 g) in a 100 mL flask. The solid mixture was dried under vacuum at 60°C overnight and dry toluene (20 mL) was added. The resulting suspension was heated to 110°C under argon and Sn(Oct)2 (MW 405, 0.065 mL, 0.2 mmol) was added. The mixture was heated at reflux for 20 h and cooled to rt. The mixture was diluted with DCM (50 mL) and filtered. After concentration to an oily residue, 10% MeOH in ethyl ether (80 mL) was added to precipitate out the polymer which was washed once with 80 mL of 10% MeOH in ether and 60 mL of ether. After drying at 30°C under vacuum overnight, PLA-PEG(3K)-MAL was obtained as a white foam (3.26 g, average MW: 24,000 by H NMR).

### Preparation of PLA-PEG-SH (Prophetic)

PLA-PEG-SH copolymer is prepared according to the literature (Nisha C. Kalarickal, et al; Macromolecules 2007, 40:1874-1880). Briefly, the following steps are performed.
Step-1. Preparation of tBuS-PEG: Anhydrous THF (22 mL), potassium naphthalene (0.2 M solution in THF, 12 mL), and tBu-SH (0.54 mL, 4.8 mmol) are charged into a sealed 100 mL round-bottom flask. The components are stirred for at least 15 min to ensure the formation of thiolates, at which point liquid ethylene oxide (EO) (11.5 mL, 0.230 mol) is added using a two-headed needle. The polymerization reaction is carried out for 48 h, and the product is recovered by precipitation in cold diethyl ether. MW of the polymer by GPC is about 2100.
Step-2. Preparation of (PEG-S)2: tBu-S-PEG from Step-1 (1.0 g) is dissolved in DMSO (19 mL) followed by addition of TFA (106 mL, 15/85 v/v) to a final polymer concentration of 8 mg/mL. The reaction is stirred for 20 min, after which TFA is removed by rotary evaporation. The residual is then precipitated twice in cold diethyl ether to recover the crude PEG disulfide. The crude (PEG-S)2 is further purified by fractional precipitation. Thus, the polymer (1.0 g) is dissolved in dichloromethane (100 mL), and then cold diethyl ether is added stepwise with stirring until the appearance of a precipitate. The solution is further stirred for 30 min, and the precipitated mass is isolated by filtration and dried in vacuo. The recovery yield of PEG disulfide, (PEG-S)2, at the end of two to three fractional precipitations is in the range 55-60%.
Step-3. Preparation of (PLA-b-PEG-S)2 by ring-opening polymerization of dl-lactide: (PEG-S)2 (0.4 g, 0.10 mmol) and dl-lactide (4.32 g, 30 mmol) are mixed in dry toluene (70 mL). The mixture is heated to reflux while 50 mL of toluene is removed by azeotrope distillation. The resulting solution was cooled to 100°C and Sn(Oct)2 (0.065 mL, 0.20 mmol) was added. The solution is heated at reflux under argon for 18-20 h and cooled to rt. The solution is then added to 150 mL of 2-propanol to precipitate out the polymer which is washed with 2-propanol (60 mL) and ether (60 mL) and dried under vacuum at 30°C for 2 days to give (PLA-PEG-S)2 (ca. 4.0 g, MW: 46000).
Step-4. Preparation of PLA-PEG-SH by reduction of (PLA-PEG-S)2: The (PLA-PEG-S)2 from Step-3 (3.2 g, 0.07 mmol) is dissolved in deoxygenated THF (25 mL), and Bu3P (1.7 mL, 7.0 mmol, 100 equiv with respect to disulfide units) is added. The reaction mixture is stirred under argon at room temperature overnight. The reduced thiolated polymer is recovered by precipitation in cold diethyl ether followed by filtration under argon atmosphere and further dried under vacuum to give PLA-PEG-SH as an off white chunky solid (ca. 3.0, MW: 23000).

### Preparation of Nanocarriers with Surface PEG-X Containing Encapsulated Ova Peptide

Nanocarriers comprising PLGA-R848, PLA-PEG-X (where X= carboxylic acid (CO2H), amine (NH2), C6-azide (C6-N3) or PEG3-azide (PEG3-N3), hydrazide (CONHNH2), maleimide (MAL), thiol (SH) and nitrilotriacetic acid group (NTA)) containing ova peptide were prepared via a double emulsion method wherein the ova peptide was encapsulated in the nanocarriers. Polyvinyl alcohol (Mw = 11 KD - 31 KD, 87-89% partially hydrolyzed) was purchased from JT Baker. Ovalbumin peptide 323-339, (sequence: H-Ile-Ser-Gln-Ala-Val-His-Ala-Ala-His-Ala-Glu-Ile-Asn-Glu-Ala-Gly-Arg-NH2, acetate salt, Lot# B06395) was obtained from Bachem Americas Inc. (3132 Kashiwa Street, Torrance CA 90505), PLA with acid end group (100DL2A) was obtained from SurModics Pharmaceuticals (756 Tom Martin Drive, Birmingham, AL 35211); PLGA-R848, and PLA-PEG-X conjugates were prepared as described above in this same example.

The above materials were used to prepare the following solutions:
1. PLGA-R848 conjugate in methylene chloride @ 100 mg/mL,
2. PLA-PEG-X in methylene chloride @ 100 mg/mL,
3. PLA (100DL2A) in methylene chloride @ 100 mg/mL,
4. Ovalbumin peptide 323 - 339 in 0.13N HCl @ 70 mg/mL, and
5. Polyvinyl alcohol in 100mM pH 8 phosphate buffer @50 mg/mL.

Solution #1 (0.50 mL), solution #2 (0.25 mL) and solution #3 (0.25 mL) were combined and solution #4 in 0.13N HCl (0.1mL) was added in a small vessel and the mixture was sonicated at 50% amplitude for 40 seconds using a Branson Digital Sonifier 250. To this emulsion was added solution # 5 (2.0 mL) and sonication at 30% amplitude for 40 seconds using the Branson Digital Sonifier 250 was performed on the second emulsion. This was then added to a stirring beaker containing a 70mM pH 8 phosphate buffer solution (30 mL), and this mixture was stirred at room temperature for 2 hours to form the nanocarriers.

To wash the nanocarriers, a portion of the nanocarrier dispersion (26.5mL) was transferred to a 50mL centrifuge tube and spun at 9500rpm (13,800g) for one hour at 4°C. The supernatant was removed, and the pellet was re-suspended in 26.5 mL of phosphate buffered saline. The centrifuge procedure was repeated and the pellet was re-suspended in 8.3g of phosphate buffered saline for a final nanocarrier dispersion of about 10 mg/mL containing encapsulated ova peptide.

### Preparation of Nanocarriers with surface PEG-X Without Encapsulated Ova Peptide

In a similar manner to the procedure described immediately above, nanocarriers without ova peptide were prepared where solution #4 was eliminated in the preparation.

### Example 14: Nanocarriers with Obtained Versus Derived Antigen (Prophetic)

Nanocarriers with PTH: Nanocarriers with surface PEG-CONHNH2 hydrazide groups are prepared as described above in Example 13. PTH (parathyroid hormone) protein is acylated via the lysine amino group with 4-formyl-benzoic acid in the presence of EDC. HCl and NHS are used to generate PTH containing benzaldehyde groups. After purification via dialfiltration with a MWCO 1K filter, the modified PTH is conjugated with the NCs containing the hydrazide on the surface in PBS buffer (pH 8-9). After purification by pellet washing with PBS buffer, the resulting NC-modified PTH conjugate is suspended in pH 7.4 buffer.

Nanocarriers with Modified PTH: Nanocarriers with surface PEG-CO2H group are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS. Modified PTH dissolved in the same PBS buffer is then added to the resulting NC suspension. The conjugation is allowed to proceed at 4°C overnight. After pellet washing with PBS buffer, the resulting NC-Modified PTH conjugate is suspended in pH 7.4 PBS buffer.

Equal portions of the two nanocarriers can then be combined to form a NC suspension for further testing.

### Example 15: Monovalent and Bivalent Nanocarriers with Antigens from the Same Genus of Infectious Agent (Prophetic)

Nanocarriers with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Human Influenza A virus HA protein trimer and HA M2e protein dissolved in pH 6.0 buffer is then added to the resulting NC suspension. The conjugation is allowed to proceed at 4°C overnight. After pellet washing with PBS buffer, the resulting NC-HA protein trimer/M2e protein conjugate is suspended in pH 7.4 PBS buffer.

In the same fashion, a NC-HA monomer protein conjugate is prepared using monomeric Human Influenza A virus HA protein.

The nanocarriers can then be combined to form a NC suspension for further testing.

### Example 16: Monovalent and Bivalent Nanocarriers with Antigens from a Different Genus of Infectious Agent (Prophetic)

Nanocarriers with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Human Influenza A virus HA protein trimer and HA M2e protein dissolved in pH 6.0 buffer is then added to the resulting NC suspension. The conjugation is allowed to proceed at 4°C overnight. After pellet washing with PBS buffer, the resulting NC-HA protein trimer/M2e protein conjugate is suspended in pH 7.4 PBS buffer.

In the same fashion, a NC- infectious salmon anemia virus conjugate is prepared using inactivated infectious salmon anemia virus.

The nanocarriers can then be combined to form a NC suspension for further testing.

### Example 17: Monovalent Nanocarriers with Antigens from the Same Species of Infectious Agent (Prophetic)

Nanocarriers with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Measles hemaglutinin antigen (a recombinant fragment containing the measles hemagglutinin immunodominant regions, amino acids 106-114 and 519-550) is dissolved in pH 6.0 buffer and then added to the resulting NC suspension. The conjugation is allowed to proceed at 4°C overnight. After pellet washing with PBS buffer, the resulting NC- measles hemaglutinin conjugate is suspended in pH 7.4 PBS buffer.

In the same fashion, NC- measles fusion antigen conjugate is prepared using a fragment of measles fusion protein (a recombinant fragment corresponding to amino acids 399-525 of measles large fusion protein).

The nanocarriers can then be combined to form a NC suspension for further testing.

### Example 18: Monovalent Nanocarriers with Antigens from Different Species of Infectious Agent (Prophetic)

Nanocarriers with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Human Influenza A virus HA protein trimer dissolved in pH 6.0 buffer is then added to the resulting NC suspension. The conjugation is allowed to proceed at 4°C overnight. After pellet washing with PBS buffer, the resulting NC-HA protein trimer conjugate is suspended in pH 7.4 PBS buffer for further testing.

Streptococcus pneumonia polysaccharide (PnPs) 6B is selected as a representative PnPs serotype. Purified native (i.e., no post purification size reduction) PnPs-6B is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, the resulting activated PnPs-6B solution is added to NCs with surface PEG-CONHNH2 (PEG-hydrazide) groups prepared as described above in Example 13 in pH 9 buffer. The resulting NCs and PnPs-6B suspension is shaken for 1 h and quenched with 2 M glycine solution. After pellet washing with PBS buffer, the resulting NC-PnPs-6B conjugate is suspended in pH 7.4 PBS buffer.

The nanocarriers can then be combined to form a NC suspension for further testing.

### Example 19: Monovalent Nanocarriers with Antigens from the Same Strain of Infectious Agent (Prophetic)

Nanocarriers with PEG-X on the surface are prepared as follows. Monodisperse PRINT nanocarriers (PRINT NCs) comprising PLGA-R848, PLA-PEG-X (where X= carboxylic acid (CO2H), amine (NH2), C6-azide (C6-N3) or PEG3-azide (PEG3-N3), hydrazide (CONHNH2), maleimide (MAL) and thiol (SH)) containing ova peptide are prepared by the Particle Replication in Non-wetting Templates (PRINT) method as described in the literature ((1) "Direct Fabrication and Harvesting of Monodisperse, Shape Specific Nano-Biomaterials"; Rolland, J. P.; Maynor, B. W.; Euliss, L. E.; Exner, A. E.; Denison, G. M.; DeSimone, J. M J. Am. Chem. Soc. 2005, 127, 10096; (2) "The Complex Role of Multivalency in Nanoparticles Targeting the Transferrin Receptor for Cancer Therapies" Jin Wang, Shaomin Tian, Robby A. Petros, Mary E. Napier and Joseph M. DeSimone; J. Am. Chem. Soc., 2010, 132 (32), pp 11306-11313). PRINT-NCs with surface PEG-CO2H groups are activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Pneumococcal surface protein A (PspA) dissolved in pH 6.0 buffer is then added to the resulting NC suspension. The conjugation is allowed to proceed at 4°C overnight. After pellet washing with PBS buffer, the resulting NC-PsPA conjugate is suspended in pH 7.4 PBS buffer.

Purified native PnPs-6B is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, the resulting activated PnPs-6B solution is added to PRINT NCs with surface PEG-CONHNH2 (PEG-hydrazide) groups prepared as described above in pH 9 buffer. The resulting NCs and PnPs-6B suspension is shaken for 1 h and quenched with 2 M glycine solution. After pellet washing with PBS buffer, the resulting NC-PnPs-6B conjugates are suspended in pH 7.4 PBS buffer.

The nanocarriers can then be combined to form a NC suspension for further testing.

### Example 20: Monovalent Nanocarriers with Antigens from Different Strains of Infectious Agent (Prophetic)

Purified native PnPs-6B is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, the resulting activated PnPs-6B solution is added to NCs with surface PEG-CONHNH2 (PEG-hydrazide) groups prepared as described above in Example 13 in pH 9 buffer. The resulting NCs and PnPs-6B suspension is shaken for 1 h and quenched with 2 M glycine solution. After pellet washing with PBS buffer, the resulting NC-PnPs-6B conjugates are suspended in pH 7.4 PBS buffer.

Purified native PnPs14 from is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, the resulting activated PnPs14 solution is added to PRINT NCs with surface PEG-CONHNH2 (PEG-hydrazide) groups prepared as described above in pH 9 buffer. The resulting PRINT NCs and PnPs14 suspension is shaken for 1 h and quenched with 2 M glycine solution. After pellet washing with PBS buffer, the resulting PRINT NC-PnPs14 conjugates are suspended in pH 7.4 PBS buffer.

Gold NCs with surface PEG-X (where X= carboxylic acid (CO2H), amine (NH2), azide (N3), hydrazide (CONHNH2) and aldehyde (CHO)) are prepared as follows.
Step-1. Formation of Gold NCs (AuNCs): A aq. solution of 500 mL of 1 mM HAuCl4 is heated to reflux for 10 min with vigorous stirring in a 1 L round-bottom flask equipped with a condenser. A solution of 50 mL of 40 mM of trisodium citrate is then rapidly added to the stirring solution. The resulting deep wine red solution is kept at reflux for 25-30 min and the heat is withdrawn and the solution is cooled to room temperature. The solution is then filtered through a 0.8 µm membrane filter to give the AuNCs solution. The AuNCs are characterized using visible spectroscopy and transmission electron microscopy. The AuNCs are ca. 20 nm diameter capped by citrate with peak absorption at 520 nm.
Step-2. AuNCs functionalized with PEG-X using HS-PEG-X: AuNCs are functionalized with HS-PEG-X (MW range: 1500-5000) (where X= carboxylic acid (CO2H), amine (NH2), azide (N3), hydrazide (CONHNH2) and aldehyde (CHO)) as follows. A solution of 150 µl of HS-PEG-X (10 µM in 10 mM pH 9.0 carbonate buffer) is added to 1 mL of 20 nm diameter citrate-capped gold nanocarriers (1.16 nM) to produce a molar ratio of thiol to gold of 2500:1. The mixture is stirred at room temperature under argon for 1 hour to allow complete exchange of thiol with citrate on the gold nanocarriers. The AuNCs with PEG-X on the surface is then purified by centrifuge at 12,000g for 30 minutes. The supernatant is decanted and the pellet containing AuNC-PEG-X is re-suspended in appropriate PBS buffer for further bioconjugation with biomolecules. Purified native PnPs-19F from is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, the resulting activated PnPs-19F solution is added to AuNCs with surface PEG-CONHNH2 (PEG-hydrazide) groups prepared as described above in pH 9 buffer. The resulting AuNCs and PnPs-19F suspension is shaken for 1 h and quenched with 2 M glycine solution. After pellet washing with PBS buffer, the resulting AuNC-PnPs-19F conjugates are suspended in pH 7.4 PBS buffer.

The nanocarriers can then be combined to form a NC suspension for further testing.

### Example 21: Monovalent Nanocarriers with the Same Antigen but Different Orientation (Prophetic)

PRINT NCs with surface nicotine analog attached via the 3'-position are prepared from PLA-PEG-3-HO-MeNic copolymer derived from trans-3'-hydroxymethylnicotine (3-HO-MeNic), PLGA-R848 and ova peptide as described above. The resulting PRINT NCs containing surface 3'-substituted nicotine analog are suspended in pH 7.4 buffer.

In a similar manner, PRINT NCs with surface nicotine analog attached via the 1'-position are prepared from PLA-PEG-1-butyl-Nic copolymer derived from 1'-butyl nicotine (1-butyl-Nic), PLGA-R848 and ova peptide as described above. The resulting PRINT NCs containing surface 1'-substituted nicotine analog are suspended in pH 7.4 buffer.

The nanocarriers can then be combined to form a NC suspension for further testing.

### Example 22: Monovalent Nanocarriers with the Same Antigen but Different Conformation (Prophetic)

Nanocarriers with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Measles virus hemagglutinin noose epitope (HNE, H379-410, disulfide intact) dissolved in pH 6.0 buffer is then added to the resulting NC suspension. The conjugation is allowed to proceed at 4°C overnight. After pellet washing with PBS buffer, the resulting NC-HNE conjugates are suspended in pH 7.4 PBS buffer.

The highly conserved hemagglutinin noose epitope (HNE, H379-410) of the measles virus contains three cysteine residues, two of which (Cys386 and Cys394) form a disulfide bridge. The HNE peptide containing the disulfide bridge is reduced using dithiothreitol (DTT) in PBS buffer to give reduced HNE. NCs with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. The reduced HNE dissolved in pH 6.0 buffer is then added to the resulting NC suspension under argon in the presence of DTT. The conjugation is allowed to proceed at 4°C overnight under argon. After pellet washing with PBS buffer, the resulting NC-reduced HNE conjugates are suspended in pH 7.4 PBS buffer.

The nanocarriers can then be combined to form a NC suspension for further testing.

### Example 23: Monovalent and Bivalent Nanocarriers with the Small Molecule Antigens of Different Structure (Prophetic)

AuNCs with surface PEG-CO2H groups are prepared as described above. The AuNCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Trans-3'-aminomethylnicotine prepared from commercially available 4-cotininecarboxylic acid (US Patent Application: US2007/0129551 A1) in pH 6.0 buffer is added to the activated AuNCs. The conjugation is allowed to proceed at 4°C overnight. After pellet washing with PBS buffer, the resulting AuNC-nicotine conjugates are suspended in pH 7.4 PBS buffer.

VLPs with surface functional groups such as azide or alkyne for CuAAC click chemistry are prepared as described in the literature ("Surface Functionalization of Virus-Like Particles by Direct Conjugation Using Azide-Alkyne Click Chemistry", Kedar G. Patel and James R. Swartz; Bioconjugate Chem., 2011, 22 (3), pp 376-387). Cocaine analog containing alkyne or azide linker and methamphetamine analog containing alkyne or azide linker are prepared according to literature procedures as surface B-cell antigen epitopes. An equal molar mixture of cocaine analog and methamphetamine analog with azide linker is treated with VLPs containing surface alkyne group under standard CuAAC condition to give VLP-cocaine-methamphetamine conjugates.

The nanocarriers can then be combined to form a NC suspension for further testing.

### Example 24: Bivalent Nanocarriers with Oligosaccharide Antigens of Different Structure (Prophetic)

Purified PnPs-6B is size reduced with dilute acid or under sonication to give oligomeric PnPs-6B which is dissolved in 2 M NaCl. Similarly, purified PnPs-3 is size reduced with dilute acid or under sonication to give oligomeric PnPs-3 which is dissolved in 2 M NaCl. An equal molar mixed solution of oligomeric PnPs-6B and PnPs-3 is prepared from these solutions. A solution of 1-cyano-4-dimethylaminopyridinium tetrafluoroborate (CDAP) in CH3CN (100 mg /mL) is added (ratio of CDAP/PnPs: 1.5 mg/mg) to the mixed PnPs solution. The pH of the resulting solution is adjusted to 9 with 0.2 M of aqueous Et3N or dilutes of NaOH solution. After 3-4 min, the resulting activated oligomeric PnPs-6B/PnPs-3 solution is added to AuNCs with surface PEG-CONHNH2 (PEG-hydrazide) groups prepared as described above in pH 9 buffer. The resulting AuNCs and activated PnPs-6B/PnPs-3 suspension is shaken for 1 h and quenched with 2 M glycine solution. After pellet washing with PBS buffer, the resulting AuNC-PnPs-6B/3 conjugates are suspended in pH 7.4 PBS buffer.

VLPs containing carboxylic acid (CO2H) groups on the surface are activated with excess EDC/NHS in pH 6 PBS buffer at 4°C for 1-2 h. The activated VLPs are then pellet washed with pH 6.0 buffer to remove un-reacted EDC/NHS and suspended in pH 6.0 buffer. Purified PnPs-4 is size reduced with dilute acid or under sonication to give oligomeric PnPs-4 which is dissolved in 2 M NaCl. Similarly, purified PnPs-19F is size reduced with dilute acid or under sonication to give oligomeric PnPs-19F which is dissolved in 2 M NaCl. An equal molar mixed solution of oligomeric PnPs-4 and PnPs-19F is prepared from these solutions. A solution of 1-cyano-4-dimethylaminopyridinium tetrafluoroborate (CDAP) in CH3CN (100 mg /mL) is added (ratio of CDAP/PnPs: 1.5 mg/mg) to the mixed PnPs solution. The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated mixed PnPs-4/19F solution. The resulting solution is mixed for 1 h and quenched with 2 M glycine solution and purified by dialysis. The purified oligomeric PnPs-4/19F with ADH linker in pH 6.0 buffer is then added to the activated VLPs in pH 6.0 buffer and the resulting suspension is mixed at 4°C overnight and purified by dialysis or pellet wash to give VLP-PnPs-4/19F conjugates for further testing.

The nanocarriers can then be combined to form a NC suspension for further testing.

### Example 25: Bivalent Nanocarriers with Polysaccharide Antigens of Different Structure (Prophetic)

Purified native PnPs-6B is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated PnPs-6B solution. The resulting solution is mixed for 1 h and purified by dialysis. The purified PnPs-6B with ADH linker is dissolved in pH 6.0 buffer for NC conjugation.

Purified N. meningitidis meningococcal polysaccharide serogroup A (NmA) is dissolved in 1 M NaCl. A solution of 1-cyano-4-dimethylaminopyridinium tetrafluoroborate (CDAP) in CH3CN (100 mg /mL) is added (ratio of CDAP/NmA: 1.5 mg/mg). The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated NmA solution. The resulting solution is mixed for 1-2 h and purified by dialysis. The purified NmA with ADH linker is dissolved in pH 6.0 buffer for NC conjugation.

NCs with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. An equal molar mixed solution of PnPs-6B with ADH linker and NmA with ADH linker in pH 6.0 buffer is added to the activated NC solution, and the resulting suspension is mixed at 4°C overnight. After pellet washing with PBS buffer, the resulting NC-PnPs6B/NmA conjugates are suspended in pH 7.4 PBS buffer.

Purified native PnPs-19F is dissolved in 2 M NaCl. A solution of 1-cyano-4-dimethylaminopyridinium tetrafluoroborate (CDAP) in CH3CN (100 mg /mL) is added (ratio of CDAP/PnPs: 1.0 mg/mg). The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated PnPs-19F solution. The resulting solution is mixed for 1 h and purified by dialysis. The purified PnPs-19F with ADH linker is dissolved in pH 6.0 buffer for NC conjugation.

Purified N. meningitidis meningococcal polysaccharide serogroup C (NmC) is dissolved in 1 M NaCl. A solution of 1-cyano-4-dimethylaminopyridinium tetrafluoroborate (CDAP) in CH3CN (100 mg /mL) is added (ratio of CDAP/NmC: 1.5 mg/mg). The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated NmC solution. The resulting solution is mixed for 1-2 h and purified by dialysis. The purified NmC with ADH linker is dissolved in pH 6.0 buffer for NC conjugation.

NCs with surface PEG-CO2H groups are prepared as described above in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. An equal molar mixed solution of PnPs-19F with ADH linker and NmC with ADH linker in pH 6.0 buffer is added to the activated NC solution, and the resulting suspension is mixed at 4°C overnight. After pellet washing with PBS buffer, the resulting NC-PnPs-19F/NmC conjugates are suspended in pH 7.4 PBS buffer.

The nanocarriers can then be combined to form a NC suspension for further testing.

### Example 26: Bivalent and Monovalent Nanocarriers with Small Molecule Antigens in Different Orientations (Prophetic)

Nanocarriers with surface PEG-CONHNH2 (PEG-hydrazide) are prepared as described above in Example 13 and suspended in pH 6.0 buffer at 4°C. A cocaine analog GNC (6-(2R,3S)-3-(benzoyloxy)-8-methyl-8-azabicyclo [3.2.1] octane-2-carbonyloxy-hexanoic acid) is prepared according to a reported procedure ("Cocaine Analog Coupled to Disrupted Adenovirus: A Vaccine Strategy to Evoke High-titer Immunity Against Addictive Drugs" Martin J Hicks, et al, Mol Ther 2011, 19: 612-619). This compound is activated with EDC/NHS in DMF, and the activated GNC-NHS ester is isolated and purified for NC conjugation. Another cocaine analog, All is prepared according to a reported procedure ("Positional linker effects in haptens for cocaine immunopharmacotherapy", Akira Ino, Tobin J. Dickerson, and Kim D. Janda; Bioorganic & Medicinal Chemistry Letters 17 (2007) 4280-4283) and activated with EDC/NHS as above. An equal molar portion of each activated cocaine analog in excess to NC surface PEG-hydrazide is mixed with the NCs in pH 6.0 buffer. The resulting suspension is mixed at 4°C overnight. After pellet washing with PBS buffer, the resulting NC-GNC/AI1 cocaine conjugates are suspended in pH 7.4 PBS buffer.

Norcocaine is treated with succinic anhydride to give cocaine containing a succinic acid linker, SNC, and then activated with EDC/NHS according to a reported procedure (Fox BS, Kantak KM, Edwards MA et al. Efficacy of a therapeutic cocaine vaccine in rodent models. Nat. Med. 2(10), 1129-1132 (1996). NCs with surface PEG-CONHNH2 (PEG-hydrazide) are prepared as described above in Example 13 and suspended in pH 6.0 buffer at 4°C. Excess amounts of activated cocaine analog, SNC, is added to the NCs. The resulting suspension is mixed at 4°C overnight. After pellet washing with PBS buffer, the resulting NC-SNC cocaine conjugates are suspended in pH 7.4 PBS buffer.

The nanocarriers can then be combined to form a NC suspension for further testing.

### Example 27: Bivalent and Monovalent Nanocarriers with Peptide Antigens with Different Attachments (Prophetic)

Nanocarriers with surface PEG-azide (PEG-N3) are prepared according to Example 13 and suspended in de-gassed pH 7 buffer with argon. Ovalbumin (325-336) peptide with a C-terminal propargyl amide group (a C-alkyne group) is prepared by standard solid phase peptide synthesis, and the resulting purified Ova (325-336)-C-alkyne peptide is dissolved in pH 7 buffer under argon. Ovalbumin (325-336) peptide with the N-terminal amine acylated with 5-hexynoic acid (an N-terminal alkyne group) is prepared by standard solid phase peptide synthesis, and the resulting purified Ova (325-336)-N-alkyne peptide is dissolved in pH 7 buffer under argon. NCs with surface PEG-N3is mixed with an equal molar amount of each ova -C-alkyne and N-alkyne peptide in pH 7 buffer under argon, and the resulting suspension is subjected to the CuAAC click reaction according to a reported protocol ("Analysis and optimization of copper-catalyzed azide-alkyne cycloaddition for bioconjugation", Hong V, Presolski SI, Ma C, Finn MG.; Angew Chem Int Ed Engl. 2009;48(52):9879-83). The resulting NC-Ova peptide-C-linked/Ova peptide-N-linked conjugates are purified by pellet wash with pH 7 buffer and suspended in pH 7 buffer.

Recombinant virus-like particles (VLP) are prepared according to a standard procedure. In particular, VLPs from rabbit hemorrhagic disease virus is prepared and conjugated with Ova (323-339) peptide via a heterobifunctional linker such as Sulfosuccinimidyl 4-(N-maleimidomethyl) cyclohexane-1-carboxylate (Sulfo-SMCC) as described by Matthew Peacey, et al. ((1) Peacey M, Wilson S, Baird MA, Ward VK. "Versatile RHDV virus-like particles: incorporation of antigens by genetic modification and chemical conjugation" Biotechnol Bioeng; 2007; 98:968-77; (2) Peacey M, Wilson S, Perret R, Ronchese F, Ward VK, Young V, Young S, Baird, MA. "Virus-like particles from rabbit hemorrhagic disease virus can induce ananti-tumor response" Vaccine; 2008; 26:5334-5337). The resulting VLP-ova peptide conjugates are purified and suspended in pH 7 buffer.

The nanocarriers can then be combined to form a NC suspension for further testing.

### Example 28: Monovalent Nanocarriers with Protein Antigens Coupled at Different Attachment Points on the Protein (Activated Versus Protein Tag) (Prophetic)

Nanocarriers with surface PEG-CO2H groups are prepared as described in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Measles hemaglutinin protein (MHP) dissolved in pH 6.0 buffer is then added to the resulting NC suspension. The conjugation is allowed to proceed at 4°C overnight. After pellet washing with PBS buffer, the resulting NC- MHP conjugate is suspended in pH 7.4 PBS buffer.

Nanocarriers with surface PEG-NTA group for Ni-His tag complexation are prepared as described in Example 13. The NCs are then treated with a solution of NiCl2 in a binding buffer (50 mM phosphate buffer system, 300 mM NaCl, 10 mM imidazole, pH 8.0) to form the NCs with surface NTA-Ni complex. After pellet washing with PBS buffer, the resulting NCs are suspended in the binding buffer under argon. A solution of His6-tagged recombinant measles hemaglutinin protein in the binding buffer is added to the NC suspension, and the suspension is incubated at 4°C overnight under argon. The resulting NC-NTA-His6-MHP conjugates are pellet washed with pH 7 buffer and suspended in PBS buffer.

The nanocarriers can then be combined to form a NC suspension for further testing.

### Example 29: Monovalent Nanocarriers with Oligosaccharide Antigens Coupled at Different Attachment Points (Activated Hydroxyl Group Versus Linker) (Prophetic)

Nanocarriers with surface PEG-CONHNH2 (PEG-hydrazide) groups are prepared as described in Example 13 and suspended in pH 9 buffer under argon. Purified PnPs-6B is size reduced with dilute acid or under sonication to give oligomeric PnPs-6B which is dissolved in 2 M NaCl. A solution of 1-cyano-4-dimethylaminopyridinium tetrafluoroborate (CDAP) in CH3CN (100 mg /mL) is added (ratio of CDAP/PnPs: 1.5 mg/mg) to the PnPs-6B solution. The pH of the resulting solution is adjusted to 9 with 0.2 M of aqueous Et3N or dilutes of NaOH solution. After 3-4 min, the resulting activated oligomeric PnPs-6B solution is added to the NCs with surface PEG-CONHNH2 (PEG-hydrazide) groups. The resulting NCs and activated PnPs-6B suspension is shaken for 1 h and quenched with 2 M glycine solution. After pellet washing with PBS buffer, the resulting NC-PnPs-6B conjugates are suspended in pH 7.4 PBS buffer.

Nanocarriers with surface PEG-CO2H groups are prepared as described in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Oligo PnPs-6B with a 3-aminopropyl linker is prepared according to a reported method ("Synthetic 6B Di-, Tri-, and Tetrasaccharide-Protein Conjugates Contain Pneumococcal Type 6A and 6B Common and 6B- Specific Epitopes That Elicit Protective Antibodies in Mice", Jansen WTM, et al. Infect Immun. 2001; 69(2): 787-793). The oligomeric PnPs-6B-3-propylamine in pH 6 buffer is added to the activated NCs. The resulting suspension is mixed at 4°C overnight under argon. After pellet washing with PBS buffer, the NC-PnPs-6B conjugates are suspended in pH 7.4 PBS buffer.

The nanocarriers can then be combined to form a NC suspension for further testing.

### Example 30: Monovalent Nanocarriers with Polysaccharide Antigens Coupled at Different Attachment Points on the Polysaccharide (Prophetic)

NmA is attached via CDAP activated hydroxyl groups to NCs with multiple attachment points. Purified N. meningitidis meningococcal polysaccharide serogroup A (NmA) is dissolved in 1 M NaCl. A solution of 1-cyano-4-dimethylaminopyridinium tetrafluoroborate (CDAP) in CH3CN (100 mg /mL) is added (ratio of CDAP/NmA: 1.5 mg/mg). The pH of the resulting solution is adjusted to 9 with 0.2 M of aqueous Et3N or dilutes of NaOH solution. After 3-4 min, a solution of adipic acid dihydrazide (ADH) linker in pH 9 buffer is added to the activated NmA solution. The resulting solution is mixed for 1-2 h and purified by dialysis. The purified NmA with ADH linker is dissolved in pH 6.0 buffer for NC conjugation.

NCs with surface PEG-CO2H groups are prepared as described in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. A solution of NmA with ADH linkers in pH 6.0 buffer is added to the activated NC solution, and the resulting suspension is mixed at 4°C overnight. After pellet washing with PBS buffer, the resulting NC-NmA conjugates are suspended in pH 7.4 PBS bufferS.

NmA is attached to NCs via a terminal amino group. NCs with surface PEG-CO2H groups are prepared as described in Example 13. The NCs are then activated with excess EDC/NHS in pH 6 PBS buffer at 4°C for 1-2 h. The activated NCs are then pellet washed with pH 6.0 buffer to remove un-reacted EDC/NHS and suspended in pH 6.0 buffer. Purified NmA is subjected to reductive amination with NH4Cl and sodium cyanoborohydride (NaCNBH3) in pH 7 buffer to give the amino-NmA according to a reported procedure ("Development and phase 1 clinical testing of a conjugate vaccine against meningococcus A and C", Costantino P, Viti S, Podda A, Velmonte MA, Nencioni L, Rappuoli R. Vaccine. 1992;10(10):691-8). The amino-NmA is then added to the activated NCs suspension, and and the resulting suspension is mixed at 4°C overnight. After pellet washing with PBS buffer, the resulting NC-NmA conjugates are suspended in pH 7.4 PBS buffer.

The nanocarriers can then be combined to form a NC suspension for further testing.

Certain embodiments of the invention are set out in the following numbered paragraphs:
1. A composition comprising:
   a dosage form comprising:
      a first population of synthetic nanocarriers that comprise a first set of surface antigens;
      a second population of synthetic nanocarriers that comprise a second set of surface antigens; and
      a pharmaceutically acceptable excipient;
   wherein the first set of surface antigens and the second set of surface antigens are structurally different.
2. The composition of paragraph 1, wherein the first set of surface antigens comprises 1, 2, 3, 4, 5, 6, 7, 8, 9, 10 or more types of antigens.
3. The composition of paragraph 1 or 2, wherein the second set of surface antigens comprises 2, 3, 4, 5, 6, 7, 8, 9, 10 or more types of antigens.
4. The composition of any of paragraphs 1-3, wherein the first set of surface antigens comprise antigens obtained or derived from a first infectious genus and the second set of surface antigens comprise antigens obtained or derived from a second infectious genus.
5. The composition of paragraph 4, wherein the first infectious genus and the second infectious genus are the same.
6. The composition of any of paragraphs 1-5, wherein the first set of surface antigens comprise antigens obtained or derived from a first infectious species and the second set of surface antigens comprise antigens obtained or derived from of a second infectious species.
7. The composition of paragraph 6, wherein the first infectious species and the second infectious species are the same.
8. The composition of any of paragraphs 1-7, wherein the first set of surface antigens comprise antigens obtained or derived from a first infectious strain and the second set of surface antigens comprise antigens obtained or derived from a second infectious strain.
9. The composition of paragraph 8, wherein the first infectious strain and second infectious strain are the same.
10. The composition of any of paragraphs 1-9, wherein the first set of surface antigens and/or second set of surface antigens comprise antigens that are obtained or derived from a virus of the Adenoviridae, Picornaviridae, Herpesviridae, Hepadnaviridae, Flaviviridae, Retroviridae, Orthomyxoviridae, Paramyxoviridae, Papillomaviridae, Rhabdoviridae, Togaviridae or Paroviridae family.
11. The composition of paragraph 10, wherein the first set of surface antigens and/or second set of surface antigens comprise antigens that are obtained or derived from adenovirus, coxsackievirus, hepatitis A virus, poliovirus, Rhinovirus, Herpes simplex virus, Varicella-zoster virus, Epstein-barr virus, Human cytomegalovirus, Human herpesvirus, Hepatitis B virus, Hepatitis C virus, yellow fever virus, dengue virus, West Nile virus, HIV, Influenza virus, Measles virus, Mumps virus, Parainfluenza virus, Respiratory syncytial virus, Human metapneumovirus, Human papillomavirus, Rabies virus, Rubella virus, Human bocarivus or Parvovirus B19.
12. The composition of paragraph 11, wherein the first set of surface antigens and/or second set of surface antigens comprise antigens that are obtained or derived from VI, VII, E1A, E3-19K, 52K, VP1, surface antigen, 3A protein, capsid protein, nucleocapsid, surface projection, transmembrane proteins, UL6, UL18, UL35, UL38, UL19, early antigen, capsid antigen, Pp65, gB, p52, latent nuclear antigen-1, NS3, envelope protein, envelope protein E2 domain, gp120, p24, lipopeptides Gag (17-35), Gag (253-284), Nef (66-97), Nef (116-145), Pol (325-355), neuraminidase, nucleocapsid protein, matrix protein, phosphoprotein, fusion protein, hemagglutinin, hemagglutinin-neuraminidase, glycoprotein, E6, E7, envelope lipoprotein or non-structural protein (NS).
13. The composition of any of paragraphs 1-9, wherein the first set of surface antigens and/or second set of surface antigens comprise antigens that are obtained or derived from a bacteria of the Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema Vibrio or Yersinia genus.
14. The composition of paragraph 13, wherein the first set of surface antigens and/or second set of surface antigens comprise antigens that are obtained or derived from Bordetella pertussis, Borrelia burgdorferi, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diphtheriae, Enterococcus faecalis, Enterococcus faecium, Escherichia coli, Francisella tularensis, Haemophilus influenzae, Helicobacter pylori, Legionella pneumophila, Leptospira interrogans, Listeria monocytogenes, Mycobacterium leprae, Mycobacterium tuberculosis, Mycobacterium ulcerans, Mycoplasma pneumoniae, Neisseria gonorrhoeae, Neisseria meningitides, Pseudomonas aeruginosa, Rickettsia rickettsii, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae or Yersinia pestis.
15. The composition of paragraph 14, wherein the first set of surface antigens and/or second set of surface antigens comprise antigens that are obtained or derived from pertussis toxin (PT), filamentous hemagglutinin (FHA), pertactin (PRN), fimbriae (FIM 2/3), VlsE; DbpA, OspA, Hia, PrpA, MltA, L7/L12, D15, 0187, VirJ, Mdh, AfuA, L7/L12, out membrane protein, LPS, antigen type A, antigen type B, antigen type C, antigen type D, antigen type E, FliC, FliD, Cwp84, alpha-toxin, theta-toxin, fructose 1,6-biphosphate-aldolase (FBA), glyceraldehydes-3-phosphate dehydrogenase (GPD), pyruvate:ferredoxin oxidoreductase (PFOR), elongation factor-G (EF-G), hypothetical protein (HP), T toxin, Toxoid antigen, capsular polysaccharide, Protein D, Mip, nucleoprotein (NP), RD1, PE35, PPE68, EsxA, EsxB, RD9, EsxV, Hsp70, lipopolysaccharide, surface antigen, Sp1, Sp2, Sp3, Glycerophosphodiester Phosphodiesterase, outer membrane protein, chaperone-usher protein, capsular protein (Fl) or V protein.
16. The composition of any of paragraphs 1-9, wherein the first set of surface antigens and/or second set of surface antigens comprise antigens that are obtained or derived from a fungus of the Candida, Aspergillus, Cryptococcus, Histoplasma, Pneumocystis or Stachybotrys genus.
17. The composition of paragraph 16, wherein the first set of surface antigens and/or second set of surface antigens comprise antigens that are obtained or derived from C. albicans, Aspergillus fumigatus, Aspergillus flavus, Cryptococcus neoformans, Cryptococcus laurentii, Cryptococcus albidus, Cryptococcus gattii, Histoplasma capsulatum, Pneumocystis jirovecii or Stachybotrys chartarum.
18. The composition of paragraph 17, wherein the first set of surface antigens and/or second set of surface antigens comprise antigens that are obtained or derived from surface antigen, capsular glycoprotein, Yps3P, Hsp60, Major surface protein, MsgC1, MsgC3, MsgC8, MsgC9 or SchS34.
19. The composition of any of paragraphs 1-3, wherein the first set of surface antigens and second set of surface antigens comprise antigens obtained or derived from an abused or addictive substance.
20. The composition of paragraph 19, wherein the abused or addictive substance is cocaine or nicotine.
21. The composition of any of paragraphs 1-20, wherein the first set of surface antigens and the second set of surface antigens comprise the same surface antigens, and wherein at least one antigen of the first set of surface antigens is presented in a different orientation than as presented in the second set of surface antigens.
22. The composition of any of paragraphs 1-20, wherein the first set of surface antigens and the second set of surface antigens comprise the same surface antigens, and wherein at least one antigen of the first set of surface antigens is presented in a different conformation than as presented in the second set of surface antigens.
23. The composition of any of paragraphs 1-20, wherein the molecular structure of the first set of surface antigens and the second set of surface antigens are different.
24. The composition of any of paragraphs 1-23, wherein the first set of surface antigens and/or the second set of surface antigens comprise surface antigens with a molecular weight of less than 10,000 Da.
25. The composition of any of paragraphs 1-24, wherein the first set of surface antigens and/or the second set of surface antigens comprise surface antigens that comprise peptides, proteins, oligosaccharides, polysaccharides and/or small molecules.
26. The composition of paragraph 25, wherein at least one surface antigen of the first set of surface antigens and/or at least one surface antigen of the second set of surface antigens has a molecular weight of less than 10,000 Da.
27. The composition of any of paragraphs 24-26, wherein the first set of surface antigens comprises surface antigens comprising peptides, and the second set of surface antigens comprises surface antigens with a molecular weight of less than 10,000 Da.
28. The composition of any of paragraphs 24-26, wherein the first set of surface antigens comprises surface antigens comprising peptides, and the second set of surface antigens comprises surface antigens comprising peptides, proteins, oligosaccharides, polysaccharides and/or small molecules.
29. The composition of paragraph 28, wherein at least one surface antigen of the second set of surface antigens has a molecular weight of less than 10,000 Da.
30. The composition of any of paragraphs 24-26, wherein the first set of surface antigens comprises surface antigens comprising proteins, and the second set of surface antigens comprises surface antigens with a molecular weight of less than 10,000 Da.
31. The composition of any of paragraphs 24-26, wherein the first set of surface antigens comprises surface antigens comprising proteins, and the second set of surface antigens comprises surface antigens comprising peptides, proteins, oligosaccharides, polysaccharides and/or small molecules.
32. The composition of paragraph 31, wherein at least one surface antigen of the second set of surface antigens has a molecular weight of less than 10,000 Da.
33. The composition of any of paragraphs 24-26, wherein the first set of surface antigens comprises surface antigens comprising oligosaccharides, and the second set of surface antigens comprises surface antigens with a molecular weight of less than 10,000 Da.
34. The composition of any of paragraphs 24-26, wherein the first set of surface antigens comprises surface antigens comprising oligosaccharides, and the second set of surface antigens comprises surface antigens comprising peptides, proteins, oligosaccharides, polysaccharides and/or small molecules.
35. The composition of paragraph 34, wherein at least one surface antigen of the second set of surface antigens has a molecular weight of less than 10,000 Da.
36. The composition of any of paragraphs 24-26, wherein the first set of surface antigens comprises surface antigens comprising polysaccharides, and the second set of surface antigens comprises surface antigens with a molecular weight of less than 10,000 Da.
37. The composition of any of paragraphs 24-26, wherein the first set of surface antigens comprises surface antigens comprising polysaccharides, and the second set of surface antigens comprises surface antigens comprising peptides, proteins, oligosaccharides, polysaccharides and/or small molecules.
38. The composition of paragraph 37, wherein at least one surface antigen of the second set of surface antigens has a molecular weight of less than 10,000 Da.
39. The composition of any of paragraphs 24-26, wherein the first set of surface antigens comprises surface antigens comprising small molecules, and the second set of surface antigens comprises surface antigens with a molecular weight of less than 10,000 Da.
40. The composition of any of paragraphs 24-26, wherein the first set of surface antigens comprises surface antigens comprising small molecules, and the second set of surface antigens comprises surface antigens comprising peptides, proteins, oligosaccharides, polysaccharides and/or small molecules.
41. The composition of paragraph 40, wherein at least one surface antigen of the second set of surface antigens has a molecular weight of less than 10,000 Da.
42. The composition of any of paragraphs 1-41, further comprising one or more adjuvants.
43. The composition of paragraph 42, wherein the first population of synthetic nanocarriers and/or the second population of synthetic nanocarriers further comprise an adjuvant coupled to the synthetic nanocarriers.
44. The composition of paragraph 42 or 43, wherein the first population of synthetic nanocarriers and/or the second population of synthetic nanocarriers further comprise an adjuvant coupled to the synthetic nanocarriers and the composition comprises one or more admixed adjuvants.
45. The composition of any of paragraphs 42-44, wherein each of the one or more adjuvants comprises a mineral salt, alum, alum combined with monphosphoryl lipid (MPL) A of Enterobacteria, MPL® (AS04), AS15, a saponin, QS-21,Quil-A, ISCOMs, ISCOMATRIX™, MF59™, Montanide® ISA 51, Montanide® ISA 720, AS02, a liposome or liposomal formulation, AS01, AS15, synthesized or specifically prepared microparticles and microcarriers, bacteria-derived outer membrane vesicles of *N. gonorrheae* or *Chlamydia trachomatis,* chitosan particles, a depot-forming agent, Pluronic® block co-polymers, specifically modified or prepared peptides, muramyl dipeptide, an aminoalkyl glucosaminide 4-phosphate, RC529, a bacterial toxoid, a toxin fragment, an agonist of Toll-Like Receptors 2, 3, 4, 5, 7, 8 or 9, an adenine derivative, immunostimulatory DNA, immunostimulatory RNA, an imidazoquinoline amine, an imidazopyridine amine, a 6,7-fused cycloalkylimidazopyridine amine, a 1,2-bridged imidazoquinoline amine, imiquimod, resiquimod, an agonist for DC surface molecule CD40, a type I interferon, poly I:C, a bacterial lipopolysacccharide (LPS), VSV-G, HMGB-1, flagellin or portions or derivatives thereof, an immunostimulatory DNA molecule comprising CpG, proinflammatory stimuli released from necrotic cells, urate crystals, an activated component of the complement cascade, an activated component of immune complexes, a complement receptor agonist, a cytokine, or a cytokine receptor agonist.
46. The composition of any of paragraphs 43-45, wherein the adjuvants are different.
47. The composition of any of paragraphs 43-46, wherein the adjuvant coupled to the first population of synthetic nanocarriers and/or the adjuvant coupled to the second population of synthetic nanocarriers comprises a TLR-2, -3, -4, -7, -8 or -9 agonist.
48. The composition of paragraph 47, wherein the adjuvant coupled to the first population of synthetic nanocarriers and/or the adjuvant coupled to the second population of synthetic nanocarriers comprises an immunostimulatory nucleic acid, imidazoquinoline, oxoadenine, MPL, imiquimod or resiquimod.
49. The composition of any of paragraphs 44-48, wherein the admixed adjuvant is an immunostimulatory nucleic acid comprising CpG, AS01, AS02, AS04, AS15, QS-21, a saponin, alum or MPL.
50. The composition of any of paragraphs 1-49, wherein the first and second populations of synthetic nanocarriers are present in an amount effective to generate an immune response to the first set of surface antigens and the second set of surface antigens in a subject.
51. The composition of paragraph 50, wherein the immune response is the generation of antibody titers specific for the first set of surface antigens and the second set of surface antigens.
52. The composition of any of paragraphs 1-51, further comprising one or more additional populations of synthetic nanocarriers, wherein each additional population of synthetic nanocarriers comprises a set of surface antigens structurally different from the other sets of surface antigens in the composition.
53. The composition of paragraph 52, wherein at least one of the one or more additional populations of synthetic nanocarriers further comprise an adjuvant coupled thereto.
54. The composition of paragraph 53, wherein the adjuvant coupled to the at least one of the one or more additional populations of synthetic nanocarriers is different from the other adjuvants in the composition.
55. The composition of any of paragraphs 1-54, wherein each set of surface antigens is a monovalent or oligovalent set of surface antigens.
56. The composition of any of paragraphs 52-55, wherein the populations of synthetic nanocarriers are present in an amount effective to generate an immune response to each set of surface antigens.
57. The composition of paragraph 56, wherein the immune response is the generation of antibody titers specific for each set of surface antigens.
58. The composition of any of paragraphs 1-57, wherein the first and/or second population of synthetic nanocarriers further comprise a universal T cell antigen coupled thereto.
59. The composition of paragraph 58, wherein the universal T cell antigen comprises a T helper cell antigen.
60. The composition of paragraph 59, wherein the T-helper cell antigen comprises a peptide obtained or derived from ovalbumin.
61. The composition of paragraph 60, wherein the peptide obtained or derived from ovalbumin comprises the sequence as set forth in SEQ ID NO: 1.
62. The composition of any of paragraphs 58-61, wherein the universal T cell antigen is coupled by encapsulation.
63. A composition comprising:
   a dosage form comprising:
      a first population of synthetic nanocarriers that comprise a first set of surface antigens;
      a second population of synthetic nanocarriers that comprise a second set of surface antigens; and
      a pharmaceutically acceptable excipient;
   wherein the first set of surface antigens and the second set of surface antigens are immunologically different.
64. The composition of paragraph 63, wherein the first set of surface antigens comprises 1, 2, 3, 4, 5, 6, 7, 8, 9, 10 or more types of antigens.
65. The composition of paragraph 63 or 64, wherein the second set of surface antigens comprises 2, 3, 4, 5, 6, 7, 8, 9, 10 or more types of antigens.
66. The composition of any of paragraphs 63-65, wherein the first set of surface antigens comprise antigens obtained or derived from a first infectious genus and the second set of surface antigens comprise antigens obtained or derived from a second infectious genus.
67. The composition of paragraph 66, wherein the first infectious genus and the second infectious genus are the same.
68. The composition of any of paragraphs 63-67, wherein the first set of surface antigens comprise antigens obtained or derived from a first infectious species and the second set of surface antigens comprise antigens obtained or derived from of a second infectious species.
69. The composition of paragraph 68, wherein the first infectious species and the second infectious species are the same.
70. The composition of any of paragraphs 63-69, wherein the first set of surface antigens comprise antigens obtained or derived from a first infectious strain and the second set of surface antigens comprise antigens obtained or derived from a second infectious strain.
71. The composition of paragraph 70, wherein the first infectious strain and second infectious strain are the same.
72. The composition of any of paragraphs 63-71, wherein the first set of surface antigens and/or second set of surface antigens comprise antigens that are obtained or derived from a virus of the Adenoviridae, Picornaviridae, Herpesviridae, Hepadnaviridae, Flaviviridae, Retroviridae, Orthomyxoviridae, Paramyxoviridae, Papillomaviridae, Rhabdoviridae, Togaviridae or Paroviridae family.
73. The composition of paragraph 72, wherein the first set of surface antigens and/or second set of surface antigens comprise antigens that are obtained or derived from adenovirus, coxsackievirus, hepatitis A virus, poliovirus, Rhinovirus, Herpes simplex virus, Varicella-zoster virus, Epstein-barr virus, Human cytomegalovirus, Human herpesvirus, Hepatitis B virus, Hepatitis C virus, yellow fever virus, dengue virus, West Nile virus, HIV, Influenza virus, Measles virus, Mumps virus, Parainfluenza virus, Respiratory syncytial virus, Human metapneumovirus, Human papillomavirus, Rabies virus, Rubella virus, Human bocarivus or Parvovirus B19.
74. The composition of paragraph 73, wherein the first set of surface antigens and/or second set of surface antigens comprise antigens that are obtained or derived from VI, VII, E1A, E3-19K, 52K, VP1, surface antigen, 3A protein, capsid protein, nucleocapsid, surface projection, transmembrane proteins, UL6, UL18, UL35, UL38, UL19, early antigen, capsid antigen, Pp65, gB, p52, latent nuclear antigen-1, NS3, envelope protein, envelope protein E2 domain, gp120, p24, lipopeptides Gag (17-35), Gag (253-284), Nef (66-97), Nef (116-145), Pol (325-355), neuraminidase, nucleocapsid protein, matrix protein, phosphoprotein, fusion protein, hemagglutinin, hemagglutinin-neuraminidase, glycoprotein, E6, E7, envelope lipoprotein or non-structural protein (NS).
75. The composition of any of paragraphs 63-71, wherein the first set of surface antigens and/or second set of surface antigens comprise antigens that are obtained or derived from a bacteria of the Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema Vibrio or Yersinia genus.
76. The composition of paragraph 75, wherein the first set of surface antigens and/or second set of surface antigens comprise antigens that are obtained or derived from Bordetella pertussis, Borrelia burgdorferi, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diphtheriae, Enterococcus faecalis, Enterococcus faecium, Escherichia coli, Francisella tularensis, Haemophilus influenzae, Helicobacter pylori, Legionella pneumophila, Leptospira interrogans, Listeria monocytogenes, Mycobacterium leprae, Mycobacterium tuberculosis, Mycobacterium ulcerans, Mycoplasma pneumoniae, Neisseria gonorrhoeae, Neisseria meningitides, Pseudomonas aeruginosa, Rickettsia rickettsii, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae or Yersinia pestis.
77. The composition of paragraph 76, wherein the first set of surface antigens and/or second set of surface antigens comprise antigens that are obtained or derived from pertussis toxin (PT), filamentous hemagglutinin (FHA), pertactin (PRN), fimbriae (FIM 2/3), VlsE; DbpA, OspA, Hia, PrpA, MltA, L7/L12, D15, 0187, VirJ, Mdh, AfuA, L7/L12, out membrane protein, LPS, antigen type A, antigen type B, antigen type C, antigen type D, antigen type E, FliC, FliD, Cwp84, alpha-toxin, theta-toxin, fructose 1,6-biphosphate-aldolase (FBA), glyceraldehydes-3-phosphate dehydrogenase (GPD), pyruvate:ferredoxin oxidoreductase (PFOR), elongation factor-G (EF-G), hypothetical protein (HP), T toxin, Toxoid antigen, capsular polysaccharide, Protein D, Mip, nucleoprotein (NP), RD1, PE35, PPE68, EsxA, EsxB, RD9, EsxV, Hsp70, lipopolysaccharide, surface antigen, Sp1, Sp2, Sp3, Glycerophosphodiester Phosphodiesterase, outer membrane protein, chaperone-usher protein, capsular protein (Fl) or V protein.
78. The composition of any of paragraphs 63-71, wherein the first set of surface antigens and/or second set of surface antigens comprise antigens that are obtained or derived from a fungus of the Candida, Aspergillus, Cryptococcus, Histoplasma, Pneumocystis or Stachybotrys genus.
79. The composition of paragraph 78, wherein the first set of surface antigens and/or second set of surface antigens comprise antigens that are obtained or derived from C. albicans, Aspergillus fumigatus, Aspergillus flavus, Cryptococcus neoformans, Cryptococcus laurentii, Cryptococcus albidus, Cryptococcus gattii, Histoplasma capsulatum, Pneumocystis jirovecii or Stachybotrys chartarum.
80. The composition of paragraph 79, wherein the first set of surface antigens and/or second set of surface antigens comprise antigens that are obtained or derived from surface antigen, capsular glycoprotein, Yps3P, Hsp60, Major surface protein, MsgC1, MsgC3, MsgC8, MsgC9 or SchS34.
81. The composition of any of paragraphs 63-80, further comprising one or more adjuvants.
82. The composition of paragraph 81, wherein the first population of synthetic nanocarriers and/or the second population of synthetic nanocarriers further comprise an adjuvant coupled to the synthetic nanocarriers.
83. The composition of paragraph 81 or 82, wherein the first population of synthetic nanocarriers and/or the second population of synthetic nanocarriers further comprise an adjuvant coupled to the synthetic nanocarriers and the composition comprises one or more admixed adjuvants.
84. The composition of any of paragraphs 81-83, wherein each of the one or more adjuvants comprises a mineral salt, alum, alum combined with monphosphoryl lipid (MPL) A of Enterobacteria, MPL® (AS04), AS15, a saponin, QS-21,Quil-A, ISCOMs, ISCOMATRIX™, MF59™, Montanide® ISA 51, Montanide® ISA 720, AS02, a liposome or liposomal formulation, AS01, synthesized or specifically prepared microparticles and microcarriers, bacteria-derived outer membrane vesicles of *N. gonorrheae* or *Chlamydia trachomatis,* chitosan particles, a depot-forming agent, Pluronic® block co-polymers, specifically modified or prepared peptides, muramyl dipeptide, an aminoalkyl glucosaminide 4-phosphate, RC529, a bacterial toxoid, a toxin fragment, an agonist of Toll-Like Receptors 2, 3, 4, 5, 7, 8 or 9, an adenine derivative, immunostimulatory DNA, immunostimulatory RNA, an imidazoquinoline amine, an imidazopyridine amine, a 6,7-fused cycloalkylimidazopyridine amine, a 1,2-bridged imidazoquinoline amine, imiquimod, resiquimod, an agonist for DC surface molecule CD40, a type I interferon, poly I:C, a bacterial lipopolysacccharide (LPS), VSV-G, HMGB-1, flagellin or portions or derivatives thereof, an immunostimulatory DNA molecule comprising CpG, proinflammatory stimuli released from necrotic cells, urate crystals, an activated component of the complement cascade, an activated component of immune complexes, a complement receptor agonist, a cytokine, or a cytokine receptor agonist.
85. The composition of any of paragraphs 82-84, wherein the adjuvants are different.
86. The composition of any of paragraphs 82-85, wherein the adjuvant coupled to the first population of synthetic nanocarriers and/or the adjuvant coupled to the second population of synthetic nanocarriers comprises a TLR-2, -3, -4, -7, -8 or -9 agonist.
87. The composition of paragraph 86, wherein the adjuvant coupled to the first population of synthetic nanocarriers and/or the adjuvant coupled to the second population of synthetic nanocarriers comprises an immunostimulatory nucleic acid, imidazoquinoline, oxoadenine, MPL, imiquimod or resiquimod.
88. The composition of any of paragraphs 83-87, wherein the admixed adjuvant is an immunostimulatory nucleic acid comprising CpG, AS01, AS02, AS04, AS15, QS-21, a saponin, alum or MPL.
89. The composition of any of paragraphs 63-88, wherein the first and second populations of synthetic nanocarriers are present in an amount effective to generate an immune response to the first set of surface antigens and the second set of surface antigens in a subject.
90. The composition of paragraph 89, wherein the immune response is the generation of antibody titers specific for the first set of surface antigens and the second set of surface antigens.
91. The composition of any of paragraphs 63-90, further comprising one or more additional populations of synthetic nanocarriers, wherein each additional population of synthetic nanocarriers comprises a set of surface antigens immunologically different from the other sets of surface antigens in the composition.
92. The composition of paragraph 91, wherein at least one of the one or more additional populations of synthetic nanocarriers further comprise an adjuvant coupled thereto.
93. The composition of paragraph 92, wherein the adjuvant coupled to the at least one of the one or more additional populations of synthetic nanocarriers is different from the other adjuvants in the composition.
94. The composition of any of paragraphs 63-93, wherein each set of surface antigens is a monovalent or oligovalent set of surface antigens.
95. The composition of any of paragraphs 91-94, wherein the populations of synthetic nanocarriers are present in an amount effective to generate an immune response to each set of surface antigens.
96. The composition of paragraph 95, wherein the immune response is the generation of antibody titers specific for each set of surface antigens.
97. The composition of any of paragraphs 63-96, wherein the first and/or second population of synthetic nanocarriers further comprise a universal T cell antigen coupled thereto.
98. The composition of paragraph 97, wherein the universal T cell antigen comprises a T helper cell antigen.
99. The composition of paragraph 98, wherein the T-helper cell antigen comprises a peptide obtained or derived from ovalbumin.
100. The composition of paragraph 99, wherein the peptide obtained or derived from ovalbumin comprises the sequence as set forth in SEQ ID NO: 1.
101. The composition of any of paragraphs 97-100, wherein the universal T cell antigen is coupled by encapsulation.
102. The composition of any of paragraphs 1-101, wherein the pharmaceutically acceptable excipient comprises a preservative, a buffer, saline, phosphate buffered saline, a colorant, or a stabilizer.
103. The composition of any of paragraphs 1-102, wherein synthetic nanocarriers of each of the populations of synthetic nanocarriers comprise lipid-based nanoparticles, polymeric nanoparticles, metallic nanoparticles, surfactant-based emulsions, dendrimers, buckyballs, nanowires, virus-like particles, peptide or protein-based particles, lipid-polymer nanoparticles, spheroidal nanoparticles, cuboidal nanoparticles, pyramidal nanoparticles, oblong nanoparticles, cylindrical nanoparticles, or toroidal nanoparticles.
104. The composition of paragraph 103, wherein each of the populations of synthetic nanocarriers comprise one or more polymers.
105. The composition of paragraph 104, wherein the one or more polymers comprise a polyester.
106. The composition of paragraph 104 or 105, wherein the one or more polymers comprise or further comprise a polyester coupled to a hydrophilic polymer.
107. The composition of paragraph 105 or 106, wherein the polyester comprises a poly(lactic acid), poly(glycolic acid), poly(lactic-co-glycolic acid), or polycaprolactone.
108. The composition of paragraph 106 or 107, wherein the hydrophilic polymer comprises a polyether.
109. The composition of paragraph 108, where in the polyether comprises polyethylene glycol.
110. A composition comprising:
   a dosage form comprising:
      a first synthetic nanocarrier means for presenting a first set of surface antigens;
      a second synthetic nanocarrier means for presenting a second set of surface antigens; and
      a pharmaceutically acceptable excipient;
   wherein the first set of surface antigens and the second set of surface antigens are structurally different.
111. The composition of paragraph 110 wherein:
   the first set of surface antigens comprises a first set of monovalent or oligovalent surface antigens; and
   the second set of surface antigens comprises a second set of monovalent or oligovalent surface antigens.
112. A composition comprising:
   a dosage form comprising:
      a first synthetic nanocarrier means for presenting a first set of surface antigens;
      a second synthetic nanocarrier means for presenting a second set of surface antigens; and
      a pharmaceutically acceptable excipient;
   wherein the first set of surface antigens and the second set of surface antigens are immunologically different.
113. The composition of paragraph 112 wherein:
   the first set of surface antigens comprises a first set of monovalent or oligovalent surface antigens; and
   the second set of surface antigens comprises a second set of monovalent or oligovalent surface antigens.
114. A method comprising:
   administering the composition of any of paragraphs 1-113 to a subject.
115. The method of paragraph 114, wherein the subject has or is at risk of having an infection or infectious disease.
116. The method of paragraph 114, wherein the subject has or is at risk of having cancer.
117. The method of paragraph 114, wherein the subject has or is at risk of having an addiction.
118. The method of any of paragraphs 114-117, wherein the composition is administered by oral, subcutaneous, pulmonary, intranasal, intradermal or intramuscular administration.
119. A method comprising:
   preparing a first population of synthetic nanocarriers that comprise a first set of surface antigens;
   preparing a second population of synthetic nanocarriers that comprise a second set of surface antigens; and
   combining the first and second populations of synthetic nanocarriers into a dosage form;
   wherein the first set of surface antigens and the second set of surface antigens are structurally different.
120. A method comprising:
   preparing a first population of synthetic nanocarriers that comprise a first set of surface antigens;
   preparing a second population of synthetic nanocarriers that comprise a second set of surface antigens; and
   combining the first and second populations of synthetic nanocarriers into a dosage form;
   wherein the first set of surface antigens and the second set of surface antigens are immunologically different.
121. The method of paragraph 119 or 120, further comprising administering the dosage form to a subject.
122. The method of paragraph 121, further comprising determining whether or not an immune response to each set of surface antigens is generated.
123. The method of paragraph 122, wherein the immune response is the generation of antibody titers specific for each set of surface antigens.
124. The method of any of paragraphs 121-123, further comprising determining the amount effective to generate the immune response to each set of surface antigens.
125. A process for producing a dosage form of a composition, the process comprising the method steps as defined in any one of paragraphs 119-124.
126. The composition of any one of paragraphs 1-113 for use in therapy or prophylaxis.
127. The composition of any one of paragraphs 1-113 for use in a method as defined in any one of paragraphs 114-118 and/or 121-124.
128. The composition of any one of paragraphs 1-113 for use in a method of treating or preventing infection or infectious disease.
129. The composition of any one of paragraphs 1-113 for use in a method of treating or preventing cancer.
130. The composition of any one of paragraphs 1-113 for use in a method of treating or preventing an addiction.
131. The composition of any of paragraphs 128-130, wherein the method comprises administration of the composition by oral, subcutaneous, pulmonary, intranasal, intradermal or intramuscular administration.
132. Use of the composition of any one of paragraphs 1-113 for the manufacture of a medicament for use in a method as defined in any one of paragraphs 114-118, 121-124 or 128-131.

## Claims

1. A composition comprising:
a dosage form comprising:
a first population of synthetic nanocarriers that comprise a first set of surface antigens;
a second population of synthetic nanocarriers that comprise a second set of surface antigens; and
a pharmaceutically acceptable excipient;
wherein the first set of surface antigens and the second set of surface antigens are structurally or immunologically different.

2. The composition of claim 1, wherein the first set of surface antigens comprises 1, 2, 3, 4, 5, 6, 7, 8, 9, 10 or more types of antigens and/or the second set of surface antigens comprises 2, 3, 4, 5, 6, 7, 8, 9, 10 or more types of antigens.

3. The composition of claim 1 or claim 2, wherein the first set of surface antigens comprise antigens obtained or derived from a first infectious genus, species or strain and the second set of surface antigens comprise antigens obtained or derived from a second infectious genus, species or strain, optionally wherein the first infectious genus, species or strain and the second infectious genus, species or strain are the same.

4. The composition of any of claims 1-3, wherein the first set of surface antigens and/or second set of surface antigens comprise antigens that are obtained or derived from:
(a) a virus of the Adenoviridae, Picornaviridae, Herpesviridae, Hepadnaviridae, Flaviviridae, Retroviridae, Orthomyxoviridae, Paramyxoviridae, Papillomaviridae, Rhabdoviridae, Togaviridae or Paroviridae family, optionally being obtained or derived from adenovirus, coxsackievirus, hepatitis A virus, poliovirus, Rhinovirus, Herpes simplex virus, Varicella-zoster virus, Epstein-barr virus, Human cytomegalovirus, Human herpesvirus, Hepatitis B virus, Hepatitis C virus, yellow fever virus, dengue virus, West Nile virus, HIV, Influenza virus, Measles virus, Mumps virus, Parainfluenza virus, Respiratory syncytial virus, Human metapneumovirus, Human papillomavirus, Rabies virus, Rubella virus, Human bocarivus or Parvovirus B19, for example being obtained or derived from VI, VII, E1A, E3-19K, 52K, VP1, surface antigen, 3A protein, capsid protein, nucleocapsid, surface projection, transmembrane proteins, UL6, UL18, UL35, UL38, UL19, early antigen, capsid antigen, Pp65, gB, p52, latent nuclear antigen-1, NS3, envelope protein, envelope protein E2 domain, gp120, p24, lipopeptides Gag (17-35), Gag (253-284), Nef (66-97), Nef (116-145), Pol (325-355), neuraminidase, nucleocapsid protein, matrix protein, phosphoprotein, fusion protein, hemagglutinin, hemagglutinin-neuraminidase, glycoprotein, E6, E7, envelope lipoprotein or non-structural protein (NS); and/or;
(b) bacteria of the Bordetella, Borrelia, Brucella, Campylobacter, Chlamydia and Chlamydophila, Clostridium, Corynebacterium, Enterococcus, Escherichia, Francisella, Haemophilus, Helicobacter, Legionella, Leptospira, Listeria, Mycobacterium, Mycoplasma, Neisseria, Pseudomonas, Rickettsia, Salmonella, Shigella, Staphylococcus, Streptococcus, Treponema Vibrio or Yersinia genus, optionally being obtained or derived from Bordetella pertussis, Borrelia burgdorferi, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diphtheriae, Enterococcus faecalis, Enterococcus faecium, Escherichia coli, Francisella tularensis, Haemophilus influenzae, Helicobacter pylori, Legionella pneumophila, Leptospira interrogans, Listeria monocytogenes, Mycobacterium leprae, Mycobacterium tuberculosis, Mycobacterium ulcerans, Mycoplasma pneumoniae, Neisseria gonorrhoeae, Neisseria meningitides, Pseudomonas aeruginosa, Rickettsia rickettsii, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae or Yersinia pestis, for example being obtained or derived from pertussis toxin (PT), filamentous hemagglutinin (FHA), pertactin (PRN), fimbriae (FIM 2/3), VlsE; DbpA, OspA, Hia, PrpA, MltA, L7/L12, D15, 0187, VirJ, Mdh, AfuA, L7/L12, out membrane protein, LPS, antigen type A, antigen type B, antigen type C, antigen type D, antigen type E, FliC, FliD, Cwp84, alpha-toxin, theta-toxin, fructose 1,6-biphosphate-aldolase (FBA), glyceraldehydes-3-phosphate dehydrogenase (GPD), pyruvate:ferredoxin oxidoreductase (PFOR), elongation factor-G (EF-G), hypothetical protein (HP), T toxin, Toxoid antigen, capsular polysaccharide, Protein D, Mip, nucleoprotein (NP), RD1, PE35, PPE68, EsxA, EsxB, RD9, EsxV, Hsp70, lipopolysaccharide, surface antigen, Sp1, Sp2, Sp3, Glycerophosphodiester Phosphodiesterase, outer membrane protein, chaperone-usher protein, capsular protein (Fl) or V protein; and/or
(c) a fungus of the Candida, Aspergillus, Cryptococcus, Histoplasma, Pneumocystis or Stachybotrys genus, optionally being obtained or derived from C. albicans, Aspergillus fumigatus, Aspergillus flavus, Cryptococcus neoformans, Cryptococcus laurentii, Cryptococcus albidus, Cryptococcus gattii, Histoplasma capsulatum, Pneumocystis jirovecii or Stachybotrys chartarum, for example being obtained or derived from surface antigen, capsular glycoprotein, Yps3P, Hsp60, Major surface protein, MsgC1, MsgC3, MsgC8, MsgC9 or SchS34.

5. The composition of any of the preceding claims, further comprising one or more adjuvants, optionally wherein the first population of synthetic nanocarriers and/or the second population of synthetic nanocarriers further comprise an adjuvant coupled to the synthetic nanocarriers.

6. The composition of claim 5, wherein: (a) the adjuvants are different; and/or (b) the adjuvant coupled to the first population of synthetic nanocarriers and/or the adjuvant coupled to the second population of synthetic nanocarriers comprises a TLR-2, -3, -4, -7, -8 or -9 agonist; and/or (c) the adjuvant coupled to the first population of synthetic nanocarriers and/or the adjuvant coupled to the second population of synthetic nanocarriers comprises an immunostimulatory nucleic acid, imidazoquinoline, oxoadenine, MPL, imiquimod or resiquimod; and/or (d) the composition comprises one or more admixed adjuvants, optionally wherein the admixed adjuvant is an immunostimulatory nucleic acid comprising CpG, AS01, AS02, AS04, AS15, QS-21, a saponin, alum or MPL; and/or (e) each of the one or more adjuvants comprises a mineral salt, alum, alum combined with monphosphoryl lipid (MPL) A of Enterobacteria, MPL® (AS04), AS15, a saponin, QS-21,Quil-A, ISCOMs, ISCOMATRIX™, MF59™, Montanide® ISA 51, Montanide® ISA 720, AS02, a liposome or liposomal formulation, AS01, AS15, synthesized or specifically prepared microparticles and microcarriers, bacteria-derived outer membrane vesicles of *N. gonorrheae* or *Chlamydia trachomatis,* chitosan particles, a depot-forming agent, Pluronic® block co-polymers, specifically modified or prepared peptides, muramyl dipeptide, an aminoalkyl glucosaminide 4-phosphate, RC529, a bacterial toxoid, a toxin fragment, an agonist of Toll-Like Receptors 2, 3, 4, 5, 7, 8 or 9, an adenine derivative, immunostimulatory DNA, immunostimulatory RNA, an imidazoquinoline amine, an imidazopyridine amine, a 6,7-fused cycloalkylimidazopyridine amine, a 1,2-bridged imidazoquinoline amine, imiquimod, resiquimod, an agonist for DC surface molecule CD40, a type I interferon, poly I:C, a bacterial lipopolysacccharide (LPS), VSV-G, HMGB-1, flagellin or portions or derivatives thereof, an immunostimulatory DNA molecule comprising CpG, proinflammatory stimuli released from necrotic cells, urate crystals, an activated component of the complement cascade, an activated component of immune complexes, a complement receptor agonist, a cytokine, or a cytokine receptor agonist.

7. The composition of any of the preceding claims wherein the first and second populations of synthetic nanocarriers are present in an amount effective to generate an immune response to the first set of surface antigens and the second set of surface antigens in a subject, optionally wherein the immune response is the generation of antibody titers specific for the first set of surface antigens and the second set of surface antigens.

8. The composition of any of the preceding claims, further comprising one or more additional populations of synthetic nanocarriers, wherein each additional population of synthetic nanocarriers comprises a set of surface antigens structurally or immunologically different from the other sets of surface antigens in the composition, optionally wherein at least one of the one or more additional populations of synthetic nanocarriers further comprise an adjuvant coupled thereto, for example wherein the adjuvant coupled to the at least one of the one or more additional populations of synthetic nanocarriers is different from the other adjuvants in the composition.

9. The composition of any of the preceding claims, wherein each set of surface antigens is a monovalent or oligovalent set of surface antigens.

10. The composition of claims 8 or 9, wherein the populations of synthetic nanocarriers are present in an amount effective to generate an immune response to each set of surface antigens, wherein the immune response is optionally the generation of antibody titers specific for each set of surface antigens.

11. The composition of any of the preceding claims, wherein the first and/or second population of synthetic nanocarriers further comprise a universal T cell antigen coupled, optionally by encapsulation, thereto, wherein the universal T cell antigen optionally comprises a T helper cell antigen, for example comprising a peptide obtained or derived from ovalbumin, e.g. comprising the sequence as set forth in SEQ ID NO: 1.

12. The composition of claim 1 or claim 2, wherein the first set of surface antigens and the second set of surface antigens are structurally or immunologically different and the first set of surface antigens and second set of surface antigens comprise antigens obtained or derived from an abused or addictive substance, optionally cocaine or nicotine.

13. The composition of any of the preceding claims, wherein: (a) the first set of surface antigens and the second set of surface antigens comprise the same surface antigens, and wherein at least one antigen of the first set of surface antigens is presented in a different orientation than as presented in the second set of surface antigens; or (b) the first set of surface antigens and the second set of surface antigens comprise the same surface antigens, and wherein at least one antigen of the first set of surface antigens is presented in a different conformation than as presented in the second set of surface antigens; or (c) the molecular structure of the first set of surface antigens and the second set of surface antigens are different.

14. The composition of any of the preceding claims, wherein: (a) the first set of surface antigens comprise surface antigens that comprise peptides, proteins, oligosaccharides, polysaccharides and/or small molecules; and/or (b) the second set of surface antigens comprise surface antigens that comprise peptides, proteins, oligosaccharides, polysaccharides and/or small molecules; and/or (c) at least one surface antigen of the first set of surface antigens and/or at least one surface antigen of the second set of surface antigens has a molecular weight of less than 10,000 Da.

15. The composition of any of the preceding claims, wherein the pharmaceutically acceptable excipient comprises a preservative, a buffer, saline, phosphate buffered saline, a colorant, or a stabilizer.

16. The composition of any of the preceding claims, wherein synthetic nanocarriers of each of the populations of synthetic nanocarriers comprise lipid-based nanoparticles, polymeric nanoparticles, metallic nanoparticles, surfactant-based emulsions, dendrimers, buckyballs, nanowires, virus-like particles, peptide or protein-based particles, lipid-polymer nanoparticles, spheroidal nanoparticles, cuboidal nanoparticles, pyramidal nanoparticles, oblong nanoparticles, cylindrical nanoparticles, or toroidal nanoparticles.

17. The composition of claim 16, wherein each of the populations of synthetic nanocarriers comprise one or more polymers, optionally wherein: (a) the one or more polymers comprise a polyester; and/or (b) the one or more polymers comprise or further comprise a polyester coupled to a hydrophilic polymer, for example wherein the polyester comprises a poly(lactic acid), poly(glycolic acid), poly(lactic-co-glycolic acid), or polycaprolactone and/or the hydrophilic polymer comprises a polyether (e.g. comprising polyethylene glycol).

18. A composition comprising:
a dosage form comprising:
a first synthetic nanocarrier means for presenting a first set of surface antigens;
a second synthetic nanocarrier means for presenting a second set of surface antigens; and
a pharmaceutically acceptable excipient;
wherein the first set of surface antigens and the second set of surface antigens are structurally or immunologically different, optionally wherein:
the first set of surface antigens comprises a first set of monovalent or oligovalent surface antigens; and
the second set of surface antigens comprises a second set of monovalent or oligovalent surface antigens.

19. The composition of any of the preceding claims for use in therapy or prophylaxis, for example for use in a method: (a) of treating or preventing infection or infectious disease; (b) treating or preventing cancer; (c) treating or preventing an addiction; and/or (d) comprising administration of the composition by oral, subcutaneous, pulmonary, intranasal, intradermal or intramuscular administration.

20. A process for producing a dosage form of a composition, the process comprising:
preparing a first population of synthetic nanocarriers that comprise a first set of surface antigens;
preparing a second population of synthetic nanocarriers that comprise a second set of surface antigens; and
combining the first and second populations of synthetic nanocarriers into a dosage form;
wherein the first set of surface antigens and the second set of surface antigens are structurally or immunologically different, and optionally further comprising the step of determining: (a) whether or not an immune response to each set of surface antigens is generated, for example the generation of antibody titers specific for each set of surface antigens; and/or (b) the amount effective to generate the immune response to each set of surface antigens.


SEQUENCE LISTING

<110>  Selecta Biosciences, Inc.

<120>  MULTIVALENT SYNTHETIC NANOCARRIER VACCINES

<130>  S1681.70014WO00

<150>  US 61/348,713
<151>  2010-05-26

<150>  US 61/348,717
<151>  2010-05-26

<150>  US 61/348,728
<151>  2010-05-26

<150>  US 61/358,635
<151>  2010-06-25

<160>  1

<170>  PatentIn version 3.5

<210>  1
<211>  17
<212>  PRT
<213>  G. gallus

<400>  1

Ile Ser Gln Ala Val His Ala Ala His Ala Glu Ile Asn Glu Ala Gly
1               5                   10                  15


Arg